(19) Europäisches Patentamt
European Patent Office
Office européen des brevets

(11) EP 4 810 463 A1

(12) **EUROPEAN PATENT APPLICATION**
published in accordance with Art. 153(4) EPC

(43) Date of publication:
**23.09.2026 Bulletin 2026/39**

(21) Application number: **24891477.2**

(22) Date of filing: **14.11.2024**

(51) International Patent Classification (IPC):
***C07C 7/09*** (2006.01) ***B01J 29/40*** (2006.01)
***C07B 61/00*** (2006.01) ***C07C 1/24*** (2006.01)
***C07C 11/04*** (2006.01) ***C07C 11/06*** (2006.01)

(52) Cooperative Patent Classification (CPC):
**B01J 29/40; C07B 61/00; C07C 1/24; C07C 7/09; C07C 11/04; C07C 11/06**

(86) International application number:
**PCT/JP2024/040521**

(87) International publication number:
**WO 2025/105441 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.11.2023 JP 2023194644**

(71) Applicant: **ASAHI KASEI KABUSHIKI KAISHA**
**Tokyo 100-0006 (JP)**

(72) Inventors:
- **URAYAMA, Teppei**
  **Tokyo 100-0006 (JP)**
- **MIYAZAKI, Ryusuke**
  **Tokyo 100-0006 (JP)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

(54) **METHOD FOR PRODUCING LIGHT OLEFIN**

(57) A method for producing light olefin, comprising:
a cracking step of introducing a naphtha feedstock to a cracking furnace to obtain a naphtha cracking fraction;
an ethanol conversion step of introducing a starting material containing ethanol to a reactor where the starting material is brought into contact with a catalyst to obtain an ethanol conversion fraction containing ethylene and propylene;
a combination step of combining at least a portion of the naphtha cracking fraction or a fraction derived therefrom with at least a portion of the ethanol conversion fraction or a fraction derived therefrom to obtain a combination fraction; and
a cryogenic separation step of introducing the combination fraction or a fraction derived therefrom to a cryogenic separation equipment to separate ethylene and propylene, wherein
a relationship between a content $C_E$ of ethylene and propylene in the ethanol conversion fraction or a fraction derived therefrom and a content Cc of ethylene and propylene in the naphtha cracking fraction or a fraction derived therefrom in the combination step satisfies the expression (A):

$$C_E > C_C \ldots (A).$$

[Figure 1]

E
EtOH
Ethanol conversion process
N
Naphtha cracking process
Naphtha
Naphtha cracking
Naphtha cracking fraction refining process
Cryogenic separation
N1
N2
N3



Processed by Luminess, 75001 PARIS (FR)

## Description

### Technical Field

The present invention relates to a method for producing light olefin.

### Background Art

Various chemical products such as monomeric starting materials have been produced by the cracking of naphtha obtained from crude oil. Among these chemical products, light olefin such as ethylene and propylene is used for a wide variety of purposes and therefore has a particularly high demand. Since propylene, in particular, has a high value, methods for selectively producing propylene from naphtha have been studied. Steam cracking is known as a representative example (e.g., Patent Document 1).

Methods using catalysts such as zeolite have been reported as methods for obtaining light olefin from an alcohol. For example, Patent Document 2 proposes, as a method for efficiently and stably producing propylene, a method for producing propylene by bringing at least one starting material selected from ethylene and ethanol into contact with an intermediate pore-size zeolite-containing catalyst in a fluidized-bed reactor.

### List of Prior Art Documents

### Patent Document

Patent Document 1: Japanese Patent Laid-Open No. 2016-117800

Patent Document 2: International Publication No. WO2009/037992

### Summary of Invention

### Problems to be Solved by Invention

An object of the present invention is to provide a method for producing light olefin which efficiently refines olefin having 2 or 3 carbon atoms.

### Means for Solving Problems

The present inventors have found that the object mentioned above can be attained by controlling the composition of each fraction in a method for producing light olefin.

The present invention encompasses the following embodiments.

<1> A method for producing light olefin, comprising:

a cracking step of introducing a naphtha feedstock to a cracking furnace to obtain a naphtha cracking fraction;

an ethanol conversion step of introducing a starting material containing ethanol to a reactor where the starting material is brought into contact with a catalyst to obtain an ethanol conversion fraction containing ethylene and propylene;

a combination step of combining at least a portion of the naphtha cracking fraction or a fraction derived therefrom with at least a portion of the ethanol conversion fraction or a fraction derived therefrom to obtain a combination fraction; and

a cryogenic separation step of introducing the combination fraction or a fraction derived therefrom to a cryogenic separation equipment to separate ethylene and propylene, wherein

a relationship between a content $C_E$ of ethylene and propylene in the ethanol conversion fraction or a fraction derived therefrom and a content Cc of ethylene and propylene in the naphtha cracking fraction or a fraction

derived therefrom in the combination step satisfies the expression (A):

$$C_E > C_C \ldots (A).$$

<2> The method for producing light olefin according to <1>, wherein a difference between the content $C_E$ and the content Cc ($C_E$ - Cc) in the combination step is 5% by mass or more.
<3> The method for producing light olefin according to <1> or <2>, wherein a relationship between a propylene/ethylene mass ratio $P/E_E$ in the ethanol conversion fraction and a propylene/ethylene mass ratio P/Ec in the naphtha cracking fraction satisfies the expression (α):

$$-0.25 < (P/E_E - P/E_C) < 0.30 \ldots (\alpha).$$

<4> The method for producing light olefin according to any of <1> to <3>, wherein a relationship between a content $C_{E_AP}$ of ethylene and propylene in the ethanol conversion fraction and a content $C_{C_AP}$ of ethylene and propylene in the naphtha cracking fraction satisfies the expression (β-1):

$$|C_{E_AP} - C_{C_AP}| < 20\% \text{ by mass} \ldots (\beta\text{-}1).$$

<5> A method for producing light olefin, comprising:

a cracking step of introducing a naphtha feedstock to a cracking furnace to obtain a naphtha cracking fraction;

an ethanol conversion step of introducing a starting material containing ethanol to a reactor where the starting material is brought into contact with a catalyst to obtain an ethanol conversion fraction containing ethylene and propylene;

a combination step of combining at least a portion of the naphtha cracking fraction or a fraction derived therefrom with at least a portion of the ethanol conversion fraction or a fraction derived therefrom to obtain a combination fraction; and

a cryogenic separation step of introducing the combination fraction or a fraction derived therefrom to a cryogenic separation equipment to separate ethylene and propylene, wherein

a relationship between a propylene/ethylene mass ratio $P/E_E$ in the ethanol conversion fraction and a propylene/ethylene mass ratio P/Ec in the naphtha cracking fraction satisfies the expression (α):

$$-0.25 < (P/E_E - P/E_C) < 0.30 \ldots (\alpha),$$

and

a relationship between a content $C_{E_AP}$ of ethylene and propylene in the ethanol conversion fraction and a content $C_{C_AP}$ of ethylene and propylene in the naphtha cracking fraction satisfies the expression (β-1):

$$|C_{E_AP} - C_{C_AP}| < 20\% \text{ by mass} \ldots (\beta\text{-}1).$$

<6> The method for producing light olefin according to <5>, wherein the relationship between the content $C_{E_AP}$ of ethylene and propylene in the ethanol conversion fraction and the content $C_{C_AP}$ of ethylene and propylene in the naphtha cracking fraction satisfies the expression (β-2):

$$0 \leq (C_{E_AP} - C_{C_AP}) < 20\% \text{ by mass} \ldots (\beta\text{-}2).$$

<7> The method for producing light olefin according to any of <1> to <6>, comprising a first cooling step of introducing the naphtha cracking fraction or the combination fraction to a first cooling tower to obtain a first cooling fraction mainly containing olefin having 6 or less carbon atoms.
<8> The method for producing light olefin according to any of <1> to <7>, comprising a second cooling step of

introducing the naphtha cracking fraction or the combination fraction or a fraction derived therefrom to a second cooling tower to obtain a second cooling fraction mainly containing olefin having 4 or less carbon atoms.

<9> The method for producing light olefin according to any of <1> to <8>, comprising a third cooling step of introducing the ethanol conversion fraction to a third cooling tower to obtain a cooled ethanol conversion fraction mainly containing olefin having 6 or less carbon atoms.

<10> The method for producing light olefin according to <9>, comprising a compression and separation step of pressure-boosting the cooled ethanol conversion fraction using a compressor to obtain a light ethanol conversion fraction mainly containing olefin having 3 or less carbon atoms as a gas component and to obtain a heavy ethanol conversion fraction mainly containing olefin having 4 or more carbon atoms as a liquid component.

<11> The method for producing light olefin according to <10>, comprising a recycling step of introducing at least a portion of the heavy ethanol conversion fraction as a portion of the starting material to the reactor.

<12> The method for producing light olefin according to any of <1> to <11>, comprising, after the combination step, a washing step of introducing the combination fraction or a fraction derived therefrom to a soda washing tower to obtain a washing fraction.

<13> The method for producing light olefin according to any of <1> to <12>, comprising, after the second cooling step and before the cryogenic separation step, a first compression step of pressure-boosting the fraction.

<14> The method for producing light olefin according to any of <1> to <13>, wherein a relationship between a ratio $O_E$ of an amount of hydrogen and methane to a total amount of ethylene and propylene in the ethanol conversion fraction and a ratio $O_C$ of an amount of hydrogen and methane to a total amount of ethylene and propylene in the naphtha cracking fraction satisfies the expression (1):

$$O_E < O_C \quad \ldots (1).$$

<15> The method for producing light olefin according to any of <1> to <14>, wherein the method satisfies any of the following (1) to (5):

(1) comprising:

a first cooling step of introducing the naphtha cracking fraction to a first cooling tower to obtain a first cooling fraction mainly containing olefin having 6 or less carbon atoms; and

a second cooling step of introducing the first cooling fraction to a second cooling tower to obtain a second cooling fraction mainly containing olefin having 4 or less carbon atoms, wherein

in the combination step, at least a portion of the second cooling fraction is combined with at least a portion of the ethanol conversion fraction or a fraction derived therefrom,

(2) comprising:

a first cooling step of introducing the naphtha cracking fraction to a first cooling tower to obtain a first cooling fraction mainly containing olefin having 6 or less carbon atoms; and

a second cooling step of introducing the combination fraction or a fraction derived therefrom to a second cooling tower to obtain a second cooling fraction mainly containing olefin having 4 or less carbon atoms, wherein

in the combination step, at least a portion of the first cooling fraction is combined with at least a portion of the ethanol conversion fraction or a fraction derived therefrom,

(3) comprising:

a first cooling step of introducing the naphtha cracking fraction to a first cooling tower to obtain a first cooling fraction mainly containing olefin having 6 or less carbon atoms;

a second cooling step of introducing the first cooling fraction to a second cooling tower to obtain a second cooling fraction mainly containing olefin having 4 or less carbon atoms;

a circulation step of introducing at least a portion of a second heavy fraction mainly containing hydrocarbon having 5 or more carbon atoms in the second cooling step to the second cooling tower; and

a first introduction step of introducing the combination fraction to the second cooling tower, wherein

in the combination step, at least a portion of the second heavy fraction is combined with at least a portion of the ethanol conversion fraction or a fraction derived therefrom,

(4) comprising:

a first cooling step of introducing the combination fraction or a fraction derived therefrom to a first cooling tower to obtain a first cooling fraction mainly containing olefin having 6 or less carbon atoms; and

a second cooling step of introducing the first cooling fraction to a second cooling tower to obtain a second cooling fraction mainly containing olefin having 4 or less carbon atoms, wherein

in the combination step, at least a portion of the naphtha cracking fraction is combined with at least a portion of the ethanol conversion fraction or a fraction derived therefrom, and

(5) comprising:

a first cooling step of introducing the naphtha cracking fraction to a first cooling tower to obtain a first cooling fraction mainly containing olefin having 6 or less carbon atoms;

a second cooling step of introducing the first cooling fraction to a second cooling tower to obtain a second cooling fraction mainly containing olefin having 4 or less carbon atoms;

a circulation step of introducing at least a portion of a second heavy fraction mainly containing hydrocarbon having 5 or more carbon atoms in the second cooling step to the first cooling tower; and

a second introduction step of introducing the combination fraction to the first cooling tower, wherein

in the combination step, at least a portion of the second heavy fraction is combined with at least a portion of the ethanol conversion fraction or a fraction derived therefrom.

## Advantages of Invention

The present invention can provide a method for producing light olefin which efficiently refines olefin having 2 or 3 carbon atoms.

## Brief Description of Drawings

[Figure 1] Figure 1 is a block diagram showing the summary of the method for producing light olefin according to the present embodiment.

[Figure 2] Figure 2 is a schematic configuration diagram of production equipment for use in the method for producing light olefin according to the present embodiment.

[Figure 3] Figure 3 is a schematic configuration diagram of a fixed-bed single-stage adiabatic reactor.

[Figure 4] Figure 4 is a schematic configuration diagram of production equipment for use in the method for producing light olefin according to the present embodiment.

[Figure 5] Figure 5 is a schematic configuration diagram of production equipment for use in the method for producing light olefin according to the present embodiment.

[Figure 6] Figure 6 is a schematic configuration diagram of production equipment for use in the method for producing light olefin according to the present embodiment.

[Figure 7] Figure 7 is a schematic configuration diagram of production equipment for use in the method for producing light olefin according to the present embodiment.

[Figure 8] Figure 8 is a schematic configuration diagram of production equipment for use in the method for producing light olefin according to the present embodiment.

[Figure 9] Figure 9 is a schematic configuration diagram of production equipment for use in the method for producing light olefin according to the present embodiment.

[Figure 10] Figure 10 is a schematic configuration diagram of production equipment for use in the method for producing light olefin according to the present embodiment.

[Figure 11] Figure 11 is a schematic configuration diagram of production equipment for use in the method for producing light olefin according to the present embodiment.

[Figure 12] Figure 12 is a schematic configuration diagram of production equipment for use in the method for producing light olefin according to the present embodiment.

[Figure 13] Figure 13 is a schematic configuration diagram of production equipment for use in the method for producing light olefin according to the present embodiment.

[Figure 14] Figure 14 is a schematic configuration diagram of production equipment in the form of a conventional front-end demethanizer for use in a method for producing light olefin.

[Figure 15] Figure 15 is a schematic configuration diagram of production equipment for use in the method for producing light olefin according to the present embodiment.

[Figure 16] Figure 16 is a schematic configuration diagram of production equipment for use in the method for producing light olefin according to the present embodiment.

[Figure 17] Figure 17 is a schematic configuration diagram of production equipment for use in the method for producing light olefin according to the present embodiment.

[Figure 18] Figure 18 is a schematic configuration diagram of production equipment for use in the method for producing light olefin according to the present embodiment.

[Figure 19] Figure 19 is a schematic configuration diagram of production equipment for use in the method for producing light olefin according to the present embodiment.

[Figure 20] Figure 20 is a schematic configuration diagram of production equipment for use in the method for producing light olefin according to the present embodiment.

[Figure 21] Figure 21 is a schematic configuration diagram of production equipment for use in the method for producing light olefin according to the present embodiment.

[Figure 22] Figure 22 is a schematic configuration diagram of production equipment for use in the method for producing light olefin according to the present embodiment.

[Figure 23] Figure 23 is a schematic configuration diagram of production equipment for use in the method for producing light olefin according to the present embodiment.

[Figure 24] Figure 24 is a schematic configuration diagram of production equipment for use in the method for producing light olefin according to the present embodiment.

[Figure 25] Figure 25 is a schematic configuration diagram of production equipment for use in the method for

producing light olefin according to the present embodiment.

[Figure 26] Figure 26 is a schematic configuration diagram of production equipment in the form of a conventional front-end depropanizer for use in a method for producing light olefin.

## Mode for Carrying Out Invention

The present invention will be specifically described below. The present invention is not limited by the following embodiments (present embodiment) and can be carried out through various changes or modifications made without departing from the spirit of the present invention.

A numerical range indicated by using the term "to" refers to a range including the numerical values described before and after the term "to" as the minimum value and the maximum value, respectively. In numerical ranges described in stages in the present specification, the upper limit value or the lower limit value of a numerical range at a certain stage can be arbitrarily combined with the upper limit value or the lower limit value of a numerical range at a different stage.

Although modifiers such as "first" and "second" may be used for discriminating elements, such modifiers do not necessarily indicate some specific order.

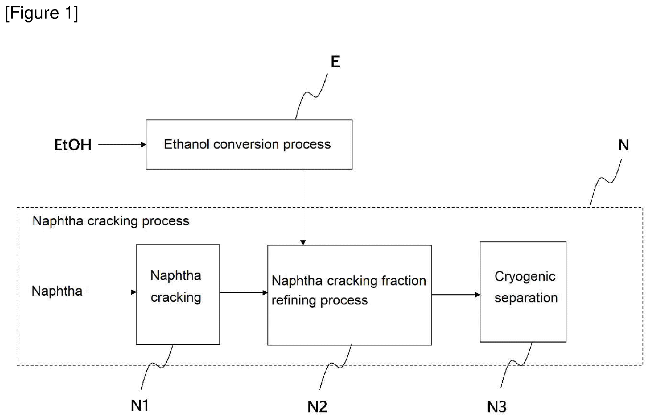

First, the method for producing light olefin according to the present embodiment will be summarized. Figure 1 is a block diagram showing the summary of the method for producing light olefin according to the present embodiment. As shown in Figure 1, the production method according to the present embodiment comprises a naphtha cracking process N and an ethanol conversion process E.

In the naphtha cracking process N, naphtha is thermally decomposed in cracking N1 and then subjected to a naphtha cracking fraction refining process N2, and ethylene and propylene are separated by cryogenic separation N3. In the cryogenic separation N3, the treatment fraction is cooled so that ethylene and propylene are liquefied to separate methane and hydrogen gases and liquid olefin. The cryogenic separation N3 requires liquefying a relatively light fraction in the naphtha cracking fraction and therefore requires pressure-boosting and cooling the fraction, and large energy is necessary for the treatment.

In the naphtha cracking process N, existing naphtha cracker equipment may be used. For example, an ethanol conversion fraction obtained by the ethanol conversion process mentioned later is introduced to the naphtha cracker equipment.

The ethanol conversion process E comprises, for example, the step of converting a starting material containing ethanol to an ethanol conversion fraction containing ethylene and propylene, and the like. In the ethanol conversion process E, use of bioethanol as the starting material produces a plant-derived ethanol conversion fraction. A portion of a petroleum-derived starting material can be replaced with a plant-derived starting material by connecting the ethanol conversion process E to existing equipment where the naphtha cracking process N is performed. Alternatively, the starting material can be converted to a plant-derived one, without changing the amount of the compound of interest produced, for example, by controlling the operating conditions of the ethanol conversion process E.

In the present embodiment, the ethanol conversion fraction obtained by the ethanol conversion process E or a fraction derived therefrom is introduced to the naphtha cracking process N, whereby load in the cryogenic separation N3 is reduced.

The naphtha cracking process N may comprise any step and comprises, for example, a cracking step, a first cooling step, a second cooling step, a combination step, a first compression step, a washing step, and a cryogenic separation step as steps mentioned later.

The ethanol conversion process E may comprise any step and comprises, for example, an ethanol conversion step, a refining step, a third cooling step, a second compression step, a distillation step, and a recycling step as steps mentioned later.

The "light olefin" is at least one member selected from the group consisting of ethylene and propylene. The light olefin is the compound of interest in the production method according to the present embodiment and is preferably propylene from the viewpoint of exploiting the features of the production method according to the present embodiment.

The "fraction derived therefrom" in relation to a specific fraction means a fraction derived from the specific fraction through some process such as distillation, cooling, or compression.

The term "mainly containing" in relation to a component in a fraction means that the fraction contains more than 50% by mass of the component.

The content of ethylene and propylene in the ethanol conversion fraction or a fraction derived therefrom in the combination step is represented by $C_E$, and the content of ethylene and propylene in the ethanol conversion fraction obtained by a reaction step without a subsequent separation process is represented by $C_{E_AP}$. The content of ethylene and propylene in the naphtha cracking fraction or a fraction derived therefrom in the combination step is represented by $C_C$, and the content of ethylene and propylene in the naphtha cracking fraction obtained by a cracking step without a subsequent separation process is represented by $C_{C_AP}$.

The method for producing light olefin according to the present embodiment encompasses an embodiment A and an embodiment B given below.

In a general method for producing light olefin by cracking, light olefin is obtained by separating and refining various components from a naphtha cracking fraction obtained by the cracking of naphtha. Although various processes are applicable thereto, the method for producing light olefin may involve a separation and refining step in which a fraction from which high-boiling components have been removed is finally liquefied by cryogenic separation, and off-gases such as hydrogen and methane are removed to obtain olefin having 2 or 3 carbon atoms, such as ethylene and propylene. In this case, further improvement in treatment efficiency is demanded.

An object of the embodiment A is to provide a method for producing light olefin which enhances the treatment efficiency of olefin having 2 or 3 carbon atoms.

The present inventors have found that the object mentioned above can be attained by controlling the composition of each fraction in a method for producing light olefin.

In the method for producing light olefin according to the embodiment A, a relationship between a content $C_E$ of ethylene and propylene in the ethanol conversion fraction or a fraction derived therefrom and a content $C_C$ of ethylene and propylene in the naphtha cracking fraction or a fraction derived therefrom in the combination step satisfies the expression (A):

$$C_E > C_C \ldots (A).$$

The expression (A) is thus satisfied, whereby the treatment efficiency of olefin having 2 or 3 carbon atoms can be enhanced. The expression (A) is satisfied, whereby, particularly, energy load in the cryogenic separation step can be reduced. For satisfying the expression (A), for example, it is preferred to design $C_E$ so as to elevate its value by establishing a cooling step prior to the combination of the ethanol conversion fraction with the naphtha cracking fraction.

In the naphtha cracking process, after naphtha cracking, light olefin is obtained by a cryogenic separation step through a refining process. In the cryogenic separation step, the fraction to be introduced to the cryogenic separation step is pressure-boosted using a compressor and cooled to a temperature equal to or lower than the boiling point of ethylene so that light olefin is liquefied to separate the light olefin from off-gases such as hydrogen and methane. In this pressure-boosting and cooling treatment, consumed energy is increased depending on the flow rate of the fraction to be introduced to the cryogenic separation step. In other words, the relationship between the content $C_E$ of ethylene and propylene in the ethanol conversion fraction and the content $C_C$ of ethylene and propylene in the naphtha cracking fraction falls within the range as mentioned above, whereby the amounts of compounds other than light olefin introduced to the cryogenic separation step are reduced, the content of ethylene and propylene in the fraction to be introduced to the cryogenic separation step can be elevated, and the flow rate of the fraction to be introduced is reduced when the amount of light olefin produced is constant; thus, cryogenic separation can be more efficiently performed. Compressors are limited by throughputs depending on their sizes. The improved content of light olefin in the fraction to be introduced to the cryogenic separation step can improve the amount of light olefin produced, for example, without resizing or remodeling a compressor.

The naphtha cracking fraction in the combination step preferably has a high content of light olefin from the viewpoint of light olefin production efficiency. For example, $C_C$ is preferably 10% by mass or more, more preferably 20% by mass or more, further preferably 30% by mass or more.

In the method for producing light olefin according to the embodiment A, a difference between the content $C_E$ and the content $C_C$ ($C_E$ - $C_C$) in the combination step is preferably 5% by mass or more, more preferably 10% by mass or more, further preferably 20% by mass or more. The upper limit of the difference ($C_E$ - $C_C$) is not particularly limited and may be, for example, 70% by mass or less.

In a general method for producing light olefin by cracking, light olefin is obtained by separating and refining various components from a naphtha cracking fraction obtained by the cracking of naphtha. The amount of light olefin produced varies depending on starting material conversion from naphtha to ethanol, and increase or decrease in operation rate of a refining system markedly deteriorates operation efficiency due to the surging of a compressor or the flooding of a distillation tower.

In the method for producing light olefin according to the embodiment B, a relationship between a propylene/ethylene mass ratio $P/E_E$ in the ethanol conversion fraction and a propylene/ethylene mass ratio P/Ec in the naphtha cracking fraction satisfies the expression (α):

$$-0.25 < (P/E_E - P/E_C) < 0.30 \ldots (\alpha), \text{ and}$$

a relationship between a content $C_{E_AP}$ of ethylene and propylene in the ethanol conversion fraction and a content $C_{C_AP}$ of ethylene and propylene in the naphtha cracking fraction satisfies the expression (β-1):

$$|C_{E_AP} - C_{C_AP}| < 20\% \text{ by mass ... } (\beta\text{-}1).$$

The expression ($\alpha$) and the expression ($\beta$-1) are thus satisfied at the same time, whereby the operation rate of a refining system can be stabilized. For satisfying the expression ($\alpha$) and the expression ($\beta$-1) at the same time, it is preferred to adjust a reaction temperature (particularly, a reactor outlet temperature), the amount of a catalyst, and the catalyst in the ethanol conversion step. The ethanol conversion step compared with the naphtha cracking step has a large fluctuation range of each variable ($P/E_E$, $C_{E_AP}$, etc.) depending on change in these conditions. Therefore, change in conditions of the ethanol conversion step is more effective.

In the naphtha cracking process, as mentioned above, light olefin is refined by cryogenic separation. A problem such as the surging of a compressor or the flooding of a distillation tower arises due to increase or decrease in operation rate in each refining equipment if the ratio among propylene, ethylene, and other components to be introduced to the cryogenic separation step differs largely from that before introduction of at least a portion of the ethanol conversion fraction or a fraction derived therefrom. Thus, efficient refining of light olefin requires keeping an operation rate constant in each refining equipment. In other words, the relationship between the propylene/ethylene mass ratio $P/E_E$ in the ethanol conversion fraction and the propylene/ethylene mass ratio P/Ec in the naphtha cracking fraction falls within the range as mentioned above in the expression ($\alpha$), and the relationship between the content $C_{E_AP}$ of ethylene and propylene in the ethanol conversion fraction and the content $C_{C_AP}$ of ethylene and propylene in the naphtha cracking fraction falls within the range as mentioned above the expression ($\beta$-1), whereby efficient refining of light olefin can be carried out.

In the method for producing light olefin according to the embodiment B, the relationship between the content $C_{E_AP}$ of ethylene and propylene in the ethanol conversion fraction and the content $C_{C_AP}$ of ethylene and propylene in the naphtha cracking fraction preferably satisfies the expression ($\beta$-2), because of excellent light olefin refining efficiency:

$$0 \leq (C_{E_AP} - C_{C_AP}) < 20\% \text{ by mass ... } (\beta\text{-}2).$$

In the method for producing light olefin according to the embodiment A as well, the relationship between the propylene/ethylene mass ratio $P/E_E$ in the ethanol conversion fraction and the propylene/ethylene mass ratio P/Ec in the naphtha cracking fraction preferably satisfies the expression ($\alpha$) mentioned above, because load in propylene refining equipment or ethylene refining equipment is kept constant without impairing light olefin refining efficiency. Also, the relationship between the content $C_{E_AP}$ of ethylene and propylene in the ethanol conversion fraction and the content $C_{C_AP}$ of ethylene and propylene in the naphtha cracking fraction preferably satisfies the expression ($\beta$-1) mentioned above.

The naphtha cracking fraction produced by the naphtha cracking process N is substituted with the ethanol conversion fraction produced by the ethanol conversion process E, whereby, particularly, in the case of using bioethanol as a starting material, a petroleum feedstock can be converted to a biomass feedstock. On the other hand, the amount of each product produced varies with increase in the rate of substitution with the ethanol conversion fraction, resulting in a problem such as the surging of a compressor or the flooding of a distillation tower arising in refining equipment. When the expression ($\alpha$) or the expression ($\beta$-1) mentioned above is satisfied, the rate of substitution with the ethanol conversion fraction can be elevated while the problem mentioned above is prevented. The rate of substitution with the ethanol conversion fraction is preferably 10% by mass or more, more preferably 20% by mass or more, further preferably 30% by mass or more, because of excellent environmental friendliness. The rate of substitution with the ethanol conversion fraction is calculated according to the following expression.

(Rate of substitution with the ethanol conversion fraction) = (Amount of ethylene produced in the ethanol conversion fraction) / [(Amount of ethylene produced in the ethanol conversion fraction) + (Amount of ethylene produced in the naphtha cracking fraction)]

The embodiment A and the embodiment B relate to the composition in any step of products of the cracking step and the ethanol conversion step. The refining step for each fraction having such composition, i.e., how to combine and refine the cracking step-derived fraction and the ethanol conversion step-derived fraction, is not particularly limited. For example, the first embodiment, the second embodiment, the third embodiment, the fourth embodiment, and the fifth embodiment given below may be used in arbitrary combination.

The method for producing light olefin according to the first embodiment comprises:

a cracking step of introducing a naphtha feedstock to a cracking furnace to obtain a naphtha cracking fraction;

a first cooling step of introducing the naphtha cracking fraction to a first cooling tower to obtain a first cooling fraction mainly containing olefin having 6 or less carbon atoms;

a second cooling step of introducing the first cooling fraction to a second cooling tower to obtain a second cooling fraction mainly containing olefin having 4 or less carbon atoms;

an ethanol conversion step of introducing a starting material containing ethanol to a reactor where the starting material is brought into contact with a catalyst to obtain an ethanol conversion fraction containing ethylene and propylene;

a combination step of combining at least a portion of the second cooling fraction with at least a portion of the ethanol conversion fraction or a fraction derived therefrom to obtain a combination fraction; and

a cryogenic separation step of introducing the combination fraction or a fraction derived therefrom to a cryogenic separation equipment to separate ethylene and propylene.

In the method for producing light olefin according to the first embodiment, a relationship between a content $C_E$ of ethylene and propylene in the ethanol conversion fraction or a fraction derived therefrom and a content $C_C$ of ethylene and propylene in the naphtha cracking fraction or a fraction derived therefrom in the combination step preferably satisfies the expression (A):

$$C_E > C_C \ldots (A).$$

As a result, the method for producing light olefin according to the first embodiment can enhance the treatment efficiency of olefin having 2 or 3 carbon atoms. The expression (A) and preferred aspects are as shown in the embodiment A mentioned above.

In the method for producing light olefin according to the first embodiment, a relationship between a propylene/ethylene mass ratio $P/E_E$ in the ethanol conversion fraction and a propylene/ethylene mass ratio $P/E_C$ in the naphtha cracking fraction preferably satisfies the expression ($\alpha$):

$$-0.25 < (P/E_E - P/E_C) < 0.30 \ldots (\alpha),$$

and
a relationship between a content $C_{E_AP}$ of ethylene and propylene in the ethanol conversion fraction and a content $C_{C_AP}$ of ethylene and propylene in the naphtha cracking fraction preferably satisfies the expression ($\beta$-1):

$$|C_{E_AP} - C_{C_AP}| < 20\% \text{ by mass} \ldots (\beta\text{-}1).$$

As a result, the method for producing light olefin according to the first embodiment can stabilize the operation rate of a refining system. The expression ($\alpha$), the expression ($\beta$-1), and preferred aspects are as shown in the embodiment B mentioned above.

As shown in the combination step according to the first embodiment, after production of the naphtha cracking fraction, components having more than 6 carbon atoms are removed in the first cooling step, and components having more than 4 carbon atoms are further removed in the second cooling step, and the resulting second cooling fraction is combined with the ethanol conversion fraction or a fraction derived therefrom. When light olefin produced by naphtha cracking is substituted with ethanol conversion and combined as mentioned above, mass flow rates to be introduced to the first cooling step and the second cooling step are reduced as compared with those before substitution, and the amount of a catalytic refrigerant necessary for the cooling is reduced, whereby energy necessary for treatment in these steps can be reduced. When such combination is added to light olefin produced by naphtha cracking, the amount of light olefin produced can be increased without increasing load in the first cooling step and the second cooling step.

The method for producing light olefin according to the second embodiment comprises:

a cracking step of introducing a naphtha feedstock to a cracking furnace to obtain a naphtha cracking fraction;

a first cooling step of introducing the naphtha cracking fraction to a first cooling tower to obtain a first cooling fraction mainly containing olefin having 6 or less carbon atoms;

an ethanol conversion step of introducing a starting material containing ethanol to a reactor where the starting material is brought into contact with a catalyst to obtain an ethanol conversion fraction containing ethylene and propylene;

a combination step of combining at least a portion of the first cooling fraction with at least a portion of the ethanol

conversion fraction or a fraction derived therefrom to obtain a combination fraction;

a second cooling step of introducing the combination fraction or a fraction derived therefrom to a second cooling tower to obtain a second cooling fraction mainly containing olefin having 4 or less carbon atoms; and

a cryogenic separation step of introducing the combination fraction or a fraction derived therefrom to a cryogenic separation equipment to separate ethylene and propylene.

In the method for producing light olefin according to the second embodiment, a relationship between a content $C_E$ of ethylene and propylene in the ethanol conversion fraction or a fraction derived therefrom and a content Cc of ethylene and propylene in the naphtha cracking fraction or a fraction derived therefrom in the combination step preferably satisfies the expression (A):

$$C_E > C_C \ldots (A).$$

As a result, the method for producing light olefin according to the second embodiment can enhance the treatment efficiency of olefin having 2 or 3 carbon atoms. The expression (A) and preferred aspects are as shown in the embodiment A mentioned above.

In the method for producing light olefin according to the second embodiment, a relationship between a propylene/ethylene mass ratio $P/E_E$ in the ethanol conversion fraction and a propylene/ethylene mass ratio P/Ec in the naphtha cracking fraction preferably satisfies the expression ($\alpha$):

$$-0.25 < (P/E_E - P/E_C) < 0.30 \ldots (\alpha), \text{ and}$$

a relationship between a content $C_{E_AP}$ of ethylene and propylene in the ethanol conversion fraction and a content $C_{C_AP}$ of ethylene and propylene in the naphtha cracking fraction preferably satisfies the expression ($\beta$-1):

$$|C_{E_AP} - C_{C_AP}| < 20\% \text{ by mass} \ldots (\beta\text{-}1).$$

As a result, the method for producing light olefin according to the second embodiment can stabilize the operation rate of a refining system. The expression ($\alpha$), the expression ($\beta$-1), and preferred aspects are as shown in the embodiment B mentioned above.

In the method for producing light olefin according to the second embodiment, before introduction to the second cooling tower, at least a portion of the first cooling fraction is combined with at least a portion of the ethanol conversion fraction or a fraction derived therefrom. Since at least a portion of components having more than 4 carbon atoms contained in a fraction derived from the ethanol conversion fraction is removed in the second cooling step, the proportion of ethylene and propylene in the fraction to be introduced to cryogenic separation can be further elevated. For example, when the ethanol conversion fraction or a fraction derived therefrom contains a hydrophilic by-product such as ethanol, the ethanol conversion fraction or the fraction derived therefrom is introduced to the second cooling tower with water as a catalytic refrigerant, whereby the hydrophilic by-product can be extracted, and the purity of light olefin can be improved.

The method for producing light olefin according to the third embodiment comprises:

a cracking step of introducing a naphtha feedstock to a cracking furnace to obtain a naphtha cracking fraction;

a first cooling step of introducing the naphtha cracking fraction to a first cooling tower to obtain a first cooling fraction mainly containing olefin having 6 or less carbon atoms;

a second cooling step of introducing the first cooling fraction to a second cooling tower to obtain a second cooling fraction mainly containing olefin having 4 or less carbon atoms;

a circulation step of introducing at least a portion of a second heavy fraction mainly containing hydrocarbon having 5 or more carbon atoms in the second cooling step to the second cooling tower;

an ethanol conversion step of introducing a starting material containing ethanol to a reactor where the starting material is brought into contact with a catalyst to obtain an ethanol conversion fraction containing ethylene and propylene;

a combination step of combining at least a portion of the second heavy fraction with at least a portion of the ethanol

conversion fraction or a fraction derived therefrom to obtain a combination fraction;

a first introduction step of introducing the combination fraction to the second cooling tower; and

a cryogenic separation step of introducing the second cooling fraction or a fraction derived therefrom to a cryogenic separation equipment to separate ethylene and propylene.

In the method for producing light olefin according to the third embodiment, a relationship between a content $C_E$ of ethylene and propylene in the ethanol conversion fraction or a fraction derived therefrom and a content $C_C$ of ethylene and propylene in the naphtha cracking fraction or a fraction derived therefrom in the combination step preferably satisfies the expression (A):

$$C_E > C_C \ldots (A).$$

As a result, the method for producing light olefin according to the third embodiment can enhance the treatment efficiency of olefin having 2 or 3 carbon atoms. The expression (A) and preferred aspects are as shown in the embodiment A mentioned above.

In the method for producing light olefin according to the third embodiment, a relationship between a propylene/ethylene mass ratio $P/E_E$ in the ethanol conversion fraction and a propylene/ethylene mass ratio P/Ec in the naphtha cracking fraction preferably satisfies the expression ($\alpha$):

$$-0.25 < (P/E_E - P/E_C) < 0.30 \ldots (\alpha),$$

and
a relationship between a content $C_{E_AP}$ of ethylene and propylene in the ethanol conversion fraction and a content $C_{C_AP}$ of ethylene and propylene in the naphtha cracking fraction preferably satisfies the expression ($\beta$-1):

$$|C_{E_AP} - C_{C_AP}| < 20\% \text{ by mass} \ldots (\beta\text{-}1).$$

As a result, the method for producing light olefin according to the third embodiment can stabilize the operation rate of a refining system. The expression ($\alpha$), the expression ($\beta$-1), and preferred aspects are as shown in the embodiment B mentioned above.

In the method for producing light olefin according to the third embodiment, at least a portion of the second heavy fraction in the circulation step is combined with at least a portion of the ethanol conversion fraction or a fraction derived therefrom. Since at least a portion of components having more than 4 carbon atoms contained in a fraction derived from the ethanol conversion fraction is removed in the second cooling step after the combination step, the proportion of ethylene and propylene in the fraction to be introduced to cryogenic separation can be further elevated. In the circulation step, at least a portion of the second heavy fraction is combined with at least a portion of the ethanol conversion fraction or a fraction derived therefrom, whereby the difference in temperature between both the components is reduced as compared with the case where at least a portion of the first cooling fraction is combined with at least a portion of the ethanol conversion fraction or a fraction derived therefrom, and change in volume caused by the liquefication of the second heavy fraction can be suppressed.

The method for producing light olefin according to the fourth embodiment comprises:

a cracking step of introducing a naphtha feedstock to a cracking furnace to obtain a naphtha cracking fraction;

an ethanol conversion step of introducing a starting material containing ethanol to a reactor where the starting material is brought into contact with a catalyst to obtain an ethanol conversion fraction containing ethylene and propylene;

a combination step of combining at least a portion of the naphtha cracking fraction with at least a portion of the ethanol conversion fraction or a fraction derived therefrom to obtain a combination fraction;

a first cooling step of introducing the combination fraction or a fraction derived therefrom to a first cooling tower to obtain a first cooling fraction mainly containing olefin having 6 or less carbon atoms;

a second cooling step of introducing the first cooling fraction to a second cooling tower to obtain a second cooling fraction mainly containing olefin having 4 or less carbon atoms; and

a cryogenic separation step of introducing the second cooling fraction to a cryogenic separation equipment to separate ethylene and propylene.

In the method for producing light olefin according to the fourth embodiment, a relationship between a content $C_E$ of ethylene and propylene in the ethanol conversion fraction or a fraction derived therefrom and a content Cc of ethylene and propylene in the naphtha cracking fraction or a fraction derived therefrom in the combination step preferably satisfies the expression (A):

$$C_E > C_C \ldots (A).$$

As a result, the method for producing light olefin according to the fourth embodiment can enhance the treatment efficiency of olefin having 2 or 3 carbon atoms. The expression (A) and preferred aspects are as shown in the embodiment A mentioned above.

In the method for producing light olefin according to the fourth embodiment, a relationship between a propylene/ethylene mass ratio $P/E_E$ in the ethanol conversion fraction and a propylene/ethylene mass ratio P/Ec in the naphtha cracking fraction preferably satisfies the expression ($\alpha$):

$$-0.25 < (P/E_E - P/E_C) < 0.30 \ldots (\alpha),$$

and

a relationship between a content $C_{E_AP}$ of ethylene and propylene in the ethanol conversion fraction and a content $C_{C_AP}$ of ethylene and propylene in the naphtha cracking fraction preferably satisfies the expression ($\beta$-1):

$$|C_{E_AP} - C_{C_AP}| < 20\% \text{ by mass} \ldots (\beta\text{-}1).$$

As a result, the method for producing light olefin according to the fourth embodiment can stabilize the operation rate of a refining system. The expression ($\alpha$), the expression ($\beta$-1), and preferred aspects are as shown in the embodiment B mentioned above.

In the method for producing light olefin according to the fourth embodiment, before introduction to the first cooling tower, at least a portion of the naphtha cracking fraction is combined with at least a portion of the ethanol conversion fraction or a fraction derived therefrom. Since at least a portion of components having more than 4 carbon atoms contained in a fraction derived from the ethanol conversion fraction is removed in the first cooling step and the second cooling step after the combination step, the proportion of ethylene and propylene in the fraction to be introduced to cryogenic separation can be further elevated. For example, when the ethanol conversion fraction or a fraction derived therefrom contains a lipophilic by-product, the ethanol conversion fraction or the fraction derived therefrom is introduced to the first cooling tower with heavy oil, cracked gasoline, or their mixture as a catalytic refrigerant, whereby the lipophilic by-product can be extracted, and the purity of light olefin can be improved. For example, when the ethanol conversion fraction or a fraction derived therefrom contains a hydrophilic by-product such as ethanol, the ethanol conversion fraction or the fraction derived therefrom is introduced to the second cooling tower with water as a catalytic refrigerant, whereby the hydrophilic by-product can be extracted, and the purity of light olefin can be improved.

The method for producing light olefin according to the fifth embodiment comprises:

a cracking step of introducing a naphtha feedstock to a cracking furnace to obtain a naphtha cracking fraction;

a first cooling step of introducing the naphtha cracking fraction to a first cooling tower to obtain a first cooling fraction mainly containing olefin having 6 or less carbon atoms;

a second cooling step of introducing the first cooling fraction to a second cooling tower to obtain a second cooling fraction mainly containing olefin having 4 or less carbon atoms;

a circulation step of introducing at least a portion of a second heavy fraction mainly containing hydrocarbon having 5 or more carbon atoms in the second cooling step to the first cooling tower;

an ethanol conversion step of introducing a starting material containing ethanol to a reactor where the starting material is brought into contact with a catalyst to obtain an ethanol conversion fraction containing ethylene and propylene;

a combination step of combining at least a portion of the second heavy fraction with at least a portion of the ethanol

conversion fraction or a fraction derived therefrom to obtain a combination fraction;

a second introduction step of introducing the combination fraction to the first cooling tower; and

a cryogenic separation step of introducing the second cooling fraction or a fraction derived therefrom to a cryogenic separation equipment to separate ethylene and propylene.

In the method for producing light olefin according to the fifth embodiment, a relationship between a content $C_E$ of ethylene and propylene in the ethanol conversion fraction or a fraction derived therefrom and a content Cc of ethylene and propylene in the naphtha cracking fraction or a fraction derived therefrom in the combination step preferably satisfies the expression (A):

$$C_E > C_C \ldots (A).$$

As a result, the method for producing light olefin according to the fifth embodiment can enhance the treatment efficiency of olefin having 2 or 3 carbon atoms. The expression (A) and preferred aspects are as shown in the embodiment A mentioned above.

In the method for producing light olefin according to the fifth embodiment, a relationship between a propylene/ethylene mass ratio $P/E_E$ in the ethanol conversion fraction and a propylene/ethylene mass ratio P/Ec in the naphtha cracking fraction preferably satisfies the expression ($\alpha$):

$$-0.25 < (P/E_E - P/E_C) < 0.30 \ldots (\alpha),$$

and

a relationship between a content $C_{E_AP}$ of ethylene and propylene in the ethanol conversion fraction and a content $C_{C_AP}$ of ethylene and propylene in the naphtha cracking fraction preferably satisfies the expression ($\beta$-1):

$$|C_{E_AP} - C_{C_AP}| < 20\% \text{ by mass} \ldots (\beta\text{-}1).$$

As a result, the method for producing light olefin according to the fifth embodiment can stabilize the operation rate of a refining system. The expression ($\alpha$), the expression ($\beta$-1), and preferred aspects are as shown in the embodiment B mentioned above.

In the method for producing light olefin according to the fifth embodiment, at least a portion of the second heavy fraction in the circulation step is combined with at least a portion of the ethanol conversion fraction or a fraction derived therefrom. At least a portion of the second heavy fraction in the circulation step is combined with at least a portion of the ethanol conversion fraction or a fraction derived therefrom, whereby the difference in temperature between both the components is reduced as compared with the case of being connected to at least a portion of the naphtha cracking fraction, and change in volume caused by the liquefication of the second weight fraction can be suppressed. Since components having more than 4 carbon atoms contained in a fraction derived from the ethanol conversion fraction are removed in the first cooling step and the second cooling step, the proportion of ethylene and propylene in the fraction to be introduced to cryogenic separation can be further elevated. For example, when the ethanol conversion fraction or a fraction derived therefrom contains a lipophilic by-product, the ethanol conversion fraction or the fraction derived therefrom is introduced to the first cooling tower with heavy oil, cracked gasoline, or their mixture as a catalytic refrigerant, whereby the lipophilic by-product can be extracted, and the purity of light olefin can be improved. For example, when the ethanol conversion fraction or a fraction derived therefrom contains a hydrophilic by-product such as ethanol, the ethanol conversion fraction or the fraction derived therefrom is introduced to the second cooling tower with water as a catalytic refrigerant, whereby the hydrophilic by-product can be extracted, and the purity of light olefin can be improved.

When the method for producing light olefin according to the present embodiment has a first compression step after the second cooling step and before the cryogenic separation step, the combination step according to the present embodiment enables the ethanol conversion fraction or a fraction derived therefrom to be also pressure-boosted in the first compression step and therefore reduces equipment introduction load in introducing the ethanol conversion fraction to the naphtha cracking process. The combination fraction is easily pressure-adjusted by pressure-boosting the naphtha cracking fraction or a fraction derived therefrom and the ethanol conversion fraction or a fraction derived therefrom in the same compression step.

In the present embodiment, when a relationship of the expression (1):

$$O_E < O_C \ldots (1)$$

is satisfied, the ethanol conversion fraction has a small amount of off-gases. Therefore, ethanol conversion fraction refining load is reduced, and the ethanol conversion fraction or a fraction derived therefrom can be introduced to the naphtha cracking process by convenient refining.

The method for producing light olefin according to the present embodiment preferably further comprises a refining step of refining the ethanol conversion fraction such that an ethylene and propylene content $C_{E1}$ after refining is higher than an ethylene and propylene content in the naphtha cracking fraction or a fraction derived therefrom in the combination step, to obtain a refined ethanol conversion fraction. This step elevates the ethylene and propylene content $C_{E1}$ in a fraction derived from the ethanol conversion fraction to be combined in the combination step, and can reduce energy load, particularly, in the cryogenic separation step. The production method according to the present embodiment preferably has the refining step after the ethanol conversion step and before the combination step.

The refining step preferably comprises a third cooling step of introducing the ethanol conversion fraction to a third cooling tower to obtain a cooled ethanol conversion fraction mainly containing olefin having 6 or less carbon atoms. The cooled ethanol conversion fraction is a fraction from which heavy components have been removed with the third cooling tower, and is preferably, for example, a fraction obtained from the tower top of the third cooling tower. The third cooling step can elevate the ethylene and propylene content $C_{E1}$ in a fraction derived from the ethanol conversion fraction to be combined in the combination step, and can reduce substances that cause phase transition from a gas to a liquid in the cryogenic separation step. Therefore, the generation of condensation heat can be prevented, and energy load for cooling in the cryogenic separation step can be reduced.

The refining step preferably comprises a compression and separation step of pressure-boosting the cooled ethanol conversion fraction using a compressor to obtain a light ethanol conversion fraction mainly containing olefin having 3 or less carbon atoms as a gas component and to obtain a heavy ethanol conversion fraction mainly containing olefin having 4 or more carbon atoms as a liquid component. The compression and separation step can elevate the ethylene and propylene content $C_{E1}$ in a fraction derived from the ethanol conversion fraction to be combined in the combination step, and can reduce substances that cause phase transition from a gas to a liquid in the cryogenic separation step. Therefore, the generation of condensation heat can be prevented, and energy load for cooling in the cryogenic separation step can be reduced. The compression and separation step can also reduce the amount of a fraction derived from the ethanol conversion fraction to be combined in the combination step and can reduce the flow rate of a fraction to be pressure-boosted and cooled in the cryogenic separation step. Thus, energy load can be reduced in the cryogenic separation step.

The method for producing light olefin according to the present embodiment preferably comprises a recycling step of introducing at least a portion of the heavy ethanol conversion fraction as a portion of the starting material to the reactor. The heavy ethanol conversion fraction mainly containing olefin having 4 or more carbon atoms is introduced to the reactor for use in the ethanol conversion step, whereby the amount of ethanol introduced in the ethanol conversion step can be decreased. Particularly, since the heavy ethanol conversion fraction contains highly reactive olefin having 4 or more carbon atoms, such a component causes further conversion reaction via a catalyst in the ethanol conversion step and may also be converted to ethylene and propylene. Since the heavy ethanol conversion fraction is free from heavy components having 9 or more carbon atoms through the third cooling step mentioned above, this fraction is unlikely to become responsible for coke generation in the reactor even if introduced to the reactor for use in the ethanol conversion step. Thus, the starting material can be effectively utilized while the activity of the catalyst is maintained.

The method for producing light olefin according to the present embodiment preferably comprises, after the combination step, a washing step of introducing the combination fraction to a soda washing tower to obtain a washing fraction. The washing fraction is a fraction from which components eluted to the washes have been removed with the soda washing tower, and is preferably, for example, a fraction obtained from the tower top of the soda washing tower. The cryogenic separation step is performed through this step, whereby an acidic gas such as carbon dioxide or hydrogen sulfide contained in the combination fraction can be removed, and the purity of light olefin obtained in the cryogenic separation step can be improved.

Hereinafter, each step of the method for producing light olefin according to the present embodiment will be described.

The method for producing light olefin according to the present embodiment comprises, as the naphtha cracking process N mentioned above:

a cracking step of introducing a naphtha feedstock to a cracking furnace to obtain a naphtha cracking fraction; and

a cryogenic separation step of introducing the fraction to a cryogenic separation equipment to separate ethylene and propylene.

The method for producing light olefin according to the present embodiment may optionally have, as the naphtha cracking process N mentioned above:

a first cooling step of introducing the naphtha cracking fraction to a first cooling tower to obtain a first cooling fraction mainly containing olefin having 6 or less carbon atoms; and

a second cooling step of introducing the first cooling fraction to a second cooling tower to obtain a second cooling fraction mainly containing olefin having 4 or less carbon atoms.

The first cooling step and/or the second cooling step established prior to a washing step mentioned later can reduce the fraction to be introduced to a soda washing tower and can improve washing efficiency in the soda washing tower. The cooling step established in two divided stages facilitates controlling the temperature or composition of a fraction obtained in the cooling step.

The method for producing light olefin according to the present embodiment may optionally have, as the naphtha cracking process N mentioned above, before introduction to the cryogenic separation equipment:

a first compression step of pressure-boosting the fraction; and

a washing step of introducing the fraction to a soda washing tower to obtain a washing fraction.

In the soda washing tower, an acid component such as carbon dioxide contained in the fraction can be removed, whereby separation efficiency in the cryogenic separation step can be improved. The first compression step established prior to the washing step can compress the volume of the fraction to be introduced to the soda washing tower and can improve operation efficiency in the soda washing tower.

The method for producing light olefin according to the present embodiment comprises, as the ethanol conversion process E mentioned above,
an ethanol conversion step of introducing a starting material containing ethanol to a reactor where the starting material is brought into contact with a catalyst to obtain an ethanol conversion fraction containing ethylene and propylene.

The method for producing light olefin according to the present embodiment may optionally have, as the ethanol conversion process E mentioned above,
a refining step of refining the ethanol conversion fraction to obtain a refined ethanol conversion fraction.

The established refining step can increase the concentration of the compound of interest contained in the ethanol conversion fraction and improves refining efficiency in the cryogenic separation step.

The method for producing light olefin according to the present embodiment may optionally have, as the refining step in the ethanol conversion process E mentioned above,
a third cooling step of introducing the ethanol conversion fraction to a third cooling tower to obtain a cooled ethanol conversion fraction mainly containing olefin having 6 or less carbon atoms.

The established third cooling step can reduce the amount of hydrocarbon having 7 or more carbon atoms contained in the ethanol conversion fraction or a fraction derived therefrom and improves refining efficiency in the cryogenic separation step.

The method for producing light olefin according to the present embodiment may optionally have, as the refining step in the ethanol conversion process E mentioned above,
a compression and separation step of pressure-boosting the cooled ethanol conversion fraction using a compressor to obtain a light ethanol conversion fraction mainly containing olefin having 3 or less carbon atoms as a gas component and to obtain a heavy ethanol conversion fraction mainly containing olefin having 4 or more carbon atoms as a liquid component.

The established compression and separation step can reduce the amount of hydrocarbon having 4 or more carbon atoms contained in the ethanol conversion fraction or a fraction derived therefrom and improves refining efficiency in the cryogenic separation step.

The method for producing light olefin according to the present embodiment may optionally have, as the ethanol conversion process E mentioned above,
a recycling step of introducing at least a portion of the aforementioned heavy ethanol conversion fraction as a portion of the starting material to the reactor.

The established recycling step can convert the olefin contained in the heavy ethanol conversion fraction to the compound of interest and improves ethylene and propylene production efficiency per starting material ethanol.

The method for producing light olefin according to the present embodiment comprises
a combination step of combining at least a portion of the naphtha cracking fraction or a fraction derived therefrom with at least a portion of the ethanol conversion fraction or a fraction derived therefrom to obtain a combination fraction.

In other words, at least a portion of the ethanol conversion fraction or a fraction derived therefrom is combined

with the naphtha cracking process, whereby cryogenic separation is efficiently performed while an ethanol-derived starting material is used.

The combination step is performed at any stage in the naphtha cracking process, as mentioned later.

The embodiments mentioned above are described in accordance with the general categories of the naphtha cracking process N and the ethanol conversion process E in the technical field in order to describe the organization of each step. However, each step according to the present embodiment may belong to any of the naphtha cracking process N and the ethanol conversion process E.

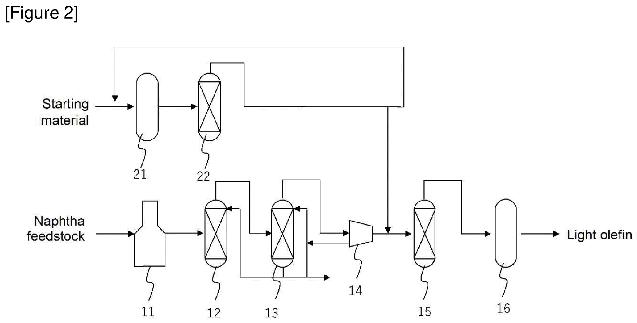

The method for producing light olefin according to the present embodiment is achieved with, for example, production equipment shown in Figure 2. The light olefin production equipment has a cracking furnace 11, a first cooling tower 12, a second cooling tower 13, a first compressor 14, a soda washing tower 15, and a cryogenic separation equipment 16. The light olefin production equipment also has a reactor 21 and a third cooling tower 22.

Hereinafter, each step according to the present embodiment will be described in detail by taking the case of using the production equipment as an example.

<Cracking step>

In the cracking furnace 11, the cracking step mentioned above is performed.

(Naphtha feedstock)

In the method for producing light olefin according to the present embodiment, a naphtha feedstock is used. The naphtha feedstock contains at least naphtha. The naphtha is a hydrocarbon mixture with a boiling point range on the order of 30 to 230°C and is divided into light naphtha and heavy naphtha depending on the boiling point range, either of which may be used. The naphtha feedstock preferably contains hydrocarbon having 2 to 40 carbon atoms, more preferably contains hydrocarbon having 2 to 30 carbon atoms, and further preferably contains hydrocarbon having 5 to 12 carbon atoms. The boiling point of the naphtha feedstock may be in the range of, for example, 30 to 230°C. The naphtha feedstock is not particularly limited and is generally derived from a fossil resource obtained from petroleum. Alternatively, bionaphtha obtained by the decomposition of an animal or plant oil may be used.

The naphtha feedstock preferably contains diluted steam from the viewpoint of being able to reduce the amount of a by-product coke. The amount of the diluted steam supplied is preferably 0.1 to 1.0, more preferably 0.3 to 0.6, in terms of a mass ratio to naphtha.

Examples of the cracking method in the cracking step include, but are not particularly limited to, a steam cracking method of thermally decomposing heated naphtha by bringing the heated naphtha into contact with steam heated to 950°C or higher, and a moving-bed method of thermally decomposing naphtha by bringing the naphtha into contact with a granular heat medium heated to a high temperature in a moving bed. Examples of the steam cracking method include a tubular heating method of circulating naphtha and steam in a heating tube and thermally decomposing the naphtha by heating from the wall of the tube. In each method, a catalyst may be used for promoting thermal decomposition.

The naphtha feedstock is introduced to the cracking furnace 11, and steam may be further introduced together with the naphtha feedstock. In this respect, the amount of the steam introduced is preferably 20 to 100 parts by mass, more preferably 30 to 70 parts by mass, more preferably 35 to 60 parts by mass, per 100 parts by mass of the naphtha feedstock. The amount of the steam introduced falls within the range mentioned above, whereby the production of a carbonaceous substance can be suppressed.

The treatment temperature in the cracking furnace is preferably 700 to 1000°C, more preferably 750 to 950°C, further preferably 800 to 900°C. Within the temperature range, the thermal decomposition of naphtha progresses favorably, and the production of a light component such as methane can be suppressed. The treatment temperature is controlled depending on the boiling point of the naphtha feedstock, whereby cracking severity is controlled, and the ratio between ethylene and propylene can be set to a suitable range. The mass ratio of propylene to ethylene (P/E mass ratio) in naphtha cracking is not particularly limited and is preferably 0.3 to 1.0.

The reaction pressure in the cracking furnace is preferably 0.01 to 1.5 MPaG, more preferably 0.05 to 0.5 MPaG, further preferably 0.07 to 0.2 MPaG.

<First cooling step>

The naphtha cracking fraction obtained in the cracking furnace 11 is introduced to the first cooling tower 12. Before being introduced to the first cooling tower 12, the naphtha cracking fraction may be mixed with light olefin such as ethane or propane recycled from downstream refining equipment. In the first cooling tower 12, the first cooling step mentioned above is performed to obtain a first cooling fraction mainly containing olefin having 6 or less carbon atoms. In the first cooling tower 12, the naphtha cracking fraction is cooled, whereby excessive progression of cracking can be

suppressed, and further, at least a portion of olefin having more than 6 carbon atoms is removed.

In the first cooling tower 12, the naphtha cracking fraction is preferably brought into contact with heavy oil, cracked gasoline, or their mixture and thereby cooled.

The pressure of the first cooling fraction after treatment with the first cooling tower 12 is preferably 0.01 to 1.5 MPaG, more preferably 0.05 to 0.5 MPaG, further preferably 0.07 to 0.2 MPaG.

The temperature of the first cooling fraction after treatment with the first cooling tower 12 is preferably 50 to 300°C, more preferably 100 to 200°C, further preferably 105 to 160°C, from the viewpoint of excellent efficiency of removal of olefin having more than 6 carbon atoms.

<Second cooling step>

The first cooling fraction obtained in the first cooling tower 12 is introduced to the second cooling tower 13. In the second cooling tower 13, the second cooling step mentioned above is performed, and the first cooling fraction is cooled so that at least a portion of olefin having more than 4 carbon atoms is removed to obtain a second cooling fraction mainly containing olefin having 4 or less carbon atoms. In this operation, a second heavy fraction mainly containing hydrocarbon having 5 or more carbon atoms and water are mainly obtained from the tower bottom.

In the second cooling tower 13, the first cooling fraction is preferably brought into contact with water and thereby cooled.

The pressure of the second cooling fraction after treatment with the second cooling tower 13 is preferably 0.01 to 1.5 MPaG, more preferably 0.05 to 0.5 MPaG, further preferably 0.07 to 0.2 MPaG.

The temperature of the second cooling fraction after treatment with the second cooling tower 13 is preferably 10 to 100°C, more preferably 30 to 90°C, from the viewpoint of excellent efficiency of removal of olefin having more than 4 carbon atoms.

<First compression step>

The second cooling fraction obtained in the second cooling tower 13 is introduced to the first compressor 14. In the first compressor 14, the compression step mentioned above is performed, and the second cooling fraction is pressure-boosted. The first compression step can be performed before introduction to the cryogenic separation equipment, and a fraction including the second cooling fraction is preferably introduced to the first compressor 14 after the second cooling step. In the first compressor 14, for example, the second cooling fraction may be pressure-boosted with one compressor, or the second cooling fraction may be pressure-boosted in stages with a plurality of, for example, two to four compressors. In the first compression step, the second cooling fraction can be pressure-boosted as a gas component. In addition, a liquid component may be obtained by the liquefication of high-boiling components, such as hydrocarbon having 5 or more carbon atoms, contained in the second cooling fraction. Such removal of high-boiling components by pressure boosting can improve the concentration of olefin having 3 or less carbon atoms in the second cooling fraction.

The pressure of the second cooling fraction after treatment with the first compressor 14 is preferably 0.10 to 10.0 MPaG, more preferably 0.20 to 5.0 MPaG, further preferably 0.50 to 1.5 MPaG.

<First circulation step>

The method for producing light olefin according to the present embodiment may have a first circulation step of introducing at least a portion of a second heavy fraction mainly containing hydrocarbon having 5 or more carbon atoms in the second cooling step to the first cooling tower. The second heavy fraction contains the heavy compound of interest such as an aromatic compound. The production efficiency of such a heavy compound of interest is reduced when the amount of the second heavy fraction circulated to the first cooling tower in the first circulation step is increased. On the other hand, a portion of olefin having 4 or less carbon atoms contained in the second heavy fraction can be introduced to the cryogenic separation step by increasing the amount of the fraction circulated. Therefore, the production efficiency of such olefin is improved. Thus, the amount of the fraction circulated in the first circulation step is preferably determined in consideration of the production balance between the heavy compound of interest such as an aromatic compound and the olefin having 4 or less carbon atoms. The amount of the fraction circulated in the first circulation step is preferably 20 to 90% by mass, more preferably 30 to 90% by mass, further preferably 40 to 90% by mass, based on the second heavy fraction from the viewpoint of excellent propylene production efficiency.

<Second circulation step>

The method for producing light olefin according to the present embodiment may have a second circulation step of introducing at least a portion of a second heavy fraction mainly containing hydrocarbon having 5 or more carbon atoms in

the second cooling step to the second cooling tower. In this circulation step, a liquid component generated in the first compression step mentioned above may be combined therewith and introduced to the second cooling tower. The second heavy fraction contains the heavy compound of interest such as an aromatic compound. The production efficiency of such a heavy compound of interest is reduced when the amount of the second heavy fraction circulated to the first cooling tower in the second circulation step is increased. On the other hand, a portion of olefin having 4 or less carbon atoms contained in the second heavy fraction can be introduced to the cryogenic separation step by increasing the amount of the fraction circulated. Therefore, the production efficiency of such olefin is improved. Thus, the amount of the fraction circulated in the second circulation step is preferably determined in consideration of the production balance between the heavy compound of interest such as an aromatic compound and the olefin having 4 or less carbon atoms. The amount of the fraction circulated in the second circulation step is preferably 20 to 90% by mass, more preferably 30 to 90% by mass, further preferably 40 to 90% by mass, based on the total amount of the liquid component in the first compression step and the second heavy fraction from the viewpoint of excellent propylene production efficiency.

<Washing step>

In the washing step, the fraction before being introduced to the cryogenic separation equipment is introduced to the soda washing tower 15 to obtain a washing fraction. In the washing step, an acidic component such as carbon dioxide in the fraction can be removed by treatment with the soda washing tower 15. Since carbon dioxide has a higher boiling point than that of ethylene, carbon dioxide, if introduced to cryogenic separation, is difficult to separate from olefin. Thus, it is preferred to remove carbon dioxide in the fraction before introduction to the cryogenic separation equipment. The washing step can be performed before introduction to the cryogenic separation equipment and is preferably performed after the first compression step because of easy operation of the soda washing tower.

In the soda washing tower 15, the fraction is preferably brought into contact with an aqueous sodium hydroxide solution to obtain a washing fraction. The amount of sodium hydroxide can be appropriately selected such that an acidic component such as carbon dioxide in the fraction can be removed.

<Cryogenic separation step>

In the cryogenic separation step, the fraction is introduced to the cryogenic separation equipment to separate ethylene and propylene. In this context, the fraction to be introduced in the washing step or the cryogenic separation step is preferably a combination fraction or a fraction derived therefrom. The "combination fraction" is a fraction obtained by the combination step mentioned above. In this context, the "fraction derived therefrom" means a fraction obtained by further refining the combination fraction with refining equipment such as a soda washing tower.

The cryogenic separation generally separates components by distillation operation under conditions of an ultralow temperature and a high pressure. For example, the fraction is liquefied under conditions of an ultralow temperature and a high pressure to separate hydrogen and methane from light olefin.

In the cryogenic separation step, light olefin such as ethylene and propylene is further separated through a demethanizer, a deethanizer, an ethylene rectifier, a depropanizer, a propylene rectifier, and the like.

The refining system of a naphtha cracker is in the form of a front-end demethanizer or a front-end depropanizer. Hereinafter, an exemplary apparatus configuration in each form will be described. However, the presence or absence or order of each separation tower is not limited thereto.

In the refining system in the form of a front-end demethanizer, light olefin is refined by, for example, the following procedures: first, an introduction fraction compressed with a compressor is introduced to a cooler where at least a portion of the light olefin in the introduction fraction is liquefied while at least a portion of hydrogen is removed. The resulting fraction is introduced to a demethanizer to obtain a methane-free fraction mainly containing light olefin from the tower bottom and an off-gas fraction containing hydrogen and methane from the tower top. The obtained methane-free fraction is introduced to a deethanizer to obtain an ethylene fraction mainly containing ethylene from the tower top and an ethane-free fraction mainly containing olefin having 3 or more carbon atoms from the tower bottom. The obtained ethylene fraction is introduced to an ethylene rectifier and separated into ethylene and ethane to obtain refined ethylene. The obtained ethane-free fraction is introduced to a depropanizer to obtain a propylene fraction mainly containing propylene from the tower top and a propane-free fraction mainly containing olefin having 4 or more carbon atom from the tower bottom. The obtained propylene fraction is introduced to a propylene rectifier and separated into propylene and propane to obtain refined propylene. The obtained propane-free fraction is introduced to a debutanizer to obtain olefin having 4 carbon atoms.

In the refining system in the form of a front-end depropanizer, light olefin is refined by, for example, the following procedures: in the refining system in the form of a front-end depropanizer, the fraction is separated into an introduction fraction mainly containing hydrocarbon having 3 or less carbon atoms and a detour fraction mainly containing hydrocarbon having more than 3 carbon atoms, whereby the amount of the fraction introduced to the cryogenic separation step is reduced, and energy consumption related to cryogenic separation can be reduced. In other words, for example, as shown

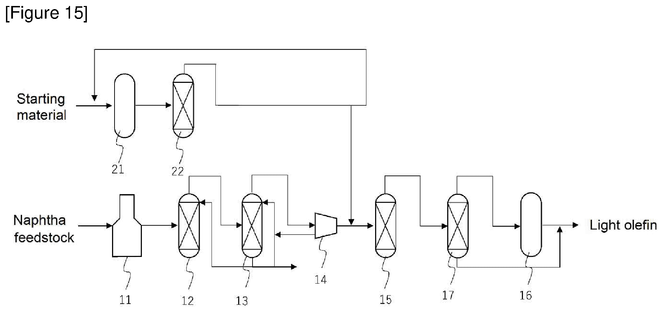

in Figure 15, in the refining system in the form of a front-end depropanizer, the fraction is introduced to a first distillation tower 17 to separate an introduction fraction to be introduced to the cryogenic separation step. Also, a detour fraction to be introduced to downstream equipment by detouring the cryogenic separation step is obtained from the tower bottom of the first distillation tower 17. In the first distillation tower 17, for example, the separation operation may be carried out with one distillation tower, or the separation operation may be carried out in stages with a plurality of, for example, two to four distillation towers. Then, the obtained introduction fraction is compressed with a compressor and then introduced to a cooler where at least a portion of the light olefin in the introduction fraction is liquefied while at least a portion of hydrogen is removed. After cooling and compression of the introduction fraction, the compound of interest can be separated from the introduction fraction by the same treatment as in the refining system in the form of a front-end demethanizer. In the refining system in the form of a front-end depropanizer, the introduction fraction obtained with the first distillation tower 17 is introduced to a deethanizer and thereby further refined and can then be introduced to the cryogenic separation step. The introduction fraction can thus be refined, in addition to the first distillation tower 17, whereby energy consumption related to the cryogenic separation step can be reduced.

<Ethanol conversion step>

A starting material containing ethanol is introduced to the reactor 21. In the reactor 21, the ethanol conversion step mentioned above is performed. In the reactor 21, the starting material containing ethanol is brought into contact with a catalyst to obtain an ethanol conversion fraction containing ethylene and propylene.

(Starting material: ethanol conversion step)

In the ethanol conversion step, a starting material containing ethanol is used. Ethanol produced by various methods can be used. Bioethanol, in particular, is preferably used from the viewpoint of excellent environmental friendliness. In recent years, the importance of replacement of fossil resources such as crude oil with regenerable plant-derived resources has been reaffirmed in order to realize sustainable society or to suppress greenhouse gas emission. Accordingly, chemical products, which heretofore been produced using fossil resources such as crude oil, can be produced using bioethanol obtained from such a regenerable plant-derived resource.

The content of ethanol in the starting material is preferably 30 to 100% by mass, more preferably 40 to 100% by mass, further preferably 50 to 100% by mass, based on the total amount of the starting material.

A starting material containing ethylene and ethanol may be used as the starting material. Use of this starting material enables propylene to be produced at a good yield by using an adiabatic reactor and controlling the internal temperature of the reactor. The starting material may also contain olefin having 4 to 6 carbon atoms.

The ethylene/ethanol molar ratio in the starting material is preferably 0.05 to 2.5, more preferably 0.20 to 2.0, further preferably 0.30 to 1.8, still further preferably 0.30 to 1.5.

In the ethanol conversion step, the starting material may contain olefin having 4 to 6 carbon atoms and an oxygen-containing compound having 1 to 6 carbon atoms other than ethanol. Examples of the oxygen-containing compound having 1 to 6 carbon atoms other than ethanol include methanol, propanol, dimethyl ether, and diethyl ether. These compounds are brought into contact with a catalyst in the reactor and can thereby give the compound of interest such as ethylene and propylene.

The starting material may contain a saturated aliphatic hydrocarbon such as paraffin, olefin having 7 or more carbon atoms, and an oxygen-containing compound having 7 or more carbon atoms. The saturated aliphatic hydrocarbon, the olefin having 7 or more carbon atoms, and the oxygen-containing compound having 7 or more carbon atoms may be brought into contact with a catalyst and converted to the compound of interest such as propylene, as in ethylene and ethanol, in combination with dehydrogenation reaction or dehydration reaction and however, are less reactive than the olefin having 4 to 6 carbon atoms and the oxygen-containing compound having 1 to 6 carbon atoms other than ethanol mentioned above.

The starting material can contain the whole amount or a portion of olefin having 4 or more carbon atoms separated by a separation step from the reaction gas containing olefin having 3 or more carbon atoms obtained by the ethanol conversion step. Use of such a so-called recycling reaction system can achieve effective utilization of the olefin starting material.

The starting material may contain an inert gas such as nitrogen, in addition to the starting material mentioned above that may be converted to the compound of interest such as propylene by the ethanol conversion step. The starting material may additionally contain hydrogen and methane as diluted gases, though it is preferred to not perform hydrogen dilution. Hydrogen may be used for suppressing the coking-induced deterioration of a catalyst.

In the ethanol conversion step, the starting material may contain water. The starting material may contain "water generated in a production step" because ethylene and ethanol contained in a mixed starting material can be produced by various production methods. In this context, the "water generated in a production step" refers to unremoved water

generated in an ethylene and/or ethanol production process.

In the ethanol conversion step, the mixed starting material can be allowed to contain steam, in addition to the "water generated in a production step". The steam is effective for suppressing coking-induced deterioration by decreasing an olefin partial pressure, and improving the yield of lower olefin. On the other hand, the steam may promote the dealumination of zeolite. Therefore, it is preferred that the mixed starting material should not be allowed to contain steam, in addition to the "water generated in a production step".

In the method for producing light olefin according to the present embodiment, a relationship between a ratio $O_E$ of an amount of hydrogen and methane to a total amount of ethylene and propylene in the ethanol conversion fraction and a ratio $O_C$ of an amount of hydrogen and methane to a total amount of ethylene and propylene in the naphtha cracking fraction preferably satisfies the expression (1):

$$O_E < O_C \ldots (1).$$

A method for producing light olefin which reduces treatment energy in cryogenic separation can be provided by satisfying this condition.

In the naphtha cracking process, after naphtha cracking, light olefin is obtained by a cryogenic separation step through a refining process. In the cryogenic separation step, the fraction to be introduced to the cryogenic separation step is pressure-boosted using a compressor and cooled to a temperature equal to or lower than the boiling point of ethylene at the pressure so that light olefin in the fraction is liquefied to separate the light olefin from off-gases such as hydrogen and methane by gas-liquid separation. In this pressure-boosting and cooling treatment, consumed energy is increased depending on the flow rate of the fraction to be introduced to the cryogenic separation step. In other words, the relationship between the ratio $O_E$ of the amount of hydrogen and methane to the total amount of ethylene and propylene in the ethanol conversion fraction and the ratio $O_C$ of the amount of hydrogen and methane to the total amount of ethylene and propylene in the naphtha cracking fraction falls within the range as mentioned above, whereby the proportion of hydrogen and methane in the fraction to be introduced to the cryogenic separation step can be decreased. Therefore, the flow rate of hydrogen and methane in the fraction to be introduced is reduced when the amount of light olefin produced is constant; thus, cryogenic separation can be more efficiently performed.

In the method for producing light olefin according to the present embodiment, a difference between the ratio $O_C$ and the ratio $O_E$ ($O_C$ - $O_E$) is preferably 5% by mass or more, more preferably 10% by mass or more, further preferably 20% by mass or more, from the viewpoint of efficient refining efficiency. Owing to the difference ($O_C$ - $O_E$), the proportion of hydrogen and methane in the fraction to be introduced to the cryogenic separation step can be decreased, and cryogenic separation can therefore be efficiently performed. The upper limit of the difference ($O_C$ - $O_E$) is not particularly limited and may be 70% by mass or less.

In the method for producing light olefin according to the present embodiment, a difference $|P/E_C - P/E_E|$ between a propylene/ethylene mass ratio $P/E_E$ in the ethanol conversion fraction and a propylene/ethylene mass ratio $P/E_C$ in the naphtha cracking fraction is preferably 0.2 or less, more preferably 0.1 or less, further preferably 0.05 or less. The lower limit value of the difference $|P/E_C - P/E_E|$ is not particularly limited and may be, for example, 0 or more (i.e., the same proportions).

(Adiabatic reactor)

In the ethanol conversion step, the reactor is not particularly limited and is preferably an adiabatic reactor because of small energy consumption. For the adiabatic reactor, see the description of Adiabatic Fixed-Bed Reactors (Elsevier, 2014, Ch.1, P.4, L.5 to 24, ISBN:978-0-12-801306-9). Examples of the adiabatic reactor include fixed-bed adiabatic reactors, moving-bed adiabatic reactors, and fluidized-bed adiabatic reactors. In the method of the present embodiment, a fixed-bed adiabatic reactor is preferred. The fixed-bed adiabatic reactor is more preferably a fixed-bed single-stage adiabatic reactor having one stage of a fixed catalyst bed. Since carbonaceous matter (coke) accumulates on a catalyst in association with reaction, a multi-tower switchable fixed-bed single-stage adiabatic reactor is preferred which is capable of removing this carbonaceous matter by combustion while the reaction is continued.

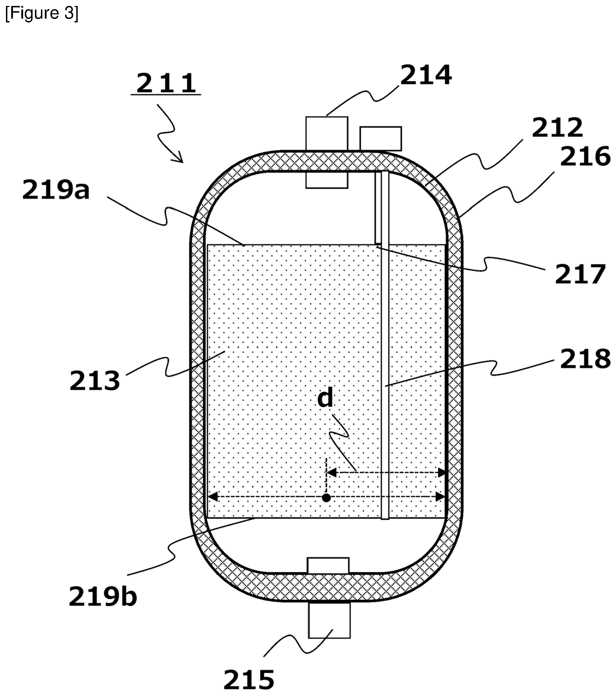

Figure 3 is a schematic configuration diagram of a fixed-bed single-stage adiabatic reactor. A fixed-bed single-stage adiabatic reactor 211 has a reaction housing 212 provided peripherally with a heat insulating material 216, a catalyst bed 213, a reactor inlet 214, and a reactor outlet 215. The reaction housing 212 is provided peripherally with the heat insulating material 216 and thereby does not allow heat in the reactor to escape to the outside. In the production method according to the present embodiment, the internal temperature of the reactor can be controlled by heat generation and heat absorption through reaction.

The catalyst bed 213 is filled with a catalyst mentioned later. A first sheath thermocouple 217 is disposed immediately before contact with a catalyst bed inlet 219a of the catalyst bed 213. A second sheath thermocouple 218 is

disposed immediately after exit from a catalyst bed outlet 219b of the catalyst bed 213. These thermocouples measure the temperatures of the mixed starting material immediately before contact with the catalyst bed inlet 219a and a reaction gas immediately after exit from the catalyst bed outlet 219b. The catalyst bed 213 may be in a multi-stage form and is preferably in a single-stage form as shown in Figure 3.

In the fixed-bed single-stage adiabatic reactor 211, the mixed starting material is introduced from the reactor inlet 214 and brought into contact with the catalyst bed 213, and a reaction gas is taken out of the reactor outlet 215.

The reaction temperature in the ethanol conversion step is preferably 300 to 600°C, more preferably 450 to 590°C, further preferably 500 to 580°C, from the viewpoint of suppressing coking-induced deterioration while enhancing reactivity. The reaction temperature is a value calculated according to the expression: [Inlet temperature of the catalyst bed + Outlet temperature of the catalyst bed] / 2. The inlet temperature of the catalyst bed is the temperature of the mixed starting material immediately before the starting material fluid comes into contact with the catalyst bed packed in the adiabatic reactor. The outlet temperature of the catalyst bed is the temperature of a reaction gas immediately after the reaction gas passes through the catalyst bed. In this context, the temperatures of the mixed starting material and the reaction gas refer to temperatures from 0d to 0.8d wherein in a plane perpendicular to the flow direction of a fluid, the center of the reactor is defined as 0, and the distance from the center of the reactor to the inner wall surface of the reactor is defined as d.

The reaction pressure in the ethanol conversion step is preferably 0.01 to 3.0 MPaG, more preferably 0.01 to 1.0 MPaG.

The supply rate of the starting material in the ethanol conversion step is preferably 0.1 to 1000 $hr^{-1}$, more preferably 0.1 to 500 $hr^{-1}$, further preferably 0.5 to 100 $hr^{-1}$, in terms of weight hourly space velocity (WHSV) based on the mass of the catalyst. In the ethanol conversion step, WHSV is calculated by using ethylene-based ethanol as represented by the following expressions.

WHSV ($hr^{-1}$) = Mass flow rate (kg/hr) of starting material supply / Amount (kg) of the catalyst

Mass flow rate (kg/hr) of starting material supply = Ethylene flow rate (kg/hr) + Ethylene-based ethanol flow rate (kg/hr) + Flow rate (kg/hr) of olefin having 4 to 6 carbon atoms + Flow rate (kg/hr) of oxygen-containing compound having 1 to 6 carbon atoms other than ethanol

**Ethylene-based** ethanol flow rate (kg/hr) = Ethanol flow rate (kg/hr) × Molecular weight (g/mol) of ethylene / Molecular weight (g/mol) of ethanol

(Catalyst)

The catalyst in the ethanol conversion step is a solid catalyst that exhibits catalytic ability to convert olefin and ethanol to the compound of interest such as propylene. Such a catalyst is preferably a zeolite-containing catalyst from the viewpoint of excellent thermal durability and propylene selectivity of the catalyst. A common problem of conventional olefin production with zeolite includes coking-induced deterioration in which zeolite is deactivated by the accumulation of heavy carbonaceous matter (coke) inside zeolite pores through reaction with hydrocarbon. For regenerating catalyst performance, it is preferred to remove coke by combustion in an atmosphere containing an oxygen molecule. However, the structural collapse of zeolite progresses along with this coke combustion and induces the permanent deterioration of the catalyst difficult to regenerate. The ethanol conversion step can suppress coke formation and therefore easily maintains catalytic activity even if the zeolite-containing catalyst is used.

<<Zeolite-containing catalyst>>

The zeolite-containing catalyst refers to a catalyst powder or a molded catalyst containing zeolite as an active species. In the ethanol conversion step, so-called intermediate pore-size zeolite having a pore size of 5 to 6 angstroms is preferably used as zeolite in the zeolite-containing catalyst described above. The intermediate pore-size zeolite means "zeolite having a pore size intermediate between the pore size of small pore-size zeolite typified by A-type zeolite and the pore size of large pore-size zeolite typified by mordenite and X-type or Y-type zeolite in the pore size range". The "intermediate pore-size zeolite" has a so-called oxygen 10-membered ring in its crystal structure.

Examples of the intermediate pore-size zeolite include MFI-type zeolite such as ZSM-5, ZSM-8, ZSM-11, ZSM-12, ZSM-21, ZSM-23, ZSM-35, ZSM-38, LZ-105, FZ-1, TS-1, and silicate. Among them, MFI-type zeolite is preferred, and ZSM-5 is more preferred. Zeolite similar to ZSM-5 or ZSM-11 described in Stud. Surf. Sci. Catal. 1987, 33, 167-215 can be used. Among them, MFI-type zeolite is preferred, and ZSM-5 is more preferred, from the viewpoint of excellent catalyst performance(catalytic activity and durability against coking).

The silica/alumina ($SiO_2/Al_2O_3$) molar ratio of zeolite contained in the zeolite-containing catalyst of the present embodiment can be appropriately selected and is preferably 20 to 2000 from the viewpoint of excellent catalytic activity and propylene selectivity, more preferably 100 to 1500, further preferably 300 to 1200, still further preferably 800 to 1200, from the viewpoint of enhancing the durability of the catalyst. The silica/alumina ($SiO_2/Al_2O_3$) molar ratio of zeolite contained in the zeolite-containing catalyst may be 20 to 400 or may be 100 to 300. The silica/alumina molar ratio of zeolite can be measured by a known method and can be determined, for example, by completely dissolving the zeolite in an alkaline aqueous solution and analyzing the resulting solution by plasma emission spectroscopy or the like.

A method for synthesizing the zeolite of the present embodiment is not particularly limited, and the zeolite can be produced by optimizing various conditions of a heretofore known hydrothermal synthesis method for MFI-type zeolite. A general approach of efficiently obtaining MFI-type zeolite by the hydrothermal synthesis method includes a hydrothermal synthesis method using a proper organic structure-directing agent (SDA), a hydrothermal synthesis method of adding hydrothermally synthesized MFI zeolite as seed crystals, or a hydrothermal synthesis method of adding a seed slurry at a crystal stage. Examples of the organic structure-directing agent (SDA) used here include ammonium salts, urea salts, amines, and alcohols. It is known that not only the organic SDA but inorganic cations or anions are also involved in the structure. Zeolite synthesis depends on composite functions of individual components. In the hydrothermal synthesis method for MFI-type zeolite as mentioned above, a suitable catalyst can be obtained by appropriately optimizing synthesis conditions such as the composition of a starting material to be added (e.g., the types of materials and an additive (SDA), the amount of the additive, pH, the silica/alumina molar ratio, and the abundance ratios of a medium, a cation, and an anion), a synthesis temperature, and a synthesis time.

Specific examples thereof include a synthesis method using a seed slurry described in Japanese Patent No. 5426983, and methods listed in The Hydrothermal Synthesis of Zeolites (Chemcal Reviews, 2003, 103, 663-702).

Commercially available zeolite can also be used as long as the zeolite is MFI zeolite having the specific physical properties and composition mentioned above.

The zeolite-containing catalyst according to the present embodiment preferably contains a phosphorus element or a silver element.

Examples of the form of the phosphorus element include polymers of phosphorus (e.g., polyphosphoric acid), oxides of phosphorus (e.g., $P_2O_5$), and compounds in the form of phosphorus added to aluminum of zeolite. Two or more of them may be contained in the catalyst. When zeolite contains aluminum, the phosphorus element is effective for suppressing the dealumination of the zeolite and may be effective for improving a propylene yield. Particularly, for purposes involving exposure to a high-temperature steam atmosphere, the effect of suppressing dealumination is more improved because the characteristics of the zeolite-containing catalyst vary easily depending on the dealumination.

The amount of the phosphorus element contained in the zeolite-containing catalyst is preferably 0.01 to 2.0% by mass based on the mass of the whole catalyst and is more preferably 0.05 to 2.0% by mass from the viewpoint of an excellent effect of suppressing dealumination.

In the present embodiment, the amount of the phosphorus element contained in the catalyst refers to a value measured using a fluorescent X-ray analysis apparatus. The amount of the phosphorus element contained can be measured under usual conditions using a commercially available fluorescent X-ray analysis apparatus in accordance with an instruction manual. In the case of using, for example, trade name "RIX3000" manufactured by Rigaku Holdings Corp., measurement conditions can be set to a tubular voltage of 50 kV and a tubular current of 50 mA using P-K$\alpha$ ray.

In the present embodiment, phosphoric acid and/or phosphate (hereinafter, also referred to as a "phosphorus source") is used as a starting material for the phosphorus element contained in the zeolite-containing catalyst. The phosphorus source is more preferably phosphate. The phosphate is more preferably a compound that exhibits a solubility of 1 g or more in 100 g of water at 25°C.

Examples of the phosphoric acid include phosphoric acid and pyrophosphoric acid. Examples of the phosphate include phosphoric acid ammonium salts such as ammonium phosphate, diammonium hydrogen phosphate, ammonium dihydrogen phosphate, and sodium ammonium hydrogen phosphate, potassium hydrogen phosphate, aluminum hydrogen phosphate, sodium phosphate, and potassium phosphate. Among them, a phosphoric acid ammonium salt having a relatively high solubility in water is preferred, and at least one member selected from the group consisting of ammonium phosphate, diammonium hydrogen phosphate, and ammonium dihydrogen phosphate is more preferred. These phosphorus sources may be used singly or in combination of two or more thereof.

The form of the silver element is, for example, a silver ion. The silver element is effective for improving the hydrothermal resistance of zeolite by controlling the acid site of the zeolite.

The amount of the silver element contained in the zeolite-containing catalyst is preferably 0.01 to 2.0% by mass based on the mass of the whole catalyst and is more preferably 0.05 to 2.0% by mass from the viewpoint of an excellent effect of improving hydrothermal resistance per content.

In the present embodiment, the amount of the silver element contained in the catalyst refers to a value measured using a fluorescent X-ray analysis apparatus. The amount of the silver element contained can be measured under usual conditions using a commercially available fluorescent X-ray analysis apparatus in accordance with an instruction manual.

In the case of using, for example, trade name "RIX3000" manufactured by Rigaku Holdings Corp., measurement conditions can be set to a tubular voltage of 50 kV and a tubular current of 50 mA using P-Kα ray.

In the present embodiment, for example, silver nitrate is used as a starting material for the silver element contained in the zeolite-containing catalyst. The zeolite-containing catalyst containing the silver element is obtained by using a zeolite-containing catalyst containing sodium as a counter cation and exchanging an ion with silver nitrate, followed by sintering. The ion exchange between the counter cation sodium in zeolite and silver nitrate can be carried out by dipping the zeolite or the zeolite-containing catalyst in an aqueous silver nitrate solution, followed by washing with water. In this respect, the ion exchange rate can be improved by carrying out dipping and washing with water a plurality of times. Alternatively, the zeolite-containing catalyst containing the silver element may be obtained by treating proton-type or ammonium-type zeolite with silver nitrate.

The zeolite-containing catalyst of the present embodiment can be produced by using zeolite having the specific physical properties and composition mentioned above, and molding the zeolite, for example, as described below. The molding method is not particularly limited, and a general method can be used. Specific examples thereof include a method of compression-molding a catalytic component, a method of extrusion-molding the component, and a spray dry molding method suitable for fluidized-bed reaction schemes.

A binder can be used in molding. The binder is not particularly limited, and, for example, silica, alumina, and kaolin can be used alone or as a mixture. Commercially available products can be used as these binders. The zeolite/binder mass ratio is preferably in the range of 10/90 to 90/10, more preferably in the range of 20/80 to 80/20. A silica binder is preferred from the viewpoint of being able to suppress coking.

In the ethanol conversion step, the zeolite-containing catalyst may be subjected to a pretreatment step prior to contact of the zeolite-containing catalyst with the starting material. Preferred examples of the pretreatment step include the step of heat-treating the catalyst at a temperature of 300°C or higher in the presence of steam. The pretreatment thus performed tends to attain a more marked effect of suppressing the deterioration of the catalyst or improving selectivity. In the case of the method described above, the treatment is preferably performed under conditions involving a temperature of 300°C or higher and 900°C or lower, an atmosphere of, but not particularly limited to, air or a mixed gas of an inert gas such as nitrogen and steam (water vapor) circulated, and a water vapor partial pressure of 0.01 atm or more. The heat treatment temperature is more preferably a temperature of 400°C or higher and 700°C or lower. This pretreatment step can be performed using the reactor for ethanol conversion.

(Product: ethanol conversion fraction containing ethylene and propylene)

In the ethanol conversion step, the starting material is brought into contact with the catalyst to obtain an ethanol conversion fraction containing ethylene and propylene. The ethanol conversion fraction may contain hydrogen, aliphatic hydrocarbon having 1 to 3 carbon atoms, aliphatic hydrocarbon having 4 to 8 carbon atoms, an aromatic compound, and hydrocarbon having 9 or more carbon atoms.

The total amount of ethylene and propylene contained in the ethanol conversion fraction is preferably 15% by mass or more, more preferably 20% by mass or more, further preferably 25% by mass or more, from the viewpoint of excellent efficiency of production of the compound of interest. The P/E mass ratio, which represents the mass ratio of propylene to ethylene, can be appropriately adjusted depending on reaction conditions and is preferably 0.40 to 1.0, more preferably 0.45 to 0.80, further preferably 0.50 to 0.70, from the viewpoint of excellent efficiency of refining of the compound of interest.

<Refining step>

The method for producing light olefin may comprise a refining step of separating the compound of interest such as ethylene and propylene from the ethanol conversion fraction.

In the refining step, the ethanol conversion fraction is preferably refined such that an ethylene and propylene content $C_{E1}$ after refining is higher than an ethylene and propylene content in the naphtha cracking fraction or a fraction derived therefrom in the combination step, to obtain a refined ethanol conversion fraction.

(Third cooling step)

The refining step preferably has a third cooling step of introducing the ethanol conversion fraction to a third cooling tower 22 to obtain a cooled ethanol conversion fraction mainly containing olefin having 6 or less carbon atoms.

The ethanol conversion fraction obtained in the reactor 21 is introduced to the third cooling tower 22. In the third cooling tower 22, the third cooling step mentioned above is performed to obtain a cooled ethanol conversion fraction mainly containing olefin having 6 or less carbon atoms. In the third cooling tower 22, the ethanol conversion fraction is cooled to remove hydrocarbon having more than 6 carbon atoms and water.

In the third cooling tower 22, the ethanol conversion fraction is preferably brought into contact with water and thereby cooled. The water for use in the third cooling tower 22 can be appropriately pH-adjusted for the purpose of suppressing the corrosion of the apparatus.
The pressure of the cooled ethanol conversion fraction after treatment with the third cooling tower 22 is preferably 0.01 to 1.5 MPaG, more preferably 0.05 to 0.5 MPaG, further preferably 0.07 to 0.2 MPaG.
The temperature of the cooled ethanol conversion fraction after treatment with the third cooling tower 22 is preferably 5 to 200°C, more preferably 10 to 100°C.
The method for producing light olefin according to the present embodiment preferably has a recycling step of introducing at least a portion of the cooled ethanol conversion fraction as a portion of the starting material to the reactor 21. The cooled ethanol conversion fraction is thus supplied to the reactor 21 and can thereby be converted to ethylene or propylene.

<Compression and separation step>

The method for producing light olefin according to the present embodiment may have a compression and separation step of pressure-boosting the cooled ethanol conversion fraction using a compressor to obtain a light ethanol conversion fraction mainly containing olefin having 3 or less carbon atoms as a gas component and to obtain a heavy ethanol conversion fraction mainly containing olefin having 4 or more carbon atoms as a liquid component.
The method may have, as the compression and separation step:

a second compression step of pressure-boosting the cooled ethanol conversion fraction using a compressor; and

a distillation step of using a distillation tower to obtain a light ethanol conversion fraction mainly containing olefin having 3 or less carbon atoms as a gas component and to obtain a heavy ethanol conversion fraction mainly containing olefin having 4 or more carbon atoms as a liquid component.

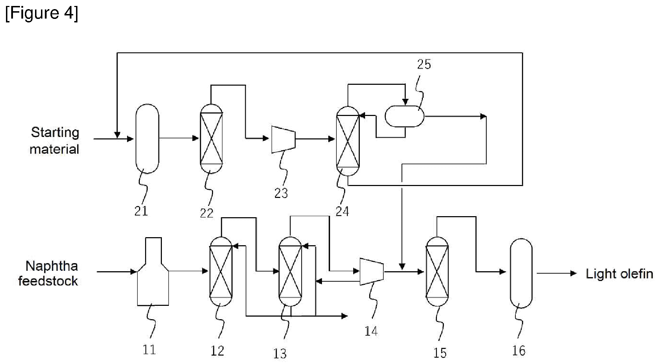

More specifically, as shown in Figure 4, the cooled ethanol conversion fraction obtained by the third cooling step may be pressure-boosted with a second compressor 23 and introduced to a distillation tower 24.
In the distillation tower 24, a light ethanol conversion fraction mainly containing olefin having 3 or less carbon atoms is obtained as a gas component, and a heavy ethanol conversion fraction mainly containing hydrocarbon having 4 or more carbon atoms is obtained as a liquid component. In this way, the distillation step is performed with the second compressor 23 and the distillation tower 24.
The obtained light ethanol conversion fraction may be introduced to a condenser 25 where a portion of the fraction is liquefied and refluxed to the distillation tower to obtain a cooled light ethanol conversion fraction. The distillation step thus established can reduce hydrocarbon having 4 or more carbon atoms and water in a fraction derived from the ethanol conversion fraction and can elevate the proportion of ethylene and propylene.
The pressure of the cooled light ethanol conversion fraction is preferably 0.5 to 3.0 MPaG, more preferably 1.0 to 2.5 MPaG, further preferably 1.1 to 2.0 MPaG.
The temperature of the cooled light ethanol conversion fraction is preferably 0 to 50°C, more preferably 5 to 30°C, further preferably 10 to 20°C.
In the refining step comprising the distillation step and the like, the ethanol conversion fraction is refined such that an ethylene and propylene content $C_{E1}$ after refining is higher than an ethylene and propylene content in the naphtha cracking fraction or a fraction derived therefrom in the combination step, to obtain a refined ethanol conversion fraction.
When the ethanol conversion fraction that has thus undergone the compression and separation step is combined in the combination step mentioned later, a fraction with a high content ratio of olefin having 3 carbon atoms from which hydrocarbon having 4 or more carbon atoms has been removed is combined with a fraction derived from the naphtha cracking fraction. Therefore, the content of hydrocarbon having 4 or more carbon atoms is decreased in the combination fraction, and the proportion of ethylene and propylene can be elevated. As a result, treatment energy can be reduced in cryogenic separation.
The method for producing light olefin according to the present embodiment preferably has a recycling step of introducing at least a portion of the heavy ethanol conversion fraction as a portion of the starting material to the reactor 21. The heavy ethanol conversion fraction is thus supplied to the reactor 21 where olefin contained in the heavy ethanol conversion fraction is converted to ethylene or propylene. Therefore, light olefin production efficiency from ethanol can be improved.
In the compression and separation step, a portion of the fraction is liquefied by pressure boosting using a compressor, and the resulting fraction may be treated with a gas-liquid separation apparatus to obtain a light ethanol conversion fraction mainly containing olefin having 3 or less carbon atoms as a gas component and to obtain a heavy ethanol conversion fraction mainly containing olefin having 4 or more carbon atoms as a liquid component. Use of the gas-

liquid separation apparatus can suppress energy consumption related to the compression and separation step as compared with the case of using a distillation tower.

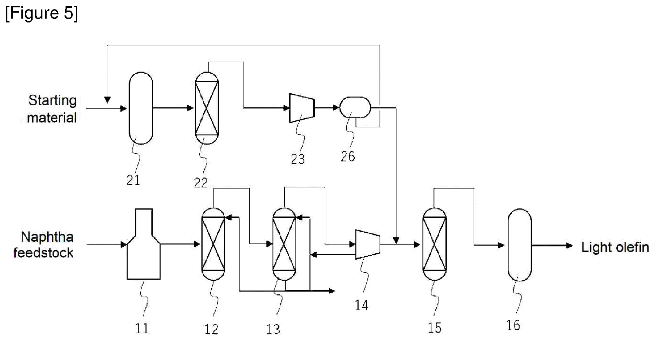

More specifically, as shown in Figure 5, the cooled ethanol conversion fraction obtained by the third cooling step may be pressure-boosted with a second compressor 23 and introduced to a gas-liquid separation drum 26.

When the ethanol conversion fraction that has thus undergone the compression and separation step with the gas-liquid separation apparatus is combined in the combination step mentioned later, a fraction with a high content ratio of olefin having 3 carbon atoms from which hydrocarbon having 4 or more carbon atoms has been removed is combined with a fraction derived from the naphtha cracking fraction. Therefore, the content of hydrocarbon having 4 or more carbon atoms is decreased in the combination fraction, and the proportion of ethylene and propylene can be elevated. As a result, treatment energy can be reduced in cryogenic separation.

The method for producing light olefin according to the present embodiment preferably has a recycling step of introducing at least a portion of the heavy ethanol conversion fraction that has undergone the compression and separation step with the gas-liquid separation apparatus as a portion of the starting material to the reactor 21. The heavy ethanol conversion fraction is thus supplied to the reactor 21 where olefin contained in the heavy ethanol conversion fraction is converted to ethylene or propylene. Therefore, light olefin production efficiency from ethanol can be improved.

<Combination step>

In the combination step, at least a portion of the naphtha cracking fraction or a fraction derived therefrom is combined with at least a portion of the ethanol conversion fraction or a fraction derived therefrom to obtain a combination fraction.

The "naphtha cracking fraction" is a fraction obtained by the cracking step mentioned above. The "fraction derived therefrom" means a fraction obtained by further refining the naphtha cracking fraction. The naphtha cracking fraction may be mixed with a gas containing hydrocarbon in order to adjust properties before refining.

The "ethanol conversion fraction" is a fraction obtained by the ethanol conversion step mentioned above. The "fraction derived therefrom" means a fraction obtained by further refining the ethanol conversion fraction.

The combination step in the method for producing light olefin according to the present embodiment will be described with reference to specific examples.

In the first embodiment, the combination step is performed after the second cooling step mentioned above, for example, as shown in Figure 4, Figure 5, Figure 16, and Figure 17.

The combination step can reduce the flow rate of the fraction to be introduced to the cryogenic separation step and can reduce load in the cryogenic separation step.

The production method mentioned above further comprises a first compression step of pressure-boosting the second cooling fraction. In the second cooling fraction, at least a portion of the naphtha cracking fraction or a fraction derived therefrom in the combination step may be compressed. In this case, the ethanol conversion fraction or a fraction derived therefrom is preferably a fraction pressure-boosted by the compression and separation step or the like.

The naphtha cracking fraction or a fraction derived therefrom thus pressure-boosted by the first compression step is combined with the ethanol conversion fraction or a fraction derived therefrom, i.e., the ethanol conversion fraction or a fraction derived therefrom does not undergo the first compression step, whereby pressure boosting load can be reduced in the naphtha cracking process, and the ethanol conversion process is easily connected with the naphtha cracking process. When the ethanol conversion fraction or a fraction derived therefrom has a higher pressure than that of the naphtha cracking fraction or a fraction derived therefrom, the ethanol conversion process is easily connected with the naphtha cracking process by lowering the pressure.

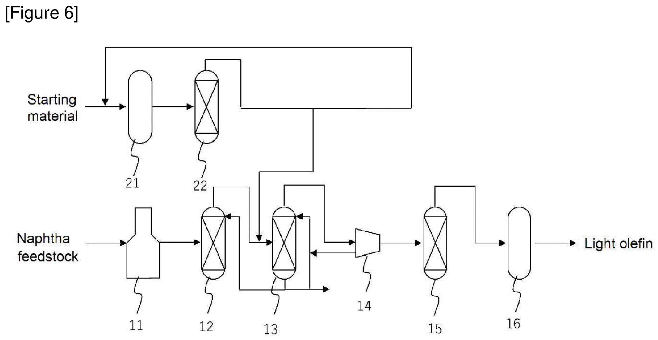

In the second embodiment, the combination step is performed after the first cooling step mentioned above, for example, as shown in Figure 6, Figure 7, Figure 9, Figure 18, Figure 19, and Figure 21.

In the second embodiment, before introduction to the second cooling tower, at least a portion of the first cooling fraction is combined with at least a portion of the ethanol conversion fraction or a fraction derived therefrom. At least a portion of components having more than 4 carbon atoms contained in a fraction derived from the ethanol conversion fraction to be combined in the combination step is removed in the second cooling step. Therefore, the proportion of ethylene and propylene in the fraction to be introduced to cryogenic separation can be further elevated. For example, when the ethanol conversion fraction or a fraction derived therefrom contains a hydrophilic by-product such as ethanol, the ethanol conversion fraction or the fraction derived therefrom is introduced to the second cooling tower with water as a catalytic refrigerant, whereby the hydrophilic by-product can be extracted, and the purity of light olefin can be improved.

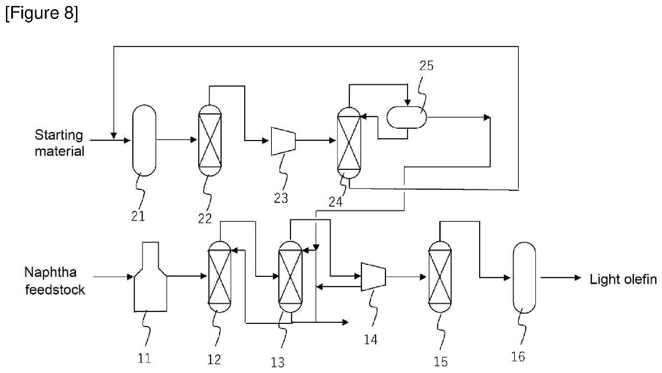

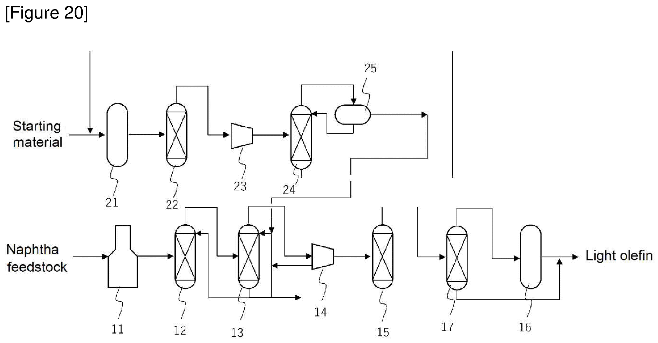

In the third embodiment, the combination step is performed, for example, as shown in Figure 8 and Figure 20, so as to be combined with a fraction in the circulation step of circulating the fraction to the second cooling tower mentioned above.

In this embodiment as well, load in the cryogenic separation step can be reduced, as in the aspects mentioned above.

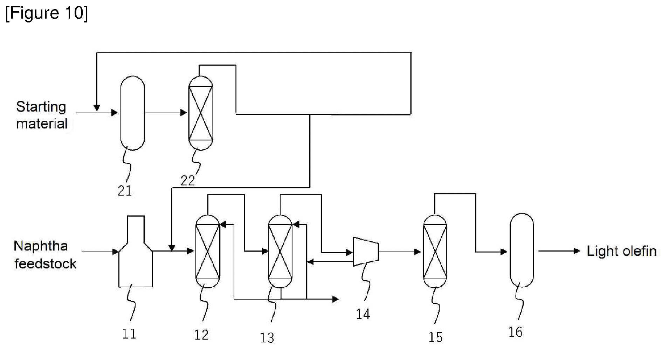

In the fourth embodiment, the combination step is performed before introduction to the first cooling tower mentioned above, for example, as shown in Figure 10, Figure 11, Figure 13, Figure 22, Figure 23, and Figure 25.

In the fourth embodiment, before introduction to the first cooling tower, at least a portion of the naphtha cracking fraction is combined with at least a portion of the ethanol conversion fraction or a fraction derived therefrom. At least a portion of components having more than 6 carbon atoms contained in a fraction derived from the ethanol conversion fraction to be combined in the combination step has been removed with the third cooling tower 22 mentioned above. In this state, the fraction is combined with the naphtha cracking fraction. Therefore, the proportion of ethylene and propylene in the combination fraction can be elevated. In addition, components having more than 4 carbon atoms contained in a fraction derived from the ethanol conversion fraction are removed in the second cooling step. Therefore, the proportion of ethylene and propylene in the fraction to be introduced to cryogenic separation can be further elevated. For example, when the ethanol conversion fraction or a fraction derived therefrom contains a lipophilic by-product, the ethanol conversion fraction or the fraction derived therefrom is introduced to the first cooling tower with heavy oil, cracked gasoline, or their mixture as a catalytic refrigerant, whereby the lipophilic by-product can be extracted, and the purity of light olefin can be improved. For example, when the ethanol conversion fraction or a fraction derived therefrom contains a hydrophilic by-product such as ethanol, the ethanol conversion fraction or the fraction derived therefrom is introduced to the second cooling tower with water as a catalytic refrigerant, whereby the hydrophilic by-product can be extracted, and the purity of light olefin can be improved.

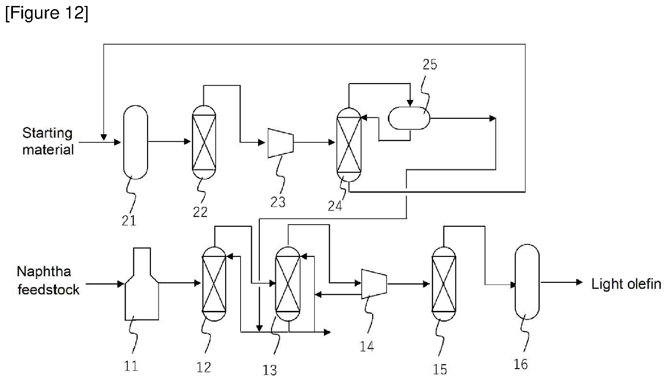

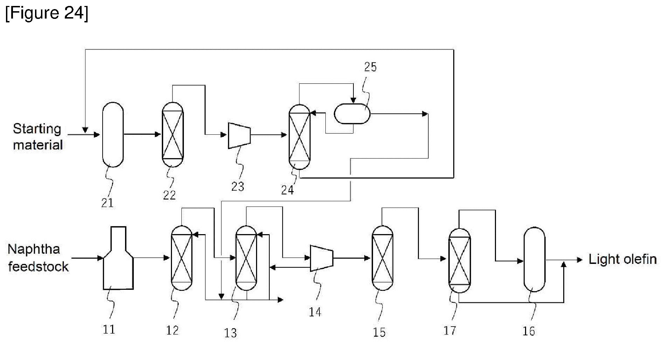

In the fifth embodiment, the combination step is performed, for example, as shown in Figure 12 and Figure 24, so as to be combined with a fraction in the circulation step of circulating the fraction to be introduced to the first cooling tower mentioned above.

In the fifth embodiment, at least a portion of the second heavy fraction in the circulation step is combined with at least a portion of the ethanol conversion fraction or a fraction derived therefrom. In this embodiment as well, load in the cryogenic separation step can be reduced, as in the aspects mentioned above. In the circulation step, at least a portion of the second heavy fraction is combined with at least a portion of the ethanol conversion fraction or a fraction derived therefrom, whereby the difference in temperature between both the components is reduced as compared with the case of being connected with at least a portion of the naphtha cracking fraction, and change in volume caused by the liquefication of the second heavy fraction can be suppressed.

A relationship between a ratio $O'_E$ of an amount of hydrogen and methane to a total amount of ethylene and propylene in the ethanol conversion fraction or a fraction derived therefrom and a ratio $O'_C$ of an amount of hydrogen and methane to a total amount of ethylene and propylene in the naphtha cracking fraction or a fraction derived therefrom in the combination step preferably satisfies the expression (1-1):

$$O'_E < O'_C \ ... \ (1\text{-}1).$$

In the method for producing light olefin according to the present embodiment, a difference between the ratio $O'_C$ and the ratio $O'_E$ ($O'_C$ - $O'_E$) is preferably 5% by mass or more, more preferably 10% by mass or more, further preferably 20% by mass or more. Since the difference ($O'_C$ - $O'_E$) can decrease the proportion of hydrogen and methane in the fraction to be introduced to the cryogenic separation step, cryogenic separation can be efficiently performed. The upper limit of the difference ($O'_C$ - $O'_E$) is not particularly limited and may be 70% by mass or less.

A relationship between a propylene/ethylene mass ratio $P'/E'_E$ in the ethanol conversion fraction and a propylene/ethylene mass ratio $P'/E'_C$ in the naphtha cracking fraction in the combination step preferably satisfies the expression (2-1):

$$|P'/E'_C - P'/E'_E| < 0.3 \ ... \ (2\text{-}1).$$

The expression (2-1) is satisfied, whereby refining equipment in an existing cracker can be used directly without the need of remodeling or the like because the fractions can have equivalent propylene/ethylene mass ratios.

$|P'/E'_C - P'/E'_E|$ is preferably 0.2 or less, more preferably 0.1 or less, further preferably 0.05 or less. The lower limit value of $|P'/E'_C - P'/E'_E|$ is not particularly limited and may be, for example, 0 or more (i.e., the same proportions).

<Light olefin>

The production method according to the present embodiment produces light olefin. The light olefin is obtained by the cryogenic separation step mentioned above. In the olefin production method according to the present embodiment, the light olefin may be any of ethylene and propylene and is preferably propylene. The production method according to the present embodiment can also produce bioresource-derived propylene by using a bioresource such as bioethanol as a starting material for use in the ethanol conversion step.

Examples

Hereinafter, the present invention will be described in more detail with reference to Examples. However, the present invention is not limited by Examples described below.

[Methods for measuring various physical properties]

Methods for measuring various physical properties are as described below.

(Silica/alumina molar ratio of zeolite in zeolite-containing catalyst)

Zeolite was completely dissolved in a sodium hydroxide solution to prepare a solution. The amounts of Si and Al contained in the solution were measured by a routine method using an ICP (inductively coupled plasma) emission spectrometer (manufactured by Rigaku Holdings Corp., trade name "JY138"), and a silica/alumina molar ratio was determined from the results. Measurement conditions were set to high-frequency power: 1 kw, plasma gas: 13 L/min, sheath gas: 0.15 L/min, nebulizer gas: 0.25 L/min, Si measurement wavelength: 251.60 nm, and Al measurement wavelength: 396.152 nm.

(Structural type of zeolite)

The structural type of zeolite in a zeolite-containing catalyst was identified by measuring the X-ray diffraction pattern of zeolite using an X-ray analysis apparatus (manufactured by Rigaku Holdings Corp., trade name "RINT"), and referring to the diffraction pattern of known zeolite. Measurement conditions are as follows.

Cu negative electrode

Tubular voltage: 40 kV

Tubular current: 30 mA

Scan speed: 1 deg/min

[Method for preparing zeolite-containing catalyst]

Clay obtained from 70 parts by mass of intermediate pore-size zeolite proton-type ZSM-5 (silica/alumina molar ratio: 980) and 30 parts by mass of silica (the amount of water was adjusted using colloidal silica and fumed silica) was kneaded and then extrusion-molded to obtain an extrudate adjusted to a diameter of 2.1 mm and a length of 4 to 6 mm. The obtained extrudate was calcined for 5 hours to obtain a proton-type zeolite-containing catalyst (ZSM-5).

The proton-type zeolite-containing catalyst was further impregnated with ammonium dihydrogen phosphate for supporting so as to attain a P/Al molar ratio of 0.7, and then calcined for 5 hours to obtain a phosphorus-supplemented zeolite-containing catalyst (P-ZSM-5).

[Ethanol conversion method]

(Starting material)

In Examples and Comparative Examples, a starting material mainly containing ethanol, ethylene, and olefin having 4 to 6 carbon atoms is used in ethanol conversion. An ethylene/ethanol molar ratio was calculated according to the following expression.

Ethylene/ethanol molar ratio (mol/mol) = Molar flow rate (mol/hr) of ethylene / Molar flow rate (mol/hr) of ethanol

(Temperature measurement)

The temperature of a catalyst bed inlet and the temperature of a catalyst bed outlet are measured with a thermocouple inserted from the outside of a reactor. Specifically, as shown in Figure 3, in a plane perpendicular to the flow direction of a fluid, the center of the reactor is defined as 0, and the distance from the center of the reactor to the inner wall surface of the reactor is defined as d. Temperatures are measured at 0.5d to 0.6d. The influence of heat release from this

thermocouple insertion is negligibly small.

(Reaction evaluation)

In accordance with Examples and Comparative Examples given below, reaction is carried out at an inlet-outlet average reaction temperature of 540°C. A portion of each fraction is sampled every 3 hours from the start of reaction, introduced to a gas chromatograph (hereinafter, also simply referred to as "GC"; TCD and FID detectors), and compositionally analyzed. The inlet-outlet average reaction temperature is calculated according to the following expression.

Inlet-outlet average reaction temperature (°C) = [Catalyst bed inlet temperature (°C) + Catalyst bed outlet temperature (°C)] / 2

(Fraction gas analysis)

Apparatus: GC-2030 manufactured by Shimadzu Corp.

Column: Custom Capillary Column SPB-1 manufactured by SUPELCO (USA) (inside diameter: 0.25 mm, length: 60 m, film thickness: 3.0 μm)

Amount of sample gas: 1 mL (the temperature of a sampling line is kept at 200°C to 300°C)

Temperature increase program: the temperature is held at 40°C for 12 minutes, subsequently increased at 5°C/min to 200°C, and then held at 200°C for 22 minutes.

Split ratio: 200:1

Carrier gas (nitrogen) flow rate: 120 mL/min

FID detector: air supply pressure: 50 kPa (approximately 500 mL/min), hydrogen supply pressure: 60 kPa (approximately 50 mL/min)

Measurement method: The TCD detector and the FID detector are linked in series. Compositional analysis is conducted on the basis of data on hydrogen detected in the TCD detector and data on oxygen-containing materials such as hydrocarbon or ethanol detected in the FID detector. The concentration of the compound of interest in a reaction gas is determined by use of the calibration curve method to determine the mass per time of the compound produced through reaction.

(C2-3 olefin treatment efficiency)

C2-3 olefin treatment efficiency is calculated from a fraction introduced to a cryogenic separation step according to the following expression.

C2-3 olefin treatment efficiency (% by mass) = (Mass of ethylene + Mass of propylene) / Mass of the introduced fraction × 100

(C2-3 olefin treatment efficiency index)

In Examples A1 to A13, the olefin treatment efficiency mentioned above is divided by the treatment efficiency of Reference Example A1, and the resulting value is used as a C2-3 olefin treatment efficiency index. In Examples B1 to B13, the olefin treatment efficiency mentioned above is divided by the treatment efficiency of Reference Example B1, and the resulting value is used as a C2-3 olefin treatment efficiency index.

(C3L volumetric efficiency)

C3L volumetric efficiency is calculated from a fraction introduced to a cryogenic separation step according to the

following expression.

C3L volumetric efficiency (% by mol) = (Molar quantity of ethylene + Molar quantity of propylene) / (Molar quantity of hydrogen + Molar quantity of methane + Molar quantity of acetylene + Molar quantity of ethylene + Molar quantity of ethane + Molar quantity of propylene + Molar quantity of propane) × 100

(C3L volumetric efficiency index)

In Examples A1 to A13, the C3L volumetric efficiency mentioned above is divided by the C3L volumetric efficiency of Reference Example A1, and the resulting value is used as a C3L volumetric efficiency index. In Examples B1 to B13, the C3L volumetric efficiency mentioned above is divided by the C3L volumetric efficiency of Reference Example B1, and the resulting value is used as a C3L volumetric efficiency index.

(Off-gas ratio in each fraction)

An off-gas ratio in a fraction is the ratio of the amount of hydrogen and methane to the total amount of ethylene and propylene in the fraction and is calculated from the composition of each fraction according to the expression given below. The off-gas ratio in an ethanol conversion fraction was defined as $O_E$, the off-gas ratio in a naphtha cracking fraction was defined as $O_C$, the off-gas ratio in the ethanol conversion fraction or a fraction derived therefrom in a combination step was defined as $O'_E$, and the off-gas ratio in the naphtha cracking fraction or a fraction derived therefrom in the combination step was defined as O'c.

Off-gas ratio (% by mass) in each fraction = (Mass of hydrogen + Mass of methane) / (Mass of ethylene + Mass of propylene) × 100

(Ethylene and propylene content in each fraction)

A content $C_E$ of ethylene and propylene in an ethanol conversion fraction or a fraction derived therefrom in a combination step, a content Cc of ethylene and propylene in a naphtha cracking fraction or a fraction derived therefrom in the combination step, a content $C_{E_AP}$ of ethylene and propylene in the ethanol conversion fraction obtained by a reaction step without a subsequent separation process, and a content $C_{C_AP}$ of ethylene and propylene in the naphtha cracking fraction obtained by a cracking step without a subsequent separation process are calculated from the composition of each fraction according to the following expression.

Content (% by mass) of ethylene and propylene in each fraction = (Mass of ethylene + Mass of propylene) / Mass of each fraction × 100

(Product ratio in recycling fraction)

In the case of recycling a portion of an ethanol conversion fraction as a recycling fraction to a reaction step, a product ratio in the recycling fraction is calculated from the composition of the recycling fraction according to the following expression.

Product ratio (% by mass) in the recycling fraction = (Mass of ethylene + Mass of propylene) / Mass of the recycling fraction × 100

(Mass ratio P/E between propylene and ethylene)

A mass ratio P/E between propylene and ethylene in each fraction is calculated from the composition of each fraction according to the following expression.

$$P/E\ (-) = \text{Mass of propylene} / \text{Mass of ethylene}$$

[Naphtha cracking method]

In Examples and Comparative Examples, naphtha and diluted steam with a mass ratio of 0.5 to naphtha are co-fed into a heated tubular reactor to obtain a naphtha cracking gas. The obtained naphtha cracking fraction is analyzed in

the same manner as the ethanol conversion method. The naphtha cracking fraction is temperature-adjusted by being mixed with a light gas recycled with downstream refining equipment prior to a downstream step.

[Reference Example A1]

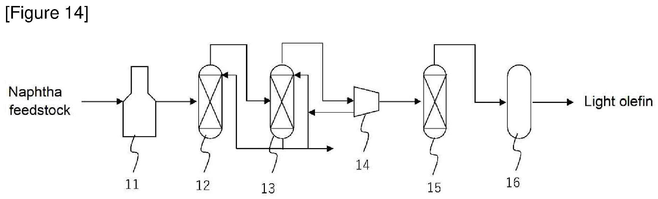

Reference Example A1 is carried out with the light olefin production equipment in the form of a front-end demethanizer shown in Figure 14. 357.3 ton/y of a naphtha feedstock containing heavy naphtha and diluted steam with a mass ratio 0.5 to naphtha is introduced to a cracking furnace under cracking conditions of 0.17 MPaG and 825°C to obtain a naphtha cracking fraction. The naphtha cracking fraction is introduced to a first cooling tower where the fraction is brought into contact with a mixture of heavy oil and cracked gasoline and thereby cooled to 110°C to obtain a first cooling fraction. Subsequently, the first cooling fraction is introduced to a second cooling tower where the fraction is brought into contact with cooling water to obtain a second cooling fraction of 0.11 MPaG and 41°C. The second cooling fraction is pressure-boosted to 0.99 MPaG with a compressor and introduced to a soda washing tower to obtain an introduction fraction to be introduced to a cryogenic separation step. The composition of the naphtha cracking fraction and the introduction fraction is shown in Table 1.

[Reference Example A2]

In Reference Example A2, a naphtha cracking fraction is obtained in the same manner as the method shown in Reference Example A1 except that the temperature of the cracking furnace is changed to 840°C; and the amount of naphtha supplied is adjusted depending on the amount of ethylene produced. The composition of the naphtha cracking fraction is shown in Table 1.

[Reference Example A3]

In Reference Example A3, a naphtha cracking fraction is obtained in the same manner as the method shown in Reference Example A1 except that heavy naphtha is changed to light naphtha; and the amount of naphtha supplied is adjusted depending on the amount of ethylene produced. The composition of the naphtha cracking fraction is shown in Table 1.

[Reference Example A4]

In Reference Example A4, a naphtha cracking fraction is obtained in the same manner as the method shown in Reference Example A3 except that the temperature of the cracking furnace is changed to 840°C; and the amount of naphtha supplied is adjusted depending on the amount of ethylene produced. The composition of the naphtha cracking fraction is shown in Table 1.

As is evident from Reference Examples A1 to A4, although the yield of methane or hydrogen varies depending on the type of the naphtha feedstock for use in naphtha cracking and the temperature of the cracking furnace, the smallest value of Oc is obtained when the cracking step is carried out at 825°C with heavy naphtha as a starting material.

[Reference Example A5]

A starting material containing 23.6% by mass of ethanol, 15.1% by mass of ethylene, and 16.0% by mass of olefin having 4 to 6 carbon atoms is heated, supplied at WHSV = 3.8 to a reactor packed with a zeolite-containing catalyst, and subjected to ethanol conversion reaction to obtain an ethanol conversion fraction. The starting material contains, in addition to those described above, paraffin, olefin having 7 or more carbon atoms, and water. In this respect, a heater temperature was adjusted such that an inlet-outlet average reaction temperature was 540°C. The ethanol conversion reaction is carried out for 48 hours. The composition of the ethanol conversion fraction at 24 hours from the start of reaction is converted to that per 100.0 ton/y in terms of the amount of ethylene produced, and shown in Table 1. The value of $O_E$ was constantly 0.6 throughout 48 hours.

As is evident from the comparison of Reference Examples A1 to A4 with Reference Example A5, the yields of methane and hydrogen are lower in the case of obtaining light olefin from a starting material containing ethanol than in the case of obtaining the same amount of ethylene by naphtha cracking.

[Table 1]

Table 1

| | | Reference Example A1 | | Reference Example A2 | Reference Example A3 | Reference Example A4 | Reference Example A5 |
|---|---|---|---|---|---|---|---|
| Naphtha feedstock | | Heavy naphtha | | Heavy naphtha | Light naphtha | Light naphtha | - |
| Amount of starting material | ton/y | 357.3 | | 319.8 | 346.9 | 284.6 | 547.4 |
| Cracking furnace temperature | °C | 825 | | 840 | 825 | 840 | - |
| Cracking furnace pressure | MPaG | 0.17 | | 0.17 | 0.17 | 0.17 | - |
| Composition of fraction | | Naphtha cracking fraction | Introduction fraction | Naphtha cracking fraction | Naphtha cracking fraction | Naphtha cracking fraction | Ethanol conversion fraction |
| Hydrogen | ton/y | 2.0 | 2.0 | 2.8 | 3.5 | 3.6 | 0.2 |
| Methane | ton/y | 48.6 | 48.3 | 54.0 | 55.2 | 55.9 | 0.8 |
| Acetylene | ton/y | 1.9 | 1.9 | 2.2 | 1.7 | 1.7 | 0.0 |
| Ethylene | ton/y | 100.0 | 100.0 | 100.0 | 100.0 | 100.0 | 100.0 |
| Ethane | ton/y | 16.7 | 16.8 | 32.1 | 16.7 | 32.4 | 3.3 |
| Propylene | ton/y | 62.3 | 62.0 | 47.3 | 68.4 | 47.7 | 62.9 |
| Propane | ton/y | 1.4 | 1.4 | 2.2 | 1.4 | 2.5 | 4.5 |
| Butenes | ton/y | 42.5 | 22.3 | 34.8 | 52.3 | 33.9 | 53.5 |
| Butanes | ton/y | 1.3 | 0.7 | 0.9 | 2.5 | 1.2 | 27.9 |
| Pentenes | ton/y | 17.8 | 4.7 | 8.9 | 33.0 | 13.8 | 27.5 |
| Pentanes | ton/y | 0.2 | 0.0 | 0.1 | 0.5 | 0.1 | 43.3 |
| Cracked gasoline | ton/y | 68.2 | 1.0 | 47.1 | 21.1 | 13.8 | 62.1 |
| Others | ton/y | 187.8 | 3.7 | 175.0 | 183.4 | 157.5 | 152.8 |
| Total | ton/y | 550.6 | 264.7 | 507.2 | 539.6 | 464.0 | 538.6 |
| P/E mass ratio | | 0.62 | | 0.47 | 0.68 | 0.48 | 0.63 |
| $O_C$ (% by mass) | | 31.1 | | 38.6 | 34.8 | 40.3 | - |
| $O_E$ (% by mass) | | - | | - | - | - | 0.6 |
| C2-3 olefin treatment efficiency | | 61.2 | | - | - | - | - |
| C2-3 olefin treatment efficiency index | | 1.00 | | - | - | - | - |
| C3L volumetric efficiency | | 52.0 | | - | - | - | - |
| C3L volumetric efficiency index | | 1.00 | | - | - | - | - |

In Examples A1 to A13 and Examples B1 to B13 given below, an ethanol conversion fraction refining step and the position of a combination step in a naphtha cracking process will be discussed. In Examples A1 to A13 and Examples B1 to B13, the ethanol conversion step is carried out in the same manner as in Reference Example A5, and the cracking step is carried out in the same manner as in Reference Example A1. In this respect, $(P/E_E - P/Ec)$ is 0.01, and $|C_{E_AP} - C_{C_AP}|$ is 0.8% by mass. Thus, the expression ($\alpha$) and the expression ($\beta$-1) are satisfied. The composition of a starting material in ethanol conversion is shown in Example A1.

[Example A1]

This Example is carried out with the light olefin production equipment shown in Figure 2.

(Ethanol conversion)

209.2 ton/y of a starting material containing 23.6% by mass of ethanol, 15.1% by mass of ethylene, 16.0% by mass of olefin having 4 to 6 carbon atoms, 13.6% by mass of paraffin having 4 to 6 carbon atoms, 4.2% by mass of an aromatic compound, and 16.7% by mass of water is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain 123.0 ton/y of a cooled ethanol conversion fraction of 35°C and 0.15 MPaG. 40.9 ton/y of the cooled ethanol conversion fraction is used as a recycling fraction to be recycled in a mixed starting material without steps such as separation and refining. In this respect, a product ratio in the recycling fraction is 49.4% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooling fraction, which is then introduced to a second cooling tower 13 where the fraction is brought into contact with cooling water to obtain a second cooling fraction of 0.11 MPaG and 41°C. The second cooling fraction is introduced to a pressure booster 14 and pressure-boosted to 0.99 MPaG.

(Combination of ethanol conversion with naphtha cracking)

253.2 ton/y of the pressure-boosted second cooling fraction is mixed with 82.1 ton/y of the cooled ethanol conversion fraction to prepare a combination fraction. The combination fraction is introduced to a soda washing tower 15 where the fraction is brought into contact with an aqueous sodium hydroxide solution to obtain a washing fraction. The washing fraction is introduced as an introduction fraction to a cryogenic separation step. In this Example, produced ethylene is 100.0 ton/y contained in the introduction fraction. The composition of the introduction fraction is shown in Table 2. As is evident from the value of a C2-3 olefin treatment efficiency index, light olefin treatment efficiency is improved in the cryogenic separation step. In addition, as is evident from the value of a C3L volumetric efficiency index, light olefin treatment efficiency per volume of the fraction introduced to the cryogenic separation step is improved.

[Example A2]

This Example is carried out with the light olefin production equipment shown in Figure 4.

(Ethanol conversion)

139.7 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a second distillation tower 24 to obtain a light ethanol conversion fraction from the tower top of the second distillation tower 24. The light ethanol conversion fraction is introduced to a condenser 25 where a portion of the fraction is liquefied and refluxed to the second distillation tower 24 to obtain a cooled light ethanol conversion fraction of 1.90 MPaG and 15°C as a gas. In this respect, 47.5 ton/y of a heavy ethanol conversion fraction obtained from the tower bottom of the second distillation tower 24 is used as a recycling fraction to be recycled in the starting material for introduction to the reactor 21 without additional steps such as refining. In this respect, a product ratio in the recycling fraction is 0.7% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking

fraction. The naphtha cracking fraction is introduced to a first cooling tower 12 where the fraction is brought into contact with a mixture of heavy oil and cracked gasoline and thereby cooled to 110°C to obtain a first cooling fraction, which is then introduced to a second cooling tower 13 where the fraction is brought into contact with cooling water to obtain a second cooling fraction of 0.11 MPaG and 41°C. The second cooling fraction is introduced to a pressure booster 14 and pressure-boosted to 0.99 MPaG.

(Combination of ethanol conversion with naphtha cracking)

253.2 ton/y of the second cooling fraction is mixed with 45.4 ton/y of the cooled light ethanol conversion fraction to prepare a combination fraction. The combination fraction is introduced to a soda washing tower 15 where the fraction is brought into contact with an aqueous sodium hydroxide solution to obtain a washing fraction. The washing fraction is further pressure-boosted and thereby introduced as an introduction fraction to a cryogenic separation step. In this Example, produced ethylene is 100.0 ton/y contained in the introduction fraction. The flow rate and composition of the introduction fraction are shown in Table 2.

As is evident from the comparison of this Example with Example A1, a distillation step of introducing the fraction obtained by ethanol conversion to the second distillation tower 24 to separate a light ethanol conversion fraction and a heavy ethanol conversion fraction is established, whereby the amount of the fraction introduced to the cryogenic separation step is reduced, and energy efficiency related to cryogenic separation can be improved. Furthermore, the distillation step is thus established, whereby the product ratio in the recycling fraction to be recycled as a mixed starting material is reduced, and light olefin such as ethylene or propylene can be efficiently produced from a smaller amount of the mixed starting material.

[Example A3]

The same procedures as in Example A2 are carried out except that the naphtha cracking-derived fraction and the ethanol conversion fraction are combined in amounts that attain 50.0 ton/y of ethylene obtained by ethanol conversion and 50.0 ton/y of ethylene obtained by naphtha cracking in 100.0 ton/y of ethylene to be introduced to the cryogenic separation step. The flow rate and composition of the introduction fraction are shown in Table 2.

[Example A4]

The same procedures as in Example A2 are carried out except that the naphtha cracking-derived fraction and the ethanol conversion fraction are combined in amounts that attain 75.0 ton/y of ethylene obtained by ethanol conversion and 25.0 ton/y of ethylene obtained by naphtha cracking in 100.0 ton/y of ethylene to be introduced to the cryogenic separation step. The flow rate and composition of the introduction fraction are shown in Table 2.

[Example A5]

This Example is carried out with the light olefin production equipment shown in Figure 5.

(Ethanol conversion)

145.6 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a gas-liquid separation drum to obtain a 51.3 ton/y of a light ethanol conversion fraction of 1.90 MPaG and 40°C as a gas. In this respect, 49.5 ton/y of a heavy ethanol conversion fraction obtained as a liquid in the gas-liquid separation drum 26 is used as a recycling fraction to be recycled in the starting material for introduction to the reactor 21 without additional steps such as refining. In this respect, a product ratio in the recycling fraction is 11.3% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction.

The first cooled naphtha cracking fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG.

(Combination of ethanol conversion with naphtha cracking)

253.2 ton/y of the second cooled naphtha cracking fraction is mixed with 51.3 ton/y of the light ethanol conversion fraction to prepare a combination fraction. The combination fraction is introduced to a soda washing tower 15 where the fraction is brought into contact with an aqueous sodium hydroxide solution to obtain a washing fraction. The washing fraction is introduced as an introduction fraction to a cryogenic separation step. In this Example, the amount of ethylene produced is the total amount of ethylene contained in the introduction fraction and is 100.0 ton/y. The flow rate and composition of the introduction fraction are shown in Table 2.

[Table 2-1]

Table 2(1/2)

| | | Example A1 | | | | Example A2 | | | | Example A3 | | | |
|---|---|---|---|---|---|---|---|---|---|---|---|---|---|
| Properties of each fraction | | Naphtha cracking-derived | Ethanol conversion-derived | Total | Difference | Naphtha cracking-derived | Ethanol conversion-derived | Total | Difference | Naphtha cracking-derived | Ethanol conversion-derived | Total | Difference |
| Amount of starting material used | ton/y | 266.5 | 209.2 | 475.7 | - | 266.5 | 139.7 | 406.2 | - | 177.7 | 279.3 | 457.0 | - |
| Temperature | °C | 41 | 35 | - | 6 | 41 | 15 | - | 26 | 41 | 15 | - | 26 |
| Pressure | MPaG | 0.99 | 0.15 | - | 0.84 | 0.99 | 1.90 | - | 1.01 | 0.99 | 1.90 | - | 1.01 |
| Product ratio in recycling fraction | % by mass | 49.4 | | | | 0.7 | | | | 0.7 | | | |
| Composition of fraction | | Naphtha cracking-derived | Ethanol conversion-derived | Combination fraction | Introduction fraction | Naphtha cracking-derived | Ethanol conversion-derived | Combination fraction | Introduction fraction | Naphtha cracking-derived | Ethanol conversion-derived | Combination fraction | Introduction fraction |
| Hydrogen | ton/y | 1.5 | 0.0 | 1.5 | 1.5 | 1.5 | 0.0 | 1.5 | 1.5 | 1.0 | 0.0 | 1.0 | 1.0 |
| Methane | ton/y | 36.2 | 0.2 | 36.4 | 36.4 | 36.2 | 0.2 | 36.4 | 36.4 | 24.2 | 0.3 | 24.5 | 24.5 |
| Acetylene | ton/y | 1.4 | 0.0 | 1.4 | 1.4 | 1.4 | 0.0 | 1.4 | 1.4 | 0.9 | 0.0 | 0.9 | 0.9 |
| Ethylene | ton/y | 75.0 | 25.0 | 100.0 | 100.0 | 75.0 | 25.0 | 100.0 | 100.0 | 50.0 | 50.0 | 100.0 | 100.0 |
| Ethane | ton/y | 12.6 | 0.7 | 13.3 | 13.3 | 12.6 | 0.7 | 13.3 | 13.3 | 8.4 | 1.5 | 9.9 | 9.9 |
| Propylene | ton/y | 46.5 | 15.5 | 62.0 | 62.0 | 46.5 | 15.2 | 61.7 | 61.7 | 31.0 | 30.4 | 61.4 | 61.4 |
| Propane | ton/y | 1.0 | 1.1 | 2.2 | 2.2 | 1.0 | 1.1 | 2.1 | 2.1 | 0.7 | 2.2 | 2.9 | 2.9 |
| Butenes | ton/y | 31.7 | 13.0 | 44.7 | 23.6 | 31.7 | 2.3 | 34.0 | 17.9 | 21.1 | 4.5 | 25.7 | 13.5 |
| Butanes | ton/y | 0.9 | 3.7 | 4.7 | 2.5 | 0.9 | 0.9 | 1.9 | 1.0 | 0.6 | 1.9 | 2.5 | 1.3 |
| Pentenes | ton/y | 13.3 | 6.7 | 20.0 | 5.3 | 13.3 | 0.0 | 13.3 | 3.5 | 8.8 | 0.0 | 8.8 | 2.4 |
| Pentanes | ton/y | 0.1 | 11.2 | 11.4 | 2.8 | 0.1 | 0.0 | 0.1 | 0.0 | 0.1 | 0.0 | 0.1 | 0.0 |
| Cracked gasoline | ton/y | 28.9 | 2.4 | 31.3 | 0.9 | 28.9 | 0.0 | 28.9 | 0.7 | 19.3 | 0.0 | 19.3 | 0.5 |
| Others | ton/y | 4.0 | 2.5 | 6.5 | 4.5 | 4.0 | 0.0 | 4.0 | 2.8 | 2.7 | 0.0 | 2.7 | 1.9 |
| Total | ton/y | 253.2 | 82.1 | 335.3 | 256.3 | 253.2 | 45.4 | 298.6 | 242.4 | 168.8 | 90.8 | 259.6 | 220.1 |
| $C_C$ (% by mass) | | 48.0 | | | | 48.0 | | | | 48.0 | | | |
| $C_E$ (% by mass) | | 49.4 | | | | 88.5 | | | | 88.5 | | | |
| ($C_E$-$C_C$) | | 1.4 | | | | 40.5 | | | | 40.5 | | | |

(continued)

| Composition of fraction | Naphtha cracking-derived | Ethanol conversion-derived | Combination fraction | Introduction fraction | Naphtha cracking-derived | Ethanol conversion-derived | Combination fraction | Introduction fraction | Naphtha cracking-derived | Ethanol conversion-derived | Combination fraction | Introduction fraction |
|---|---|---|---|---|---|---|---|---|---|---|---|---|
| $O_C$ (% by mass) | 31.1 | | | | 31.1 | | | | 31.1 | | | |
| $O_E$ (% by mass) | 0.6 | | | | 0.6 | | | | 0.6 | | | |
| $O'_C$ (% by mass) | 31.0 | | | | 31.0 | | | | 31.0 | | | |
| $O'_E$ (% by mass) | 0.4 | | | | 0.4 | | | | 0.4 | | | |
| C2.3 olefin treatment efficiency | 63.2 | | | | 66.7 | | | | 73.3 | | | |
| C2.3 olefin treatment efficiency index | 1.03 | | | | 1.09 | | | | 1.20 | | | |
| C3L volumetric efficiency | 58.7 | | | | 58.7 | | | | 67.3 | | | |
| C3L volumetric efficiency index | 1.13 | | | | 1.13 | | | | 1.29 | | | |

[Table 2-2]

Table 2(2/2)

| | | Example A4 | | | | Example A5 | | | |
|---|---|---|---|---|---|---|---|---|---|
| Properties of each fraction | | Naphtha cracking-derived | Ethanol conversion-derived | Total | Difference | Naphtha cracking-derived | Ethanol conversion-derived | Total | Difference |
| Amount of starting material used | ton/y | 88.8 | 419.0 | 507.8 | - | 266.5 | 145.6 | 412.1 | - |
| Temperature | °C | 41 | 15 | - | 26 | 41 | 40 | - | 1 |
| Pressure | MPaG | 0.99 | 1.90 | - | 1.01 | 0.99 | 1.90 | - | 0.91 |
| Product ratio in recycling fraction | % by mass | 0.7 | | | | 11.3 | | | |
| Composition of fraction | | Naphtha cracking-derived | Ethanol conversion-derived | Combination fraction | Introduction fraction | Naphtha cracking-derived | Ethanol conversion-derived | Combination fraction | Introduction fraction |
| Hydrogen | ton/y | 0.5 | 0.0 | 0.5 | 0.5 | 1.5 | 0.0 | 1.5 | 1.5 |
| Methane | ton/y | 12.1 | 0.5 | 12.5 | 12.5 | 36.2 | 0.2 | 36.4 | 36.4 |
| Acetylene | ton/y | 0.5 | 0.0 | 0.5 | 0.5 | 1.4 | 0.0 | 1.4 | 1.4 |
| Ethylene | ton/y | 25.0 | 75.0 | 100.0 | 100.0 | 75.0 | 25.0 | 100.0 | 100.0 |
| Ethane | ton/y | 4.2 | 2.2 | 6.4 | 6.4 | 12.6 | 0.8 | 13.4 | 13.4 |
| Propylene | ton/y | 15.5 | 45.6 | 61.1 | 61.1 | 46.5 | 12.7 | 59.2 | 59.2 |
| Propane | ton/y | 0.3 | 3.3 | 3.7 | 3.7 | 1.0 | 0.9 | 1.9 | 1.9 |
| Butenes | ton/y | 10.6 | 6.8 | 17.4 | 9.2 | 31.7 | 6.0 | 37.7 | 19.9 |
| Butanes | ton/y | 0.3 | 2.8 | 3.1 | 1.7 | 0.9 | 2.9 | 3.8 | 2.0 |
| Pentenes | ton/y | 4.4 | 0.0 | 4.4 | 1.2 | 13.3 | 0.9 | 14.2 | 3.8 |
| Pentanes | ton/y | 0.0 | 0.0 | 0.0 | 0.0 | 0.1 | 1.6 | 1.7 | 0.4 |
| Cracked gasoline | ton/y | 9.6 | 0.0 | 9.6 | 0.2 | 28.9 | 0.2 | 29.1 | 0.8 |
| Others | ton/y | 1.3 | 0.0 | 1.3 | 0.9 | 4.0 | 0.1 | 4.2 | 2.9 |
| Total | ton/y | 84.4 | 136.2 | 220.6 | 197.8 | 253.2 | 51.3 | 304.5 | 243.5 |
| $C_C$ (% by mass) | | 48.0 | | | | 48.0 | | | |
| $C_E$ (% by mass) | | 88.5 | | | | 73.5 | | | |
| ($C_E$-$C_C$) | | 40.5 | | | | 25.6 | | | |

(continued)

| Composition of fraction | Naphtha cracking-derived | Ethanol conversion-derived | Combination fraction | Introduction fraction | Naphtha cracking-derived | Ethanol conversion-derived | Combination fraction | Introduction fraction |
|---|---|---|---|---|---|---|---|---|
| Oc (% by mass) | 31.1 | | | | 31.1 | | | |
| $O_E$ (% by mass) | 0.6 | | | | 0.6 | | | |
| $O'_C$ (% by mass) | 31.0 | | | | 31.0 | | | |
| $O'_E$ (% by mass) | 0.4 | | | | 0.7 | | | |
| C2-3 olefin treatment efficiency | 81.4 | | | | 65.4 | | | |
| C2-3 olefin treatment efficiency index | 1.33 | | | | 1.07 | | | |
| C3L volumetric efficiency | 78.9 | | | | 58.3 | | | |
| C3L volumetric efficiency index | 1.52 | | | | 1.12 | | | |

[Example A6]

This Example is carried out with the light olefin production equipment shown in Figure 6.

(Ethanol conversion)

209.2 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain 138.6 ton/y of a cooled ethanol conversion fraction of 45°C and 15 MPaG. 46.1 ton/y of the cooled ethanol conversion fraction is used as a recycling fraction to be recycled in a mixed starting material without steps such as separation and refining. In this respect, a product ratio in the recycling fraction is 43.8% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooling fraction.

(Combination of ethanol conversion with naphtha cracking)

474.1 ton/y of the first cooling fraction is mixed with 92.5 ton/y of the cooled ethanol conversion fraction to prepare a combination fraction. The combination fraction is introduced to a second cooling tower 13 where the fraction is brought into contact with cooling water to obtain a second cooling fraction of 41°C. The second cooling fraction is introduced to a pressure booster 14 and pressure-boosted to 0.99 MPaG. The pressure-boosted second cooling fraction is introduced to a soda washing tower 15 where the fraction is brought into contact with an aqueous sodium hydroxide solution to obtain a washing fraction. The washing fraction is further pressure-boosted and thereby introduced as an introduction fraction to a cryogenic separation step. In this Example, produced ethylene is 100.0 ton/y contained in the introduction fraction. The flow rate and composition of the introduction fraction are shown in Table 3.

[Example A7]

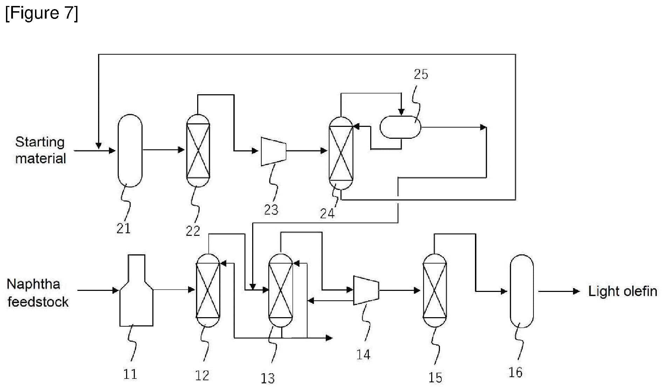

This Example is carried out with the light olefin production equipment shown in Figure 7.

(Ethanol conversion)

139.7 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a second distillation tower 24 to obtain a light ethanol conversion fraction from the tower top of the second distillation tower 24. The light ethanol conversion fraction is introduced to a condenser 25 where a portion of the fraction is liquefied and refluxed to the second distillation tower 24 to obtain a cooled light ethanol conversion fraction of 1.90 MPaG and 15°C as a gas. In this respect, 47.5 ton/y of a heavy ethanol conversion fraction obtained from the tower bottom of the second distillation tower 24 is used as a recycling fraction to be recycled in a mixed starting material without additional steps such as refining. In this respect, a product ratio in the recycling fraction is 0.7% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooling fraction.

(Combination of ethanol conversion with naphtha cracking)

474.1 ton/y of the first cooling fraction is mixed with 45.4 ton/y of the cooled light ethanol conversion fraction to prepare a combination fraction. The combination fraction is introduced to a second cooling tower 13 where the fraction is brought into contact with cooling water to obtain a second cooling fraction of 41°C. The second cooling fraction is introduced to a pressure booster 14 and pressure-boosted to 0.99 MPaG. The pressure-boosted second cooling fraction is introduced to a soda washing tower 15 where the fraction is brought into contact with an aqueous sodium hydroxide solution to obtain a washing fraction. The washing fraction is further pressure-boosted and thereby introduced as an introduction fraction to a cryogenic separation step. In this Example, produced ethylene is 100.0 ton/y contained in the introduction fraction. The flow rate and composition of the introduction fraction are shown in Table 3.

[Example A8]

This Example is carried out with the light olefin production equipment shown in Figure 8. This Example is carried out in the same manner as in Example A7 except that the location of connection is changed. In this Example, the cooled light ethanol conversion fraction of 15°C is combined with a second tower bottom reflux fraction of 68°C that includes a fraction obtained from the tower bottom of the second cooling tower 13 and is refluxed into the second cooling tower 13, to prepare a combination fraction. The amount of the second tower bottom reflux fraction is determined depending on the production balance of the compound of interest. In this Example, 52.6 ton/y of the second tower bottom reflux fraction is used.

Since the second tower bottom reflux fraction and the ethanol conversion fraction are combined so that the ethanol conversion fraction is introduced to the second cooling tower 13, the composition or flow rate of the resulting introduction fraction is not different between Example A7 and this Example. At the location, the naphtha cracking fraction and the ethanol conversion fraction are combined, whereby the difference in temperature between the fractions is 53K, which is smaller than 96K of Example A7, and change in volume associated with the liquefication of a high-temperature fraction can be suppressed.

[Example A9]

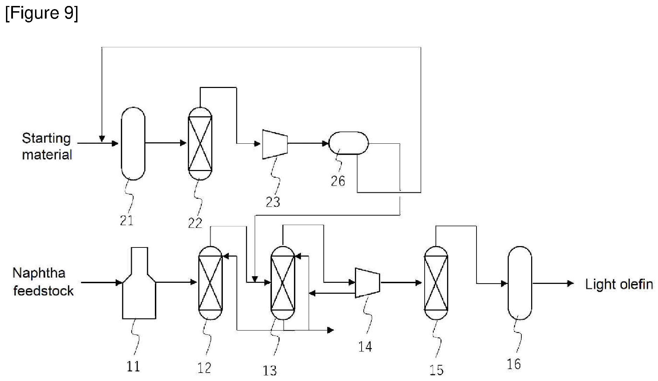

This Example is carried out with the light olefin production equipment shown in Figure 9.

(Ethanol conversion)

145.6 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a gas-liquid separation drum 26 to obtain a 51.3 ton/y of a light ethanol conversion fraction of 1.90 MPaG and 40°C as a gas. In this respect, 49.4 ton/y of a heavy ethanol conversion fraction obtained as a liquid in the gas-liquid separation drum 26 is used as a recycling fraction to be recycled in the starting material for introduction to the reactor 21 without additional steps such as refining. In this respect, a product ratio in the recycling fraction is 11.3% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction.

(Combination of ethanol conversion with naphtha cracking)

474.1 ton/y of the first cooled naphtha cracking fraction is mixed with 51.3 ton/y of the light ethanol conversion fraction to prepare a combination fraction. The combination fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG. The combination fraction is introduced to a soda washing tower where the fraction is brought into contact with an aqueous sodium hydroxide solution to obtain a washing fraction. The washing fraction is further pressure-boosted and

thereby introduced as an introduction fraction to a cryogenic separation step. The composition of the introduction fraction is shown in Table 3. In this Example, the amount of ethylene produced is the total amount of ethylene contained in the introduction fraction and is 100.0 ton/y.

[Table 3-1]

Table 3(1/2)

| | | Example A6 | | | | Example A7 | | | |
|---|---|---|---|---|---|---|---|---|---|
| Properties of each fraction | | Naphtha cracking-derived | Ethanol conversion-derived | Total | Difference | Naphtha cracking-derived | Ethanol conversion-derived | Total | Difference |
| Amount of starting material used | ton/y | 266.5 | 209.2 | 475.7 | - | 266.5 | 139.7 | 406.2 | - |
| Temperature | °C | 110 | 45 | - | 65 | 110 | 15 | - | 95 |
| Pressure | MPaG | 0.13 | 0.15 | - | 0.02 | 0.13 | 1.90 | - | 1.77 |
| Product ratio in recycling fraction | % by mass | 43.8 | | | | 0.7 | | | |
| Composition of fraction | | Naphtha cracking-derived | Ethanol conversion-derived | Combination fraction | Introduction fraction | Naphtha cracking-derived | Ethanol conversion-derived | Combination fraction | Introduction fraction |
| Hydrogen | ton/y | 1.5 | 0.0 | 1.5 | 1.5 | 1.5 | 0.0 | 1.5 | 1.5 |
| Methane | ton/y | 36.2 | 0.2 | 36.4 | 36.4 | 36.2 | 0.2 | 36.4 | 36.4 |
| Acetylene | ton/y | 1.4 | 0.0 | 1.4 | 1.4 | 1.4 | 0.0 | 1.4 | 1.4 |
| Ethylene | ton/y | 75.0 | 25.0 | 100.0 | 100.0 | 75.0 | 25.0 | 100.0 | 100.0 |
| Ethane | ton/y | 12.6 | 0.7 | 13.3 | 13.3 | 12.6 | 0.7 | 13.3 | 13.3 |
| Propylene | ton/y | 46.5 | 15.5 | 62.0 | 62.0 | 46.5 | 15.2 | 61.7 | 61.7 |
| Propane | ton/y | 1.0 | 1.1 | 2.2 | 2.2 | 1.0 | 1.1 | 2.1 | 2.1 |
| Butenes | ton/y | 31.7 | 13.0 | 44.7 | 23.6 | 31.7 | 2.3 | 34.0 | 17.9 |
| Butanes | ton/y | 0.9 | 3.7 | 4.7 | 2.5 | 0.9 | 0.9 | 1.9 | 1.0 |
| Pentenes | ton/y | 13.3 | 6.7 | 20.0 | 5.3 | 13.3 | 0.0 | 13.3 | 3.5 |
| Pentanes | ton/y | 0.1 | 11.2 | 11.4 | 2.8 | 0.1 | 0.0 | 0.1 | 0.0 |
| Cracked gasoline | ton/y | 113.8 | 12.8 | 126.6 | 0.9 | 113.8 | 0.0 | 113.8 | 0.7 |
| Others | ton/y | 140.1 | 2.5 | 142.6 | 2.8 | 140.1 | 0.0 | 140.1 | 2.8 |
| Total | ton/y | 474.1 | 92.5 | 566.6 | 254.6 | 474.1 | 45.4 | 519.5 | 242.4 |
| $C_C$ (% by mass) | | 25.6 | | | | 25.6 | | | |
| $C_E$ (% by mass) | | 43.8 | | | | 88.5 | | | |
| ($C_E$-$C_C$) | | 18.2 | | | | 62.9 | | | |

(continued)

| Composition of fraction | Naphtha cracking-derived | Ethanol conversion-derived | Combination fraction | Introduction fraction | Naphtha cracking-derived | Ethanol conversion-derived | Combination fraction | Introduction fraction |
|---|---|---|---|---|---|---|---|---|
| $O_C$ (% by mass) | 31.1 | | | | 31.1 | | | |
| $O_E$ (% by mass) | 0.6 | | | | 0.6 | | | |
| $O'_C$ (% by mass) | 31.0 | | | | 31.0 | | | |
| $O'_E$ (% by mass) | 0.4 | | | | 0.4 | | | |
| C2-3 olefin treatment efficiency | 63.6 | | | | 66.7 | | | |
| C2-3 olefin treatment efficiency index | 1.04 | | | | 1.09 | | | |
| C3L volumetric efficiency | 58.7 | | | | 58.7 | | | |
| C3L volumetric efficiency index | 1.13 | | | | 1.13 | | | |

[Table 3-2]

| Table 3(2/2) | | | | | | | | | |
|---|---|---|---|---|---|---|---|---|---|
| | | Example A8 | | | | Example A9 | | | |
| Properties of each fraction | | Naphtha cracking-derived | Ethanol conversion-derived | Total | Difference | Naphtha cracking. derived | Ethanol conversion-derived | Total | Difference |
| Amount of starting material used | ton/y | 266.5 | 139.7 | 406.2 | - | 266.5 | 145.6 | 412.1 | - |
| Temperature | °C | 68 | 15 | - | 53 | 110 | 40 | - | 70 |
| Pressure | MPaG | 0.21 | 1.90 | - | 1.69 | 0.13 | 1.90 | - | 1.77 |
| Product ratio in recycling fraction | % by mass | 0.7 | | | | 11.3 | | | |
| Composition of fraction | | Naphtha cracking-derived | Ethanol conversion. derived | Combination fraction | Introduction fraction | Naphtha cracking. derived | Ethanol conversion-derived | Combination fraction | Introduction fraction |
| Hydrogen | ton/y | 0.0 | 0.0 | 0.0 | 1.5 | 1.5 | 0.0 | 1.5 | 1.5 |
| Methane | ton/y | 0.0 | 0.2 | 0.2 | 36.4 | 36.2 | 0.2 | 36.4 | 36.4 |
| Acetylene | ton/y | 0.0 | 0.0 | 0.0 | 1.4 | 1.4 | 0.0 | 1.4 | 1.4 |
| Ethylene | ton/y | 0.2 | 25.0 | 25.2 | 100.0 | 75.0 | 25.0 | 100.0 | 100.0 |
| Ethane | ton/y | 0.0 | 0.7 | 0.8 | 13.3 | 12.6 | 0.8 | 13.4 | 13.4 |
| Propylene | ton/y | 0.4 | 15.2 | 15.6 | 61.7 | 46.5 | 12.7 | 59.2 | 59.2 |
| Propane | ton/y | 0.0 | 1.1 | 1.1 | 2.1 | 1.0 | 0.9 | 1.9 | 1.9 |
| Butenes | ton/y | 1.0 | 2.3 | 3.2 | 17.9 | 31.7 | 6.0 | 37.7 | 19.9 |
| Butanes | ton/y | 0.0 | 0.9 | 1.0 | 1.0 | 0.9 | 2.9 | 3.8 | 2.0 |
| Pentenes | ton/y | 1.0 | 0.0 | 1.0 | 3.5 | 13.3 | 0.9 | 14.2 | 3.8 |
| Pentanes | ton/y | 0.0 | 0.0 | 0.0 | 0.0 | 0.1 | 1.6 | 1.7 | 0.4 |
| Cracked gasoline | ton/y | 36.8 | 0.0 | 36.8 | 0.7 | 113.8 | 0.2 | 114.0 | 0.7 |
| Others | ton/y | 13.1 | 0.0 | 13.1 | 2.8 | 140.1 | 0.1 | 140.2 | 2.8 |
| Total | ton/y | 52.6 | 45.4 | 98.0 | 242.4 | 474.1 | 51.3 | 525.4 | 243.5 |
| $C_C$ (% by mass) | | 1.1 | | | | 25.6 | | | |
| $C_E$ (% by mass) | | 88.5 | | | | 73.5 | | | |
| $(C_E-C_C)$ | | 87.4 | | | | 47.9 | | | |

(continued)

| Composition of fraction | Naphtha cracking-derived | Ethanol conversion. derived | Combination fraction | Introduction fraction | Naphtha cracking. derived | Ethanol conversion-derived | Combination fraction | Introduction fraction |
|---|---|---|---|---|---|---|---|---|
| $O_C$ (% by mass) | 31.1 | | | | 31.1 | | | |
| $O_E$ (% by mass) | 0.6 | | | | 0.6 | | | |
| $O'_C$ (% by mass) | 3.9 | | | | 31.0 | | | |
| $O'_E$ (% by mass) | 0.4 | | | | 0.7 | | | |
| C2-3 olefin treatment efficiency | 66.7 | | | | 65.4 | | | |
| C2-3 olefin treatment efficiency index | 1.09 | | | | 1.07 | | | |
| C3L volumetric efficiency | 58.7 | | | | 58.3 | | | |
| C3L volumetric efficiency index | 1.13 | | | | 1.12 | | | |

[Example A10]

This Example is carried out with the light olefin production equipment shown in Figure 10.

(Ethanol conversion)

209.2 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain 138.6 ton/y of a cooled ethanol conversion fraction of 45°C and 0.15 MPaG. 46.1 ton/y of the cooled ethanol conversion fraction is used as a recycling fraction to be recycled in a mixed starting material without steps such as separation and refining. In this respect, a product ratio in the recycling fraction is 43.8% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction.

(Combination of ethanol conversion with naphtha cracking)

417.2 ton/y of the naphtha cracking fraction of 360°C is mixed with 92.5 ton/y of the cooled ethanol conversion fraction to prepare a combination fraction. The combination fraction is introduced to a first cooling tower 12 where the fraction is brought into contact with a mixture of heavy oil and cracked gasoline and thereby cooled to 110°C to obtain a first cooling fraction. The first cooling fraction is introduced to a second cooling tower 13 where the fraction is brought into contact with cooling water to obtain a second cooling fraction of 41°C. The second cooling fraction is introduced to a pressure booster 14 and pressure-boosted to 0.99 MPaG. The pressure-boosted second cooling fraction is introduced to a soda washing tower 15 where the fraction is brought into contact with an aqueous sodium hydroxide solution to obtain a washing fraction. The washing fraction is further pressure-boosted and thereby introduced as an introduction fraction to a cryogenic separation step. In this Example, produced ethylene is 100.0 ton/y contained in the introduction fraction. The flow rate and composition of the introduction fraction are shown in Table 4.

[Example A11]

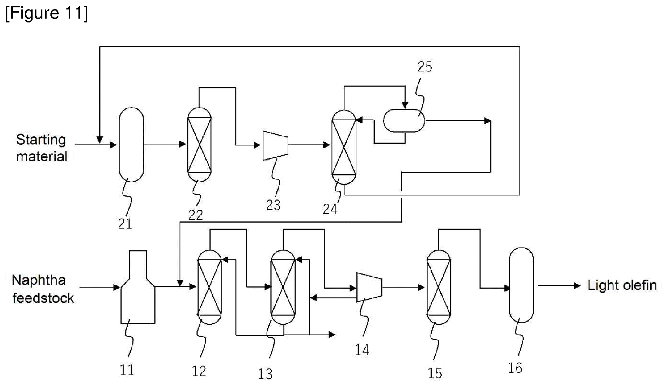

This Example is carried out with the light olefin production equipment shown in Figure 11.

(Ethanol conversion)

139.7 ton/y of a starting material was heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a second distillation tower 24 to obtain a light ethanol conversion fraction from the tower top of the second distillation tower 24. The light ethanol conversion fraction is introduced to a condenser 25 where a portion of the fraction is liquefied and refluxed to the second distillation tower 24 to obtain a cooled light ethanol conversion fraction of 1.90 MPaG and 15°C as a gas. In this respect, 47.5 ton/y of a heavy ethanol conversion fraction obtained from the tower bottom of the second distillation tower 24 is used as a recycling fraction to be recycled in a mixed starting material without additional steps such as refining. In this respect, a product ratio in the recycling fraction is 0.7% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction.

(Combination of ethanol conversion with naphtha cracking)

417.2 ton/y of the naphtha cracking fraction of 360°C is mixed with 45.4 ton/y of the cooled light ethanol conversion fraction to prepare a combination fraction. The combination fraction is introduced to a first cooling tower 12 where the fraction is brought into contact with a mixture of heavy oil and cracked gasoline and thereby cooled to 110°C to obtain a first cooling fraction. The first cooling fraction is introduced to a second cooling tower 13 where the fraction is brought into contact with cooling water to obtain a second cooling fraction of 41°C. The second cooling fraction is introduced to a pressure booster 14 and pressure-boosted to 0.99 MPaG. The pressure-boosted second cooling fraction is introduced to a soda washing tower 15 where the fraction is brought into contact with an aqueous sodium hydroxide solution to obtain a washing fraction. The washing fraction is further pressure-boosted and thereby introduced as an introduction fraction to a cryogenic separation step. In this Example, produced ethylene is 100.0 ton/y contained in the introduction fraction. The flow rate and composition of the introduction fraction are shown in Table 4.

[Example A12]

This Example is carried out with the light olefin production equipment shown in Figure 12. This Example is carried out in the same manner as in Example A9 except that the location of connection is changed. In this Example, the cooled light ethanol conversion fraction of 15°C is combined with a first tower bottom reflux fraction of 80°C that is obtained from the tower bottom of the second cooling tower 13 and refluxed into the first cooling tower 12, to prepare a combination fraction.

Since the first tower bottom reflux fraction and the ethanol conversion fraction are combined so that the ethanol conversion fraction is introduced to the first cooling tower 12, the composition or flow rate of the resulting introduction fraction is not different between Example A9 and Example A10. At the location, the naphtha cracking fraction and the ethanol conversion fraction are combined, whereby the difference in temperature between the fractions is 65K, which is smaller than 345K of Example A9, and change in volume associated with the liquefication of a high-temperature fraction can be suppressed.

[Example A13]

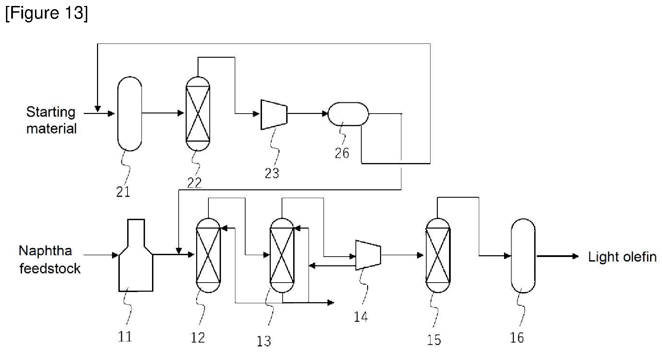

This Example is carried out with the light olefin production equipment shown in Figure 13.

(Ethanol conversion)

145.6 ton/y of a mixed starting material gas is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a gas-liquid separation drum 26 to obtain a 51.3 ton/y of a light ethanol conversion fraction of 1.90 MPaG and 40°C as a gas. In this respect, 49.4 ton/y of a heavy ethanol conversion fraction obtained as a liquid in the gas-liquid separation drum 26 is used as a recycling fraction to be recycled in the starting material for introduction to the reactor 21 without additional steps such as refining. In this respect, a product ratio in the recycling fraction is 11.3% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction.

(Combination of ethanol conversion with naphtha cracking)

417.2 ton/y of the naphtha cracking fraction is mixed with 51.3 ton/y of the light ethanol conversion fraction to prepare a combination fraction. The combination fraction is introduced to a first cooling tower 12 where the fraction is brought into contact with a mixture of heavy oil and cracked gasoline and thereby cooled to 110°C to obtain a first cooled naphtha cracking fraction. This fraction is introduced to the first cooling tower 12 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG. The pressure-boosted second cooled naphtha cracking fraction is introduced to a soda washing tower 15 where the fraction is brought

into contact with an aqueous sodium hydroxide solution to obtain a washing fraction. The composition of the introduction fraction is shown in Table 4. The washed combination fraction is further pressure-boosted and thereby introduced as an introduction fraction to a cryogenic separation step. In this Example, the amount of ethylene produced is the total amount of ethylene contained in the introduction fraction and is 100.0 ton/y.

[Table 4]

Table 4

| | | Example A10 | | | | Example A11 | | | | Example A13 | | | |
|---|---|---|---|---|---|---|---|---|---|---|---|---|---|
| Properties of each fraction | | Naphtha cracking. derived | Ethanol conversion-derived | Total | Difference | Naphtha cracking. derived | Ethanol conversion-derived | Total | Difference | Naphtha cracking. derived | Ethanol conversion-derived | Total | Difference |
| Amount of starting material used | ton/y | 266.5 | 209.2 | 377.8 | - | 266.5 | 139.7 | 377.8 | - | 266.5 | 145.6 | 377.8 | - |
| Temperature | °C | 360 | 45 | - | 315 | 360 | 15 | - | 345 | 360 | 40 | - | 320 |
| Pressure | MPaG | 0.15 | 0.15 | - | 0.00 | 0.15 | 1.90 | - | 1.75 | 0.15 | 1.90 | - | 1.75 |
| Product ratio in recycling fraction | % by mass | 43.8 | | | | 0.7 | | | | 11.3 | | | |
| Composition of fraction | | Naphtha cracking. derived | Ethanol conversion-derived | Combination fraction | Introduction fraction | Naphtha cracking. derived | Ethanol conversion-derived | Combination fraction | Introduction fraction | Naphtha cracking. derived | Ethanol conversion-derived | Combination fraction | Introduction fraction |
| Hydrogen | ton/y | 1.5 | 0.0 | 1.5 | 1.5 | 1.5 | 0.0 | 1.5 | 1.5 | 1.5 | 0.0 | 1.5 | 1.5 |
| Methane | ton/y | 36.2 | 0.2 | 36.4 | 36.4 | 36.2 | 0.2 | 36.4 | 36.4 | 36.2 | 0.2 | 36.4 | 36.4 |
| Acetylene | ton/y | 1.4 | 0.0 | 1.4 | 1.4 | 1.4 | 0.0 | 1.4 | 1.4 | 1.4 | 0.0 | 1.4 | 1.4 |
| Ethylene | ton/y | 75.0 | 25.0 | 100.0 | 100.0 | 75.0 | 25.0 | 100.0 | 100.0 | 75.0 | 25.0 | 100.0 | 100.0 |
| Ethane | ton/y | 12.6 | 0.7 | 13.3 | 13.3 | 12.6 | 0.7 | 13.3 | 13.3 | 12.6 | 0.8 | 13.3 | 13.4 |
| Propylene | ton/y | 46.5 | 15.5 | 62.0 | 62.0 | 46.5 | 15.2 | 61.7 | 61.7 | 46.5 | 12.7 | 61.7 | 59.2 |
| Propane | ton/y | 1.0 | 1.1 | 2.2 | 2.2 | 1.0 | 1.1 | 2.1 | 2.1 | 1.0 | 0.9 | 2.1 | 1.9 |
| Butenes | ton/y | 31.7 | 13.0 | 44.7 | 23.6 | 31.7 | 2.3 | 34.0 | 17.9 | 31.7 | 6.0 | 34.0 | 19.9 |
| Butanes | ton/y | 0.9 | 3.7 | 4.7 | 2.5 | 0.9 | 0.9 | 1.9 | 1.0 | 0.9 | 2.9 | 1.9 | 2.0 |
| Pentenes | ton/y | 13.3 | 6.7 | 20.0 | 5.3 | 13.3 | 0.0 | 13.3 | 3.5 | 13.3 | 0.9 | 13.3 | 3.8 |
| Pentanes | ton/y | 0.1 | 11.2 | 11.4 | 2.8 | 0.1 | 0.0 | 0.1 | 0.0 | 0.1 | 1.6 | 0.1 | 0.4 |
| Cracked gasoline | ton/y | 50.9 | 12.8 | 63.7 | 0.9 | 50.9 | 0.0 | 50.9 | 0.7 | 50.9 | 0.2 | 50.9 | 0.7 |
| Others | ton/y | 146.1 | 2.5 | 148.5 | 2.8 | 146.1 | 0.0 | 146.1 | 2.8 | 146.1 | 0.1 | 146.1 | 2.8 |
| Total | ton/y | 417.2 | 92.5 | 509.7 | 254.6 | 417.2 | 45.4 | 462.6 | 242.4 | 417.2 | 51.3 | 462.6 | 243.5 |
| $C_C$ (% by mass) | | 29.1 | | | | 29.1 | | | | 29.1 | | | |
| $C_E$ (% by mass) | | 43.8 | | | | 88.5 | | | | 73.5 | | | |
| $(C_E-C_C)$ | | 14.7 | | | | 59.4 | | | | 44.4 | | | |

(continued)

| Composition of fraction | Naphtha cracking. derived | Ethanol conversion-derived | Combination fraction | Introduction fraction | Naphtha cracking. derived | Ethanol conversion-derived | Combination fraction | Introduction fraction | Naphtha cracking. derived | Ethanol conversion-derived | Combination fraction | Introduction fraction |
|---|---|---|---|---|---|---|---|---|---|---|---|---|
| $O_C$ (% by mass) | 31.1 | | | | 31.1 | | | | 31.1 | | | |
| $O_E$ (% by mass) | 0.6 | | | | 0.6 | | | | 0.6 | | | |
| $O'_C$ (% by mass) | 31.0 | | | | 31.0 | | | | 31.0 | | | |
| $O'_E$ (% by mass) | 0.4 | | | | 0.4 | | | | 0.7 | | | |
| C2-3 olefin treatment efficiency | 63.6 | | | | 66.7 | | | | 65.4 | | | |
| C2.3 olefin treatment efficiency index | 1.04 | | | | 1.09 | | | | 1.07 | | | |
| C3L volumetric efficiency | 58.7 | | | | 58.7 | | | | 58.3 | | | |
| C3L volumetric efficiency index | 1.13 | | | | 1.13 | | | | 1.12 | | | |

[Reference Example B1]

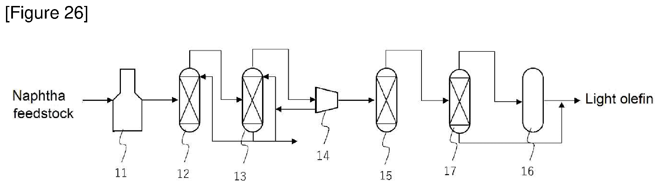

Reference Example B1 is carried out with the light olefin production equipment in the form of a front-end depropanizer shown in Figure 26. 357.3 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction. The first cooled naphtha cracking fraction is introduced to a second cooling tower 13 where the fraction is brought into contact with cooling water to obtain a second cooled naphtha cracking fraction of 0.11 MPaG and 41°C. 339.5 ton/y of the second cooled naphtha cracking fraction is introduced to a pressure booster 14, pressure-boosted to 0.99 MPaG, and introduced to a soda washing tower 15 to obtain a washing fraction. The washing fraction is further pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained from the tower bottom. The composition of the introduction fraction and the detour fraction is shown in Table 5.

In the form of a front-end depropanizer, the naphtha cracking fraction is separated into an introduction fraction mainly containing hydrocarbon having 3 or less carbon atoms and a detour fraction mainly containing hydrocarbon having 3 or more carbon atoms, whereby the amount of the fraction introduced to the cryogenic separation step is reduced, and energy consumption related to cryogenic separation can be reduced.

[Example B1]

This Example is carried out with the light olefin production equipment shown in Figure 15.

(Ethanol conversion)

209.2 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain 123.0 ton/y of a cooled ethanol conversion fraction of 35°C and 0.15 MPaG. 40.9 ton/y of the cooled ethanol conversion fraction is used as a recycling fraction to be recycled in the starting material for introduction to the reactor 21 without steps such as separation and refining. In this respect, a product ratio in the recycling fraction is 49.4% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction. The first cooled naphtha cracking fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG.

(Combination of ethanol conversion with naphtha cracking)

253.2 ton/y of the pressure-boosted second cooling fraction is mixed with 82.1 ton/y of the cooled ethanol conversion fraction to prepare a combination fraction. The combination fraction is introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 5. In this Example, the amount of ethylene produced is the total amount of ethylene contained in the introduction fraction and the detour fraction and is 100.0 ton/y.

[Example B2]

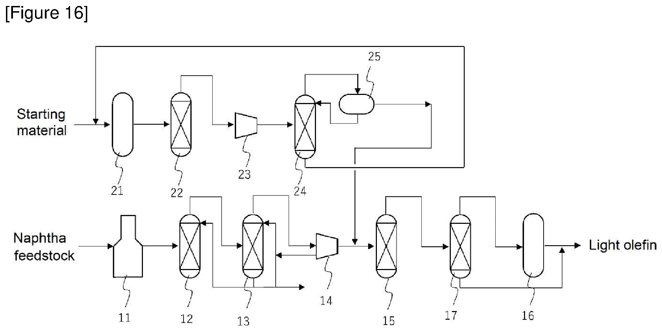

This Example is carried out with the light olefin production equipment shown in Figure 16.

(Ethanol conversion)

139.7 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a second distillation tower 24 to obtain a light ethanol conversion fraction from the tower top of the second distillation tower 24. The light ethanol conversion fraction is introduced to a condenser 25 where a portion of the fraction is liquefied and refluxed to the second distillation tower 24 to obtain a cooled light ethanol conversion fraction of 1.90 MPaG and 15°C as a gas. In this respect, 47.5 ton/y of a heavy ethanol conversion fraction obtained from the tower bottom of the second distillation tower 24 is used as a recycling fraction to be recycled in a mixed starting material without additional steps such as refining. In this respect, a product ratio in the recycling fraction is 0.7% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction. The first cooled naphtha cracking fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG.

(Combination of ethanol conversion with naphtha cracking)

253.2 ton/y of the second cooled naphtha cracking fraction is mixed with 45.4 ton/y of the cooled light ethanol conversion fraction to prepare a combination fraction. The combination fraction is introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 5. In this Example, the amount of ethylene produced is the total amount of ethylene contained in the introduction fraction and the detour fraction and is 100.0 ton/y.

As is evident from the comparison of this Example with Example B1, a fraction mainly containing light olefin is separated by distillation from the ethanol conversion fraction before the ethanol conversion fraction is combined with the naphtha cracking fraction, whereby the amount of the fraction introduced to the cryogenic separation step can be reduced, and energy efficiency related to refining can be improved.

As is evident from the comparison of this Example with Example B1, use of the cooled ethanol conversion fraction as a recycling fraction without additional steps such as separation and refining elevates a product ratio in the recycling fraction, increases the amount of the starting material necessary for production, and reduces ethylene production efficiency per starting material.

[Example B3]

The same procedures as in Example B2 are carried out except that the naphtha cracking-derived fraction and the ethanol conversion fraction are combined in amounts that attain 50.0 ton/y of ethylene obtained by ethanol conversion and 50.0 ton/y of ethylene obtained by naphtha cracking in 100.0 ton/y of ethylene to be produced. The composition of the introduction fraction and the detour fraction is shown in Table 5.

[Example B4]

The same procedures as in Example B2 are carried out except that the naphtha cracking-derived fraction and the

ethanol conversion fraction are combined in amounts that attain 75.0 ton/y of ethylene obtained by ethanol conversion and 25.0 ton/y of ethylene obtained by naphtha cracking in 100.0 ton/y of ethylene to be produced. The composition of the introduction fraction and the detour fraction is shown in Table 5.

[Example B5]

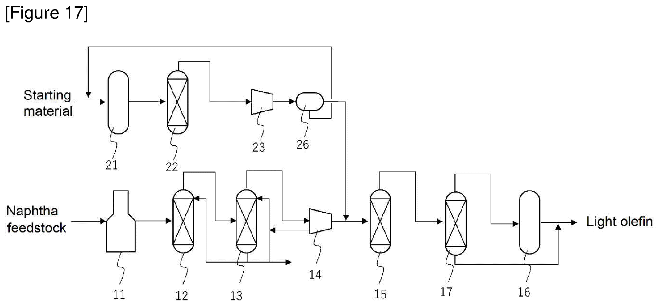

This Example is carried out with the light olefin production equipment shown in Figure 17.

(Ethanol conversion)

145.6 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a gas-liquid separation drum 26 to obtain a 51.3 ton/y of a light ethanol conversion fraction of 1.90 MPaG and 40°C as a gas. In this respect, 49.5 ton/y of a heavy ethanol conversion fraction obtained as a liquid in the gas-liquid separation drum 26 is used as a recycling fraction to be recycled in the starting material for introduction to the reactor 21 without additional steps such as refining. In this respect, a product ratio in the recycling fraction is 11.3% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction. The first cooled naphtha cracking fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG.

(Combination of ethanol conversion with naphtha cracking)

253.2 ton/y of the second cooled naphtha cracking fraction is mixed with 51.3 ton/y of the light ethanol conversion fraction to prepare a combination fraction. The combination fraction is introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 5. In this Example, the amount of ethylene produced is the total amount of ethylene contained in the introduction fraction and the detour fraction and is 100.0 ton/y.

[Table 5-1]

| Table 5(1/2) | | | | | | | | | | | | |
|---|---|---|---|---|---|---|---|---|---|---|---|---|
| | | Reference Example B1 | | | Example B1 | | | | Example B2 | | | |
| Properties of each fraction | | - | | | Naphtha cracking-derived | Ethanol conversion-derived | Total | Difference | Naphtha cracking-derived | Ethanol conversion-derived | Total | Difference |
| Amount of starting material used | ton/y | 357.3 | | | 266.5 | 209.2 | 475.7 | - | 266.5 | 139.7 | 406.2 | - |
| Temperature | °C | - | | | 41 | 35 | - | 6 | 41 | 15 | - | 26 |
| Pressure | MPaG | - | | | 0.99 | 0.15 | - | 0.84 | 0.99 | 1.90 | - | 0.91 |
| Amount introduced to soda washing tower | ton/y | 339.5 | | | 335.3 | | | | 298.6 | | | |
| Product ratio in recycling fraction | % by mass | - | | | 43.8 | | | | 0.7 | | | |
| Composition of fraction | | Naphtha cracking derived | Introduction fraction | Detour fraction | Naphtha cracking. derived | Ethanol conversion-derived | Introduction fraction | Detour fraction | Naphtha cracking-derived | Ethanol conversion-derived | Introduction fraction | Detour fraction |
| Hydrogen ton/y Methane | ton/y | 2.0 48.6 | 1.8 42.8 | 0.1 5.6 | 1.5 36.2 | 0.0 0.2 | 1.4 32.2 | 0.1 4.2 | 1.5 36.2 | 0.0 0.2 | 1.4 32.2 | 0.1 4.2 |
| Acetylene | ton/y | 1.9 | 0.0 | 1.9 | 1.4 | 0.0 | 0.0 | 1.4 | 1.4 | 0.0 | 0.0 | 1.4 |
| Ethylene | ton/y | 100.0 | 67.7 | 32.3 | 75.0 | 25.0 | 67.7 | 32.3 | 75.0 | 25.0 | 67.7 | 32.3 |
| Ethane | ton/y | 16.7 | 9.1 | 7.6 | 12.6 | 0.7 | 7.3 | 6.1 | 12.6 | 0.7 | 7.3 | 6.1 |
| Propylene | ton/y | 62.3 | 3.3 | 58.7 | 46.5 | 15.5 | 3.3 | 58.7 | 46.5 | 15.2 | 3.3 | 58.4 |
| Propane | ton/y | 1.4 | 0.1 | 1.3 | 1.0 | 1.1 | 0.1 | 2.1 | 1.0 | 1.1 | 0.1 | 2.0 |
| Butenes | ton/y | 42.5 | 0.0 | 22.3 | 31.7 | 13.0 | 0.0 | 23.6 | 31.7 | 2.3 | 0.0 | 17.9 |
| Butanes | ton/y | 1.3 | 0.0 | 0.7 | 0.9 | 3.7 | 0.0 | 2.5 | 0.9 | 0.9 | 0.0 | 1.0 |
| Pentenes | ton/y | 17.8 | 0.0 | 4.7 | 13.3 | 6.7 | 0.0 | 5.3 | 13.3 | 0.0 | 0.0 | 3.5 |
| Pentanes | ton/y | 0.2 | 0.0 | 0.0 | 0.1 | 11.2 | 0.0 | 2.8 | 0.1 | 0.0 | 0.0 | 0.0 |
| Cracked gasoline | ton/y | 68.2 | 0.0 | 1.0 | 28.9 | 2.4 | 0.0 | 0.9 | 28.9 | 0.0 | 0.0 | 0.7 |

(continued)

| Composition of fraction | | Naphtha cracking derived | Introduction fraction | Detour fraction | Naphtha cracking. derived | Ethanol conversion-derived | Introduction fraction | Detour fraction | Naphtha cracking-derived | Ethanol conversion-derived | Introduction fraction | Detour fraction |
|---|---|---|---|---|---|---|---|---|---|---|---|---|
| Others | ton/y | 187.8 | 0.1 | 3.6 | 4.0 | 2.5 | 0.2 | 4.3 | 4.0 | 0.0 | 0.1 | 2.7 |
| Total | ton/y | 550.6 | 124.9 | 139.8 | 253.2 | 82.1 | 112.0 | 144.2 | 253.2 | 45.4 | 112.0 | 130.4 |
| $C_C$ (% by mass) | | - | | | 48.0 | | | | 48.0 | | | |
| $C_E$ (% by mass) | | - | | | 49.4 | | | | 88.5 | | | |
| $(C_E-C_C)$ | | - | | | 1.4 | | | | 40.5 | | | |
| $O_C$ (% by mass) | | 31.1 | | | 31.1 | | | | 31.1 | | | |
| $O_E$ (% by mass) | | - | | | 0.6 | | | | 0.6 | | | |
| $O'_C$ (% by mass) | | - | | | 31.0 | | | | 31.0 | | | |
| $O'_E$ (% by mass) | | - | | | 0.4 | | | | 0.4 | | | |
| C2.3 olefin treatment efficiency | | 56.8 | | | 63.3 | | | | 63.3 | | | |
| C2-3 olefin treatment efficiency index | | 1.00 | | | 1.12 | | | | 1.12 | | | |
| C3L volumetric efficiency | | 39.1 | | | 46.0 | | | | 46.0 | | | |
| C3L volumetric efficiency index | | 1.00 | | | 1.17 | | | | 1.17 | | | |

[Table 5-2]

| Table 5(2/2) | | | | | | | | | | | | | |
|---|---|---|---|---|---|---|---|---|---|---|---|---|---|
| | | Example B3 | | | | Example B4 | | | | Example B5 | | | |
| Properties of each fraction | | Naphtha cracking. derived | Ethanol conversion-derived | Total | Difference | Naphtha cracking. derived | Ethanol conversion-derived | Total | Difference | Naphtha cracking. derived | Ethanol conversion-derived | Total | Difference |
| Amount of starting material used | ton/y | 177.7 | 279.3 | 457.0 | - | 88.8 | 252.9 | 341.7 | - | 266.5 | 145.6 | 412.1 | - |
| Temperature | °C | 41 | 15 | - | 26 | 41 | 15 | - | 26 | 41 | 40 | - | 1 |
| Pressure | MPaG | 0.99 | 1.90 | - | 0.91 | 0.99 | 1.90 | - | 0.91 | 0.99 | 1.90 | - | 0.91 |
| Amount introduced to soda washing tower | ton/y | 259.6 | | | | 220.6 | | | | 304.5 | | | |
| Product ratio in recycling fraction | % by mass | 0.7 | | | | 0.7 | | | | 11.3 | | | |
| Composition of fraction | | Naphtha cracking- | Ethanol conversio derived n-derived | Introduction fraction | Detour fraction | Naphtha cracking. derived | Ethanol conversion-derived | Introduction fraction | Detour fraction | Naphtha cracking. derived | Ethanol conversion-derived | Introduction fraction | Detour fraction |
| Hydrogen ton/y Methane | ton/y | 1.0 24.2 | 0.0 0.3 | 0.9 21.6 | 0.1 2.8 | 0.5 12.1 | 0.0 0.5 | 0.5 11.1 | 0.0 1.4 | 1.5 36.2 | 0.0 0.2 | 1.4 32.3 | 0.1 4.2 |
| Acetylene | ton/y | 0.9 | 0.0 | 0.0 | 0.9 | 0.5 | 0.0 | 0.0 | 0.5 | 1.4 | 0.0 | 0.0 | 1.4 |
| Ethylene | ton/y | 50.0 | 50.0 | 67.7 | 32.3 | 25.0 | 75.0 | 67.7 | 32.3 | 75.0 | 25.0 | 67.7 | 32.3 |
| Ethane | ton/y | 8.4 | 1.5 | 5.4 | 4.5 | 4.2 | 2.2 | 3.5 | 2.9 | 12.6 | 0.8 | 7.3 | 6.1 |
| Propylene | ton/y | 31.0 | 30.4 | 3.2 | 58.1 | 15.5 | 45.6 | 3.2 | 57.8 | 46.5 | 12.7 | 3.1 | 56.1 |
| Propane | ton/y | 0.7 | 2.2 | 0.1 | 2.8 | 0.3 | 3.3 | 0.2 | 3.5 | 1.0 | 0.9 | 0.1 | 1.8 |
| Butenes | ton/y | 21.1 | 4.5 | 0.0 | 13.5 | 10.6 | 6.8 | 0.0 | 9.2 | 31.7 | 6.0 | 0.0 | 19.9 |
| Butanes | ton/y | 0.6 | 1.9 | 0.0 | 1.3 | 0.3 | 2.8 | 0.0 | 1.7 | 0.9 | 2.9 | 0.0 | 2.0 |
| Pentenes | ton/y | 8.8 | 0.0 | 0.0 | 2.4 | 4.4 | 0.0 | 0.0 | 1.2 | 13.3 | 0.9 | 0.0 | 3.8 |
| Pentanes | ton/y | 0.1 | 0.0 | 0.0 | 0.0 | 0.0 | 0.0 | 0.0 | 0.0 | 0.1 | 1.6 | 0.0 | 0.4 |

(continued)

| Composition of fraction | | Naphtha cracking- | Ethanol conversio derived n-derived | Introducti on fraction | Detour fraction | Naphtha cracking. derived | Ethanol conversio n-derived | Introducti on fraction | Detour fraction | Naphtha cracking. derived | Ethanol conversio n-derived | Introducti on fraction | Detour fraction |
|---|---|---|---|---|---|---|---|---|---|---|---|---|---|
| Cracked gasoline | ton/y | 19.3 | 0.0 | 0.0 | 0.5 | 9.6 | 0.0 | 0.0 | 0.2 | 28.9 | 0.2 | 0.0 | 0.8 |
| Others | ton/y | 2.7 | 0.0 | 0.1 | 1.8 | 1.3 | 0.0 | 0.0 | 0.9 | 4.0 | 0.1 | 0.1 | 2.7 |
| Total | ton/y | 168.8 | 90.8 | 99.1 | 121.1 | 84.4 | 136.2 | 86.1 | 111.7 | 253.2 | 51.3 | 111.9 | 131.6 |
| $C_C$ (% by mass) | | 48.0 | | | | 48.0 | | | | 48.0 | | | |
| $C_E$ (% by mass) | | 88.5 | | | | 88.5 | | | | 73.5 | | | |
| ($C_E$-$C_C$) | | 40.5 | | | | 40.5 | | | | 25.6 | | | |
| $O_C$ (% by mass) | | 31.1 | | | | 31.1 | | | | 31.1 | | | |
| $O_E$ (% by mass) | | 0.6 | | | | 0.6 | | | | 0.6 | | | |
| $O'_C$ (% by mass) | | 31.0 | | | | 31.0 | | | | 31.0 | | | |
| $O'_E$ (% by mass) | | 0.4 | | | | 0.4 | | | | 0.7 | | | |
| C2-3 olefin treatment efficiency | | 71.6 | | | | 82.3 | | | | 63.2 | | | |
| C2-3 olefin treatment efficiency index | | 1.26 | | | | 1.45 | | | | 1.11 | | | |
| C3L volumetric efficiency | | 55.7 | | | | 70.6 | | | | 45.7 | | | |
| C3L volumetric efficiency index | | 1.42 | | | | 1.80 | | | | 1.17 | | | |

[Example B6]

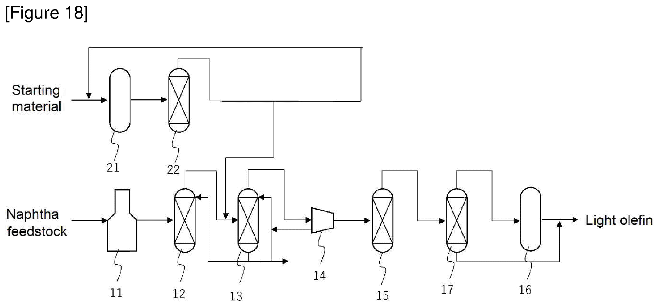

This Example is carried out with the light olefin production equipment shown in Figure 18.

(Ethanol conversion)

209.2 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain 138.6 ton/y of a cooled ethanol conversion fraction of 45°C and 0.15 MPaG. 46.1 ton/y of the cooled ethanol conversion fraction is used as a recycling fraction to be recycled in the starting material for introduction to the reactor 21 without steps such as separation and refining. In this respect, a product ratio in the recycling fraction is 43.8% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction.

(Combination of ethanol conversion with naphtha cracking)

474.1 ton/y of the first cooled naphtha cracking fraction is mixed with 92.5 ton/y of the cooled ethanol conversion fraction to prepare a combination fraction. The combination fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14, pressure-boosted to 0.99 MPaG, and introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 6. In this Example, the amount of ethylene produced is the total amount of ethylene contained in the introduction fraction and the detour fraction and is 100.0 ton/y.

[Example B7]

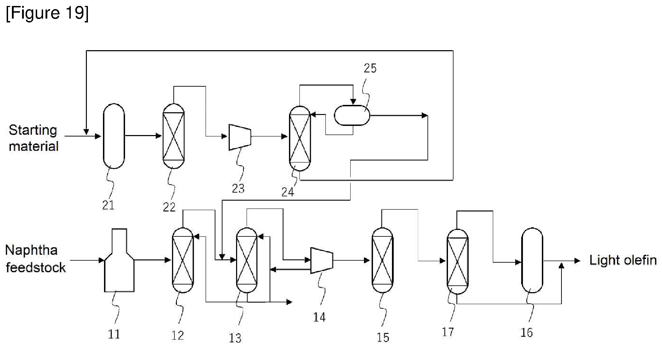

This Example is carried out with the light olefin production equipment shown in Figure 19.

(Ethanol conversion)

139.7 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a second distillation tower 24 to obtain a light ethanol conversion fraction from the tower top of the second distillation tower 24. The light ethanol conversion fraction is introduced to a condenser 25 where a portion of the fraction is liquefied and refluxed to the second distillation tower 24 to obtain a cooled light ethanol conversion fraction of 1.90 MPaG and 15°C as a gas. In this respect, 47.5 ton/y of a heavy ethanol conversion fraction obtained from the tower bottom of the second distillation tower 24 is used as a recycling fraction to be recycled in the starting material for introduction to the reactor 21 without additional steps such as refining. In this respect, a product ratio in the recycling fraction is 0.7% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with

a mixture of heavy oil and cracked gasoline and thereby cooled to 110°C to obtain a first cooled naphtha cracking fraction.

(Combination of ethanol conversion with naphtha cracking)

474.1 ton/y of the first cooled naphtha cracking fraction is mixed with 45.4 ton/y of the cooled light ethanol conversion fraction to prepare a combination fraction. The combination fraction is introduced to a second cooling tower 13 where the fraction is brought into contact with cooling water to obtain a second cooling fraction of 41°C. The second cooling fraction is introduced to a pressure booster 14 and pressure-boosted to 0.99 MPaG. The pressure-boosted second cooling fraction is introduced to a soda washing tower where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 6. In this Example, the amount of ethylene produced is the total amount of ethylene contained in the introduction fraction and the detour fraction and is 100.0 ton/y.

[Example B8]

This Example is carried out with the light olefin production equipment shown in Figure 20. This Example is carried out in the same manner as in Example B7 except that the location of connection is changed. In this Example, the cooled light ethanol conversion fraction of 15°C is combined with a second tower bottom reflux fraction of 68°C that includes a fraction obtained from the tower bottom of the second cooling tower 13 and is refluxed into the tower, to prepare a combination fraction. The amount of the second tower bottom reflux fraction is determined depending on the production balance of the compound of interest. In this Example, 52.6 ton/y of the second tower bottom reflux fraction is used.

Since the second tower bottom reflux fraction and the ethanol conversion fraction are combined so that the ethanol conversion fraction is introduced to the second cooling tower 13, the composition or flow rate of the resulting introduction fraction and detour fraction is not different between this Example and Example B7. The difference in temperature between the naphtha cracking fraction and the ethanol conversion fraction is as small as 53K, and change in volume associated with the cooling and liquefication of a high-temperature naphtha fraction can be suppressed.

[Example B9]

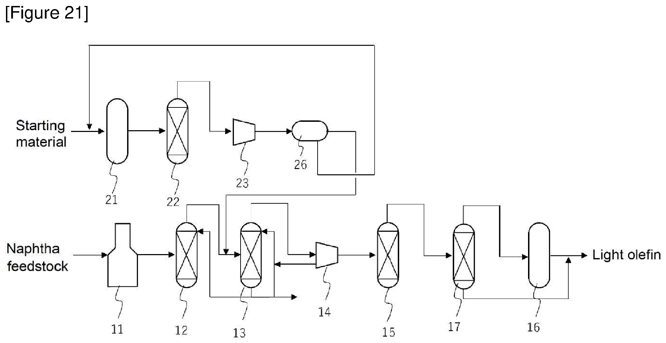

This Example is carried out with the light olefin production equipment shown in Figure 21.

(Ethanol conversion)

145.6 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a gas-liquid separation drum 26 to obtain a 51.3 ton/y of a light ethanol conversion fraction of 1.90 MPaG and 40°C as a gas. In this respect, 49.4 ton/y of a heavy ethanol conversion fraction obtained as a liquid in the gas-liquid separation drum 26 is used as a recycling fraction to be recycled in the starting material for introduction to the reactor 21 without additional steps such as refining. In this respect, a product ratio in the recycling fraction is 11.3% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction. The naphtha cracking fraction is introduced to a first cooling tower 12 where the fraction is brought into contact with a mixture of heavy oil and cracked gasoline and thereby cooled to 110°C to obtain a first cooled naphtha cracking fraction.

(Combination of ethanol conversion with naphtha cracking)

474.1 ton/y of the first cooled naphtha cracking fraction is mixed with 51.3 ton/y of the light ethanol conversion fraction to prepare a combination fraction. The combination fraction is introduced to a second cooling tower 13 where the fraction is brought into contact with cooling water to obtain a second cooled naphtha cracking fraction of 0.11 MPaG and

41°C. The second cooled naphtha cracking fraction is introduced to a pressure booster 14 and pressure-boosted to 0.99 MPaG. The combination fraction is introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 6. In this Example, the amount of ethylene produced is the total amount of ethylene contained in the introduction fraction and the detour fraction and is 100.0 ton/y.

[Table 6-1]

Table 6(1/2)

| | | Example B6 | | | | Example B7 | | | |
|---|---|---|---|---|---|---|---|---|---|
| Properties of each fraction | | Naphtha cracking-derived | Ethanol conversion-derived | Total | Difference | Naphtha cracking-derived | Ethanol conversion-derived | Total | Difference |
| Amount of starting material used | ton/y | 266.5 | 209.2 | 475.7 | - | 266.5 | 139.7 | 406.2 | |
| Temperature | °C | 110 | 45 | - | 65 | 110 | 15 | - | 95 |
| Pressure | MPaG | 0.13 | 0.15 | - | 0.02 | 0.13 | 1.90 | - | 1.77 |
| Amount introduced to soda washing tower | ton/y | 334.5 | | | | 298.6 | | | |
| Product ratio in recycling fraction | % by mass | 43.8 | | | | 0.7 | | | |
| Composition of fraction | | Naphtha cracking-derived | Ethanol conversion-derived | Introduction fraction | Detour fraction | Naphtha cracking-derived | Ethanol conversion-derived | Introduction fraction | Detour fraction |
| Hydrogen | ton/y | 1.5 | 0.0 | 1.4 | 0.1 | 1.5 | 0.0 | 1.4 | 0.1 |
| Methane | ton/y | 36.2 | 0.2 | 32.2 | 4.2 | 36.2 | 0.2 | 32.2 | 4.2 |
| Acetylene | ton/y | 1.4 | 0.0 | 0.0 | 1.4 | 1.4 | 0.0 | 0.0 | 1.4 |
| Ethylene | ton/y | 75.0 | 25.0 | 67.7 | 32.3 | 75.0 | 25.0 | 67.7 | 32.3 |
| Ethane | ton/y | 12.6 | 0.7 | 7.3 | 6.1 | 12.6 | 0.7 | 7.3 | 6.1 |
| Propylene | ton/y | 46.5 | 15.5 | 3.3 | 58.7 | 46.5 | 15.2 | 3.3 | 58.4 |
| Propane | ton/y | 1.0 | 1.1 | 0.1 | 2.1 | 1.0 | 1.1 | 0.1 | 2.0 |
| Butenes | ton/y | 31.7 | 13.0 | 0.0 | 23.6 | 31.7 | 2.3 | 0.0 | 17.9 |
| Butanes | ton/y | 0.9 | 3.7 | 0.0 | 2.5 | 0.9 | 0.9 | 0.0 | 1.0 |
| Pentenes | ton/y | 13.3 | 6.7 | 0.0 | 5.3 | 13.3 | 0.0 | 0.0 | 3.5 |
| Pentanes | ton/y | 0.1 | 11.2 | 0.0 | 2.8 | 0.1 | 0.0 | 0.0 | 0.0 |
| Cracked gasoline | ton/y | 113.8 | 12.8 | 0.0 | 0.9 | 113.8 | 0.0 | 0.0 | 0.7 |
| Others | ton/y | 140.1 | 2.5 | 0.0 | 2.8 | 140.1 | 0.0 | 0.1 | 2.7 |
| Total | ton/y | 474.1 | 92.5 | 111.9 | 142.7 | 474.1 | 45.4 | 112.0 | 130.4 |
| $C_C$ (% by mass) | | 25.6 | | | | 25.6 | | | |
| $C_E$ (% by mass) | | 43.8 | | | | 88.5 | | | |

(continued)

| Composition of fraction | Naphtha cracking-derived | Ethanol conversion-derived | Introduction fraction | Detour fraction | Naphtha cracking-derived | Ethanol conversion-derived | Introduction fraction | Detour fraction |
|---|---|---|---|---|---|---|---|---|
| ($C_E$-$C_C$) | 18.2 | | | | 62.9 | | | |
| $O_C$ (% by mass) | 31.1 | | | | 31.1 | | | |
| $O_E$ (% by mass) | 0.6 | | | | 0.6 | | | |
| $O'_C$ (% by mass) | 31.0 | | | | 31.0 | | | |
| $O'_E$ (% by mass) | 0.4 | | | | 0.4 | | | |
| C2-3 olefin treatment efficiency | 63.4 | | | | 63.3 | | | |
| C2-3 olefin treatment efficiency index | 1.12 | | | | 1.12 | | | |
| C3L volumetric efficiency | 46.0 | | | | 46.0 | | | |
| C3L volumetric efficiency index | 1.18 | | | | 1.18 | | | |

[Table 6-2]

Table 6(2/2)

| | | Example B8 | | | | Example B9 | | | |
|---|---|---|---|---|---|---|---|---|---|
| Properties of each fraction | | Naphtha cracking. derived | Ethanol conversion. derived | Total | Difference | Naphtha cracking. derived | Ethanol conversion. derived | Total | Difference |
| Amount of starting material used | ton/y | 266.5 | 139.7 | 406.2 | - | 266.5 | 145.6 | 412.1 | |
| Temperature | °C | 68 | 15 | - | 53 | 110 | 40 | - | 70 |
| Pressure | MPaG | 0.21 | 1.90 | - | 1.69 | 0.13 | 1.90 | - | 1.77 |
| Amount introduced to soda washing tower | ton/y | 298.6 | | | | 304.3 | | | |
| Product ratio in recycling fraction | % by mass | 0.7 | | | | 11.3 | | | |
| Composition of fraction | | Naphtha cracking-derived | Ethanol conversion-derived | Introduction fraction | Detour fraction | Naphtha cracking-derived | Ethanol conversion-derived | Introduction fraction | Detour fraction |
| Hydrogen | ton/y | 0.0 | 0.0 | 1.4 | 0.1 | 1.5 | 0.0 | 1.4 | 0.1 |
| Methane | ton/y | 0.0 | 0.2 | 32.2 | 4.2 | 36.2 | 0.2 | 32.3 | 4.2 |
| Acetylene | ton/y | 0.0 | 0.0 | 0.0 | 1.4 | 1.4 | 0.0 | 0.0 | 1.4 |
| Ethylene | ton/y | 0.2 | 25.0 | 67.7 | 32.3 | 75.0 | 25.0 | 67.7 | 32.3 |
| Ethane | ton/y | 0.0 | 0.7 | 7.3 | 6.1 | 12.6 | 0.8 | 7.3 | 6.1 |
| Propylene | ton/y | 0.4 | 15.2 | 3.3 | 58.4 | 46.5 | 12.7 | 3.1 | 56.1 |
| Propane | ton/y | 0.0 | 1.1 | 0.1 | 2.0 | 1.0 | 0.9 | 0.1 | 1.8 |
| Butenes | ton/y | 1.0 | 2.3 | 0.0 | 17.9 | 31.7 | 6.0 | 0.0 | 19.9 |
| Butanes | ton/y | 0.0 | 0.9 | 0.0 | 1.0 | 0.9 | 2.9 | 0.0 | 2.0 |
| Pentenes | ton/y | 1.0 | 0.0 | 0.0 | 3.5 | 13.3 | 0.9 | 0.0 | 3.8 |
| Pentanes | ton/y | 0.0 | 0.0 | 0.0 | 0.0 | 0.1 | 1.6 | 0.0 | 0.4 |
| Cracked gasoline | ton/y | 36.8 | 0.0 | 0.0 | 0.7 | 113.8 | 0.2 | 0.0 | 0.7 |
| Others | ton/y | 13.1 | 0.0 | 0.1 | 2.7 | 140.1 | 0.1 | 0.1 | 2.7 |
| Total | ton/y | 52.6 | 45.4 | 112.0 | 130.4 | 474.1 | 51.3 | 111.9 | 131.5 |
| $C_C$ (% by mass) | | 1.1 | | | | 25.6 | | | |
| $C_E$ (% by mass) | | 88.5 | | | | 73.5 | | | |

(continued)

| Composition of fraction | Naphtha cracking-derived | Ethanol conversion-derived | Introduction fraction | Detour fraction | Naphtha cracking-derived | Ethanol conversion-derived | Introduction fraction | Detour fraction |
|---|---|---|---|---|---|---|---|---|
| ($C_E$-$C_C$) | 87.4 | | | | 47.9 | | | |
| Oc (% by mass) | 31.1 | | | | 31.1 | | | |
| $O_E$ (% by mass) | 0.6 | | | | 0.6 | | | |
| $O'_C$ (% by mass) | 3.9 | | | | 31.0 | | | |
| $O'_E$ (% by mass) | 0.4 | | | | 0.7 | | | |
| C2-3 olefin treatment efficiency | 63.3 | | | | 63.2 | | | |
| C2-3 olefin treatment efficiency index | 1.12 | | | | 1.11 | | | |
| C3L volumetric efficiency | 46.0 | | | | 45.7 | | | |
| C3L volumetric efficiency index | 1.18 | | | | 1.17 | | | |

[Example B10]

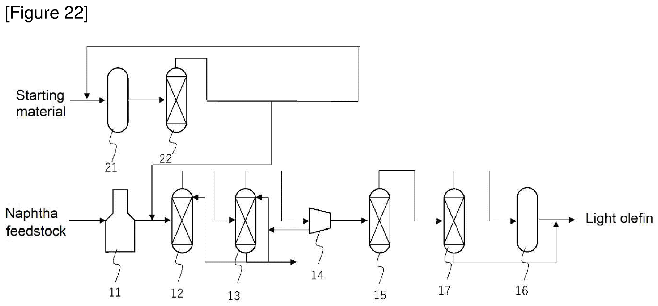

This Example is carried out with the light olefin production equipment shown in Figure 22.

(Ethanol conversion)

209.2 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain 138.6 ton/y of a cooled ethanol conversion fraction of 45°C and 0.15 MPaG. 46.1 ton/y of the cooled ethanol conversion fraction is used as a recycling fraction to be recycled in the starting material for introduction to the reactor 21 without steps such as separation and refining. In this respect, a product ratio in the recycling fraction is 43.8% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction.

(Combination of ethanol conversion with naphtha cracking)

417.2 ton/y of the naphtha cracking fraction is mixed with 92.5 ton/y of the cooled ethanol conversion fraction to prepare a combination fraction. The combination fraction is introduced to a first cooling tower 12 where the fraction is brought into contact with a mixture of heavy oil and cracked gasoline and thereby cooled to 110°C to obtain a first cooled naphtha cracking fraction. The first cooled naphtha cracking fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14, pressure-boosted to 0.99 MPaG, and introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 7. In this Example, the amount of ethylene produced is the total amount of ethylene contained in the introduction fraction and the detour fraction and is 100.0 ton/y.

[Example B11]

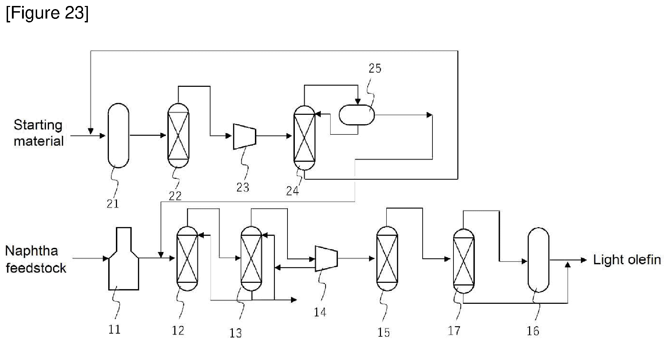

This Example is carried out with the light olefin production equipment shown in Figure 23.

(Ethanol conversion)

139.7 ton/y of a starting material gas is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a second distillation tower 24 to obtain a light ethanol conversion fraction from the tower top of the second distillation tower 24. The light ethanol conversion fraction is introduced to a condenser 25 where a portion of the fraction is liquefied and refluxed to the second distillation tower 24 to obtain a cooled light ethanol conversion fraction of 1.90 MPaG and 15°C as a gas. In this respect, 47.5 ton/y of a heavy ethanol conversion fraction obtained from the tower bottom of the second distillation tower 24 is used as a recycling fraction to be recycled in the starting material for introduction to the reactor 21 without additional steps such as refining. In this respect, a product ratio in the recycling fraction is 0.7% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking

fraction.

(Combination of ethanol conversion with naphtha cracking)

417.2 ton/y of the naphtha cracking fraction of 360°C is mixed with 45.4 ton/y of the cooled light ethanol conversion fraction to prepare a combination fraction. The combination fraction is introduced to a first cooling tower 12 where the fraction is brought into contact with a mixture of heavy oil and cracked gasoline and thereby cooled to 110°C to obtain a first cooling fraction. The first cooling fraction is introduced to a second cooling tower 13 where the fraction is brought into contact with cooling water to obtain a second cooling fraction of 41°C. The second cooling fraction is introduced to a pressure booster 14 and pressure-boosted to 0.99 MPaG. The pressure-boosted second cooling fraction is introduced to a soda washing tower 15 where the fraction is brought into contact with an aqueous sodium hydroxide solution to obtain a washing fraction. The washing fraction is further pressure-boosted and thereby introduced as an introduction fraction to a cryogenic separation step. In this respect, the amount of ethylene in the combination fraction is 100.0 ton/y. The flow rate and composition of the combination fraction are shown in Table 7.

[Example B12]

Example B12 is carried out by the process flow shown in Figure 24. Example B12 is carried out in the same manner as in Example B11 except that the location of connection is changed. In Example B12, the cooled light ethanol conversion fraction of 15°C is combined with a first tower bottom reflux fraction of 80°C that includes a fraction obtained from the tower bottom of the second cooling tower 13 and is refluxed into the first cooling tower 12, to prepare a combination fraction.

Since the first tower bottom reflux fraction and the ethanol conversion fraction are combined so that the ethanol conversion fraction is introduced to the first cooling tower 12, the composition or flow rate of the resulting introduction fraction is not different between Example B11 and Example B12. The difference in temperature between the naphtha cracking fraction and the ethanol conversion fraction is as small as 65K, and change in volume associated with the cooling and liquefication of a high-temperature naphtha fraction can be suppressed.

[Example B13]

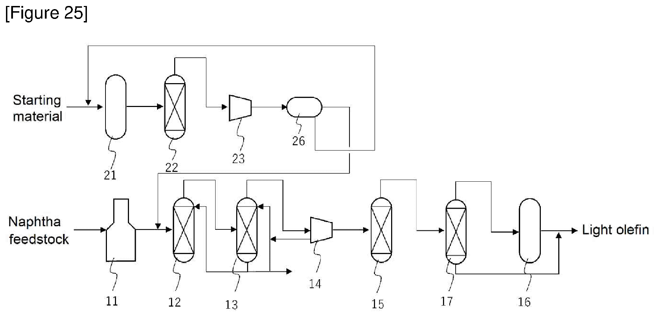

This Example is carried out with the light olefin production equipment shown in Figure 25.

(Ethanol conversion)

145.6 ton/y of a starting material is heated, supplied at WHSV = 3.8 to a reactor 21 packed with a zeolite-containing catalyst, and subjected to ethanol conversion reaction at a reaction temperature of 540°C to obtain an ethanol conversion fraction. The ethanol conversion fraction is introduced to a third cooling tower 22 where the fraction is brought into contact with cooling water to obtain a cooled ethanol conversion fraction of 45°C. The cooled ethanol conversion fraction is pressure-boosted with a pressure booster 23 and introduced to a gas-liquid separation drum 26 to obtain a 51.3 ton/y of a light ethanol conversion fraction of 1.90 MPaG and 40°C as a gas. In this respect, 49.4 ton/y of a heavy ethanol conversion fraction obtained as a liquid in the gas-liquid separation drum 26 is used as a recycling fraction to be recycled in the starting material for introduction to the reactor 21 without additional steps such as refining. In this respect, a product ratio in the recycling fraction is 11.3% by mass.

(Naphtha cracking)

266.5 ton/y of a naphtha feedstock containing heavy naphtha and diluted steam with a mass ratio 0.5 to naphtha is introduced to a cracking furnace 11 under cracking conditions of 0.17 MPaG and 825°C to obtain a naphtha cracking fraction.

(Combination of ethanol conversion with naphtha cracking)

417.2 ton/y of the naphtha cracking fraction is mixed with 51.3 ton/y of the light ethanol conversion fraction to prepare a combination fraction. The combination fraction is introduced to a first cooling tower 12 where the fraction is brought into contact with a mixture of heavy oil and cracked gasoline and thereby cooled to 110°C to obtain a first cooled naphtha cracking fraction. This fraction is introduced to a second cooling tower 13 where the fraction is brought into contact with cooling water to obtain a second cooled naphtha cracking fraction of 0.11 MPaG and 41°C. The second cooled naphtha cracking fraction is introduced to a pressure booster 14 and pressure-boosted to 0.99 MPaG. The pressure-

boosted second cooled naphtha cracking fraction is introduced to a soda washing tower 15 where the fraction is brought into contact with an aqueous sodium hydroxide solution to obtain a washing fraction. The washing fraction is pressure-boosted to 1.82 MPaG and then introduced to a first distillation tower 17 to obtain an introduction fraction to be introduced to a cryogenic separation step, at -18°C from the tower top. Also, a detour fraction to be introduced to downstream equipment by detouring the cryogenic separation step is obtained at 16°C from the tower bottom. The composition of the introduction fraction and the detour fraction is shown in Table 7. In this Example, the amount of ethylene produced is the total amount of ethylene contained in the introduction fraction and the detour fraction and is 100.0 ton/y.

[Table 7]

Table 7

| | | Example B10 | | | | Example B11 | | | | Example B13 | | | |
|---|---|---|---|---|---|---|---|---|---|---|---|---|---|
| Properties of each fraction | | Naphtha cracking. derived | Ethanol conversion-derived | Total | Difference | Naphtha cracking. derived | Ethanol conversion-derived | Total | Difference | Naphtha cracking. derived | Ethanol conversion-derived | Total | Difference |
| Amount of starting material used | ton/y | 266.5 | 209.2 | 475.7 | - | 266.5 | 139.7 | 406.2 | - | 266.5 | 145.6 | 412.1 | - |
| Temperature | °C | 360 | 45 | - | 315 | 360 | 15 | - | 345 | 360 | 40 | - | 320 |
| Pressure | MPaG | 0.15 | 0.15 | - | trace | 0.15 | 1.90 | - | 1.75 | 0.15 | 1.90 | - | 1.75 |
| Amount introduced to soda washing tower | ton/y | 335.6 | | | | 298.6 | | | | 304.3 | | | |
| Product ratio in recycling fraction | % by mass | 43.8 | | | | 0.7 | | | | 11.3 | | | |
| Composition of fraction | | Naphtha cracking. derived | Ethanol conversion-derived | Introduction fraction | Detour fraction | Naphtha cracking. derived | Ethanol conversion-derived | Introduction fraction | Detour fraction | Naphtha cracking derived | Ethanol conversion-derived | Introduction fraction | Detour fraction |
| Hydrogen ton/y | | 1.5 | | 1.4 | 0.1 | 1.5 | | 1.4 | 0.1 | 1.5 | | 1.4 | 0.1 |
| Methane | ton/y | 36.2 | 0.2 | 32.2 | 4.2 | 36.2 | 0.2 | 32.2 | 4.2 | 36.2 | 0.2 | 32.3 | 4.2 |
| Acetylene | ton/y | 1.4 | 0.0 | 0.0 | 1.4 | 1.4 | 0.0 | 0.0 | 1.4 | 1.4 | 0.0 | 0.0 | 1.4 |
| Ethylene | ton/y | 75.0 | 25.0 | 67.7 | 32.3 | 75.0 | 25.0 | 67.7 | 32.3 | 75.0 | 25.0 | 67.7 | 32.3 |
| Ethane | ton/y | 12.6 | 0.7 | 7.3 | 6.1 | 12.6 | 0.7 | 7.3 | 6.1 | 12.6 | 0.8 | 7.3 | 6.1 |
| Propylene | ton/y | 46.5 | 15.5 | 3.3 | 58.7 | 46.5 | 15.2 | 3.3 | 58.4 | 46.5 | 12.7 | 3.1 | 56.1 |
| Propane | ton/y | 1.0 | 1.1 | 0.1 | 2.1 | 1.0 | 1.1 | 0.1 | 2.0 | 1.0 | 0.9 | 0.1 | 1.8 |
| Butenes | ton/y | 31.7 | 13.0 | 0.0 | 23.6 | 31.7 | 2.3 | 0.0 | 17.9 | 31.7 | 6.0 | 0.0 | 19.9 |
| Butanes | ton/y | 0.9 | 3.7 | 0.0 | 2.5 | 0.9 | 0.9 | 0.0 | 1.0 | 0.9 | 2.9 | 0.0 | 2.0 |
| Pentenes | ton/y | 13.3 | 6.7 | 0.0 | 5.3 | 13.3 | 0.0 | 0.0 | 3.5 | 13.3 | 0.9 | 0.0 | 3.8 |
| Pentanes | ton/y | 0.1 | 11.2 | 0.0 | 2.8 | 0.1 | 0.0 | 0.0 | 0.0 | 0.1 | 1.6 | 0.0 | 0.4 |
| Cracked gasoline | ton/y | 50.9 | 12.8 | 0.0 | 0.9 | 26.4 | 0.0 | 0.0 | 0.0 | 26.4 | 0.2 | 0.0 | 0.7 |
| Others | ton/y | 146.1 | 2.5 | 0.0 | 2.8 | 146.1 | 0.0 | 0.1 | 2.7 | 146.1 | 0.1 | 0.1 | 2.7 |
| Total | ton/y | 417.2 | 92.5 | 111.9 | 142.7 | 417.2 | 45.4 | 112.0 | 130.4 | 417.2 | 51.3 | 111.9 | 131.5 |

(continued)

| Composition of fraction | Naphtha cracking. derived | Ethanol conversion-derived | Introduction fraction | Detour fraction | Naphtha cracking. derived | Ethanol conversion-derived | Introduction fraction | Detour fraction | Naphtha cracking derived | Ethanol conversion-derived | Introduction fraction | Detour fraction |
|---|---|---|---|---|---|---|---|---|---|---|---|---|
| $C_C$ (% by mass) | 29.1 | | | | 29.1 | | | | 29.1 | | | |
| $C_E$ (% by mass) | 43.8 | | | | 88.5 | | | | 73.5 | | | |
| ($C_E$-$C_C$) | 14.7 | | | | 59.4 | | | | 44.4 | | | |
| $O_C$ (% by mass) | 31.1 | | | | 31.1 | | | | 31.1 | | | |
| $O_E$ (% by mass) | 0.6 | | | | 0.6 | | | | 0.6 | | | |
| $O'_C$ (% by mass) | 31.0 | | | | 31.0 | | | | 31.0 | | | |
| $O'_E$ (% by mass) | 0.4 | | | | 0.4 | | | | 0.7 | | | |
| C2-3 olefin treatment efficiency | 63.4 | | | | 63.3 | | | | 63.2 | | | |
| C2-3 olefin treatment efficiency index | 1.12 | | | | 1.12 | | | | 1.11 | | | |
| C3L volumetric efficiency | 46.0 | | | | 46.0 | | | | 45.7 | | | |
| C3L volumetric efficiency index | 1.18 | | | | 1.18 | | | | 1.17 | | | |

As is evident from the results of Examples and Comparative Examples, the method for producing light olefin according to the present embodiment can decrease the amount of off-gases such as hydrogen and methane and can reduce treatment energy in cryogenic separation.

In Examples C1 to C5 and Comparative Examples C1 to C4, the influence of the difference in composition between an ethanol conversion fraction obtained in an ethanol conversion step and a naphtha cracking fraction obtained in a naphtha cracking step on refining equipment will be discussed.

(Production rate of each compound)

In Examples C1 to C5 and Comparative Examples C1 to C4, the amount of each product produced in a naphtha cracking furnace is decreased, and the decrement is substituted with an ethanol conversion fraction so as to compensate therefor. The amount of each product produced after substitution with the ethanol conversion fraction is divided by the amount of each product produced in the naphtha cracking furnace before decreased production, and the resulting value is referred to as a production rate. In this context, the amount of each product produced is the total sum of the amount of each product in the ethanol conversion fraction and the amount of each product in the naphtha cracking fraction.

[Examples C1 to C4 and Comparative Examples C1 to C4]

In Examples C1 to C4 and Comparative Examples C1 to C4, 100 ton/y of ethylene produced in a naphtha cracking furnace is decreased to 75 ton/y, and an ethanol conversion fraction is connected instead. In these Examples and Comparative Examples, an ethanol conversion step and a cracking step are carried out under the conditions shown in Table 8, and light olefin is then refined through a combination step and a cryogenic separation step. In this respect, the purity of a product is decreased when a production rate exceeds 100%. Therefore, the amount of the ethanol conversion fraction produced is adjusted such that the production rates of ethylene, propylene, and hydrocarbon having 4 or more carbon atoms do not exceed 100%.

As is evident from the comparison of Examples with Comparative Examples, the production rates of ethylene, propylene, and hydrocarbon having 4 or more carbon atoms do not fall below 90% when the ethanol conversion fraction and the naphtha cracking fraction are used in combination so as to satisfy the expression (ex) and the expression ($\beta$-1). The production rate of each product that falls below 90% is not preferred because refining efficiency is drastically reduced due to a problem arising in refining equipment, for example, the occurrence of surging in a compressor.

[Example C5]

Example C5 is carried out in the same manner as in Example C4 except that the amount of ethylene produced in a naphtha cracking furnace is decreased to 50 ton/y. A higher rate of substitution of naphtha cracking with ethanol conversion more largely influences the production rate of a product.

[Table 8]

Table 8

| | | | Example C1 | Example C2 | Example C3 | Example C4 | Example C5 | Comparativ e Example C1 | Comparativ e Example C2 | Comparati ve Example C3 | Comparati ve Example C4 |
|---|---|---|---|---|---|---|---|---|---|---|---|
| Ethanol conversion step | **Catalyst** | | P-ZSM-5 | P-ZSM-5 | ZSM-5 | ZSM-5 | ZSM-5 | P-ZSM-5 | P-ZSM-5 | P-ZSM-5 | P-ZSM-5 |
| | **Temperature** | °C | 560°C | 460°C | 540°C | 540°C | 540°C | 500°C | 560°C | 485°C | 485°C |
| | **WHSV** | - | 1.84 | 2.28 | 3.80 | 3.80 | 3.80 | 3.43 | 1.84 | 7.02 | 7.02 |
| | **$P/E_E$** | - | 0.82 | 0.41 | 0.63 | 0.63 | 0.63 | 0.39 | 0.82 | 0.24 | 0.24 |
| | **$C_{E_AP}$** | % by mass | 41.1 | 30.8 | 29.7 | 29.7 | 29.7 | 28.6 | 41.1 | 54.0 | 54.0 |
| | **Amount of ethylene produced** | ton/y | 17.0 | 25.0 | 24.8 | 22.1 | 44.2 | 23.4 | 14.3 | 25.0 | 25.0 |
| | **Amount of propylene produced** | ton/y | 13.9 | 10.3 | 15.6 | 13.9 | 27.8 | 9.1 | 11.8 | 6.0 | 6.0 |
| Cracking step | **Starting material** | | Heavy naphtha | Light naphtha | Light naphtha | Heavy naphtha | Heavy naphtha | Light naphtha | Heavy naphtha | Heavy naphtha | Heavy naphtha |
| | **Temperature** | °C | 840°C | 830°C | 830°C | 830°C | 830°C | 830°C | 850°C | 825°C | 840°C |
| | **$P/E_c$** | - | 0.56 | 0.65 | 0.65 | 0.56 | 0.56 | 0.65 | 0.47 | 0.67 | 0.56 |
| | **$C_{C_AP}$** | % by mass | 27.2 | 30.6 | 30.6 | 27.2 | 27.2 | 30.6 | 26.1 | 28.7 | 27.2 |
| | **Amount of ethylene produced** | ton/y | 75.0 | 75.0 | 75.0 | 75.0 | 50.0 | 75.0 | 75.0 | 75.0 | 75.0 |
| | **Amount of propylene produced** | ton/y | 41.7 | 48.9 | 48.9 | 41.7 | 27.8 | 48.9 | 35.3 | 46.5 | 41.70 |
| **$P/E_E$-$P/E_C$** | | - | 0.26 | -0.24 | -0.02 | 0.07 | 0.07 | -0.26 | 0.35 | -0.43 | -0.32 |
| **$CE_{AP}$-$C_{C_AP}$** | | % by mass | 13.9 | 0.2 | -0.9 | 2.5 | 2.5 | -2.0 | 15.0 | 25.3 | 26.8 |
| **Production rate of ethylene** | | ton/y | 92.0% | 100.0% | 99.8% | 97.1% | 94.2% | 98.4% | 89.3% | 100.0% | 100.0% |
| **Production rate of propylene** | | ton/y | 100.0% | 90.7% | 98.9% | 100.0% | 100.0% | 89.0% | 100.0% | 78.1% | 85.8% |
| **Production rate of hydrocarbon having 4 or more carbon atoms** | | ton/y | 91.5% | 99.8% | 100.0% | 97.9% | 97.9% | 100.0% | 90.9% | 88.3% | 87.6% |

**Reference Signs List**

11 ... cracking furnace, 12 ... cooling tower, 13 ... second cooling tower, 14 ... first compressor, 15 ... soda washing tower, 16 ... cryogenic separation equipment, 21 ... reactor, and 22 ... third cooling tower.

## Claims

1. A method for producing light olefin, comprising:

   a cracking step of introducing a naphtha feedstock to a cracking furnace to obtain a naphtha cracking fraction;
   an ethanol conversion step of introducing a starting material containing ethanol to a reactor where the starting material is brought into contact with a catalyst to obtain an ethanol conversion fraction containing ethylene and propylene;
   a combination step of combining at least a portion of the naphtha cracking fraction or a fraction derived therefrom with at least a portion of the ethanol conversion fraction or a fraction derived therefrom to obtain a combination fraction; and
   a cryogenic separation step of introducing the combination fraction or a fraction derived therefrom to a cryogenic separation equipment to separate ethylene and propylene, wherein
   a relationship between a content $C_E$ of ethylene and propylene in the ethanol conversion fraction or a fraction derived therefrom and a content $C_C$ of ethylene and propylene in the naphtha cracking fraction or a fraction derived therefrom in the combination step satisfies the expression (A):

$$C_E > C_C \ ...\ (A).$$

2. The method for producing light olefin according to claim 1, wherein a difference between the content $C_E$ and the content $C_C$ ($C_E$ - $C_C$) in the combination step is 5% by mass or more.

3. The method for producing light olefin according to claim 1, wherein a relationship between a propylene/ethylene mass ratio $P/E_E$ in the ethanol conversion fraction and a propylene/ethylene mass ratio P/Ec in the naphtha cracking fraction satisfies the expression ($\alpha$):

$$-0.25 < (P/E_E - P/E_C) < 0.30\ ...\ (\alpha).$$

4. The method for producing light olefin according to claim 1, wherein a relationship between a content $C_{E_AP}$ of ethylene and propylene in the ethanol conversion fraction and a content $C_{C_AP}$ of ethylene and propylene in the naphtha cracking fraction satisfies the expression ($\beta$-1):

$$|C_{E_AP} - C_{C_AP}| < 20\%\text{ by mass}\ ...\ (\beta\text{-}1).$$

5. A method for producing light olefin, comprising:

   a cracking step of introducing a naphtha feedstock to a cracking furnace to obtain a naphtha cracking fraction;
   an ethanol conversion step of introducing a starting material containing ethanol to a reactor where the starting material is brought into contact with a catalyst to obtain an ethanol conversion fraction containing ethylene and propylene;
   a combination step of combining at least a portion of the naphtha cracking fraction or a fraction derived therefrom with at least a portion of the ethanol conversion fraction or a fraction derived therefrom to obtain a combination fraction; and
   a cryogenic separation step of introducing the combination fraction or a fraction derived therefrom to a cryogenic separation equipment to separate ethylene and propylene, wherein
   a relationship between a propylene/ethylene mass ratio $P/E_E$ in the ethanol conversion fraction and a propylene/ethylene mass ratio P/Ec in the naphtha cracking fraction satisfies the expression ($\alpha$):

$$-0.25 < (P/E_E - P/E_C) < 0.30\ ...\ (\alpha),\ \text{and}$$

a relationship between a content $C_{E_AP}$ of ethylene and propylene in the ethanol conversion fraction and a content $C_{C_AP}$ of ethylene and propylene in the naphtha cracking fraction satisfies the expression (β-1):

$$|C_{E_AP} - C_{C_AP}| < 20\% \text{ by mass ... } (\beta\text{-1}).$$

6. The method for producing light olefin according to claim 5, wherein the relationship between the content $C_{E_AP}$ of ethylene and propylene in the ethanol conversion fraction and the content $C_{C_AP}$ of ethylene and propylene in the naphtha cracking fraction satisfies the expression (β-2):

$$0 \leq (C_{E_AP} - C_{C_AP}) < 20\% \text{ by mass ... } (\beta\text{-2}).$$

7. The method for producing light olefin according to claim 1 or 5, comprising a first cooling step of introducing the naphtha cracking fraction or the combination fraction to a first cooling tower to obtain a first cooling fraction mainly containing olefin having 6 or less carbon atoms.

8. The method for producing light olefin according to claim 1 or 5, comprising a second cooling step of introducing the naphtha cracking fraction or the combination fraction or a fraction derived therefrom to a second cooling tower to obtain a second cooling fraction mainly containing olefin having 4 or less carbon atoms.

9. The method for producing light olefin according to claim 1 or 5, comprising a third cooling step of introducing the ethanol conversion fraction to a third cooling tower to obtain a cooled ethanol conversion fraction mainly containing olefin having 6 or less carbon atoms.

10. The method for producing light olefin according to claim 9, comprising a compression and separation step of pressure-boosting the cooled ethanol conversion fraction using a compressor to obtain a light ethanol conversion fraction mainly containing olefin having 3 or less carbon atoms as a gas component and to obtain a heavy ethanol conversion fraction mainly containing olefin having 4 or more carbon atoms as a liquid component.

11. The method for producing light olefin according to claim 10, comprising a recycling step of introducing at least a portion of the heavy ethanol conversion fraction as a portion of the starting material to the reactor.

12. The method for producing light olefin according to claim 1 or 5, comprising, after the combination step, a washing step of introducing the combination fraction or a fraction derived therefrom to a soda washing tower to obtain a washing fraction.

13. The method for producing light olefin according to claim 1 or 5, comprising, after the second cooling step and before the cryogenic separation step, a first compression step of pressure-boosting the fraction.

14. The method for producing light olefin according to claim 1 or 5, wherein a relationship between a ratio $O_E$ of an amount of hydrogen and methane to a total amount of ethylene and propylene in the ethanol conversion fraction and a ratio $O_C$ of an amount of hydrogen and methane to a total amount of ethylene and propylene in the naphtha cracking fraction satisfies the expression (1):

$$O_E < O_C \text{ ... } (1).$$

15. The method for producing light olefin according to claim 1 or 5, wherein the method satisfies any of the following (1) to (5):

(1) comprising:

a first cooling step of introducing the naphtha cracking fraction to a first cooling tower to obtain a first cooling fraction mainly containing olefin having 6 or less carbon atoms; and
a second cooling step of introducing the first cooling fraction to a second cooling tower to obtain a second cooling fraction mainly containing olefin having 4 or less carbon atoms, wherein
in the combination step, at least a portion of the second cooling fraction is combined with at least a portion of the ethanol conversion fraction or a fraction derived therefrom,

(2) comprising:

a first cooling step of introducing the naphtha cracking fraction to a first cooling tower to obtain a first cooling fraction mainly containing olefin having 6 or less carbon atoms; and
a second cooling step of introducing the combination fraction or a fraction derived therefrom to a second cooling tower to obtain a second cooling fraction mainly containing olefin having 4 or less carbon atoms, wherein
in the combination step, at least a portion of the first cooling fraction is combined with at least a portion of the ethanol conversion fraction or a fraction derived therefrom,

(3) comprising:

a first cooling step of introducing the naphtha cracking fraction to a first cooling tower to obtain a first cooling fraction mainly containing olefin having 6 or less carbon atoms;
a second cooling step of introducing the first cooling fraction to a second cooling tower to obtain a second cooling fraction mainly containing olefin having 4 or less carbon atoms;
a circulation step of introducing at least a portion of a second heavy fraction mainly containing hydrocarbon having 5 or more carbon atoms in the second cooling step to the second cooling tower; and
a first introduction step of introducing the combination fraction to the second cooling tower, wherein
in the combination step, at least a portion of the second heavy fraction is combined with at least a portion of the ethanol conversion fraction or a fraction derived therefrom,

(4) comprising:

a first cooling step of introducing the combination fraction or a fraction derived therefrom to a first cooling tower to obtain a first cooling fraction mainly containing olefin having 6 or less carbon atoms; and
a second cooling step of introducing the first cooling fraction to a second cooling tower to obtain a second cooling fraction mainly containing olefin having 4 or less carbon atoms, wherein
in the combination step, at least a portion of the naphtha cracking fraction is combined with at least a portion of the ethanol conversion fraction or a fraction derived therefrom, and

(5) comprising:

a first cooling step of introducing the naphtha cracking fraction to a first cooling tower to obtain a first cooling fraction mainly containing olefin having 6 or less carbon atoms;
a second cooling step of introducing the first cooling fraction to a second cooling tower to obtain a second cooling fraction mainly containing olefin having 4 or less carbon atoms;
a circulation step of introducing at least a portion of a second heavy fraction mainly containing hydrocarbon having 5 or more carbon atoms in the second cooling step to the first cooling tower; and
a second introduction step of introducing the combination fraction to the first cooling tower, wherein
in the combination step, at least a portion of the second heavy fraction is combined with at least a portion of the ethanol conversion fraction or a fraction derived therefrom.

[Figure 1]

E
EtOH
Ethanol conversion process
N
Naphtha cracking process
Naphtha
Naphtha cracking
Naphtha cracking fraction refining process
Cryogenic separation
N1
N2
N3

[Figure 2]

Starting
material
Naphtha
feedstock
Light olefin
21
22
11
12
13
14
15
16

[Figure 3]

211
214
212
216
219a
217
213
218
d
219b
215

[Figure 4]

Starting material
Naphtha feedstock
Light olefin
11
12
13
14
15
16
21
22
23
24
25

[Figure 5]

Starting material
21
22
23
26
Naphtha feedstock
11
12
13
14
15
16
Light olefin

[Figure 6]

Starting
material
21
22
Naphtha
feedstock
11
12
13
14
15
16
Light olefin

[Figure 7]

Starting material
21
22
23
24
25
Naphtha feedstock
11
12
13
14
15
16
Light olefin

[Figure 8]

25
Starting
material
21
22
23
24
Naphtha
feedstock
Light olefin
11
12
13
14
15
16

[Figure 9]

Starting material
26
23
21
22
Naphtha feedstock
Light olefin
14
11
12
13
15
16

[Figure 10]

Starting material
21
22
Naphtha feedstock
11
12
13
14
15
16
Light olefin

[Figure 11]

25
Starting material
21
22
23
24
Naphtha feedstock
Light olefin
11
12
13
14
15
16

[Figure 12]

25
Starting material
21
22
23
24
Naphtha feedstock
Light olefin
11
12
13
14
15
16

[Figure 13]

Starting
material
21
22
23
26
Naphtha
feedstock
11
12
13
14
15
16
Light olefin

[Figure 14]

Naphtha
feedstock
Light olefin
11
12
13
14
15
16

[Figure 15]

Starting material
21
22
Naphtha feedstock
11
12
13
14
15
17
16
Light olefin

[Figure 16]

25
Starting material
21
22
23
24
Naphtha feedstock
Light olefin
11
12
13
14
15
17
16

[Figure 17]

Starting material
21
22
23
26
Naphtha feedstock
11
12
13
14
15
17
16
Light olefin

[Figure 18]

Starting material
21
22
Naphtha feedstock
11
12
13
14
15
17
16
Light olefin

[Figure 19]

25
Starting
material
23
24
21
22
Naphtha
feedstock
Light olefin
14
11
12
13
15
17
16

[Figure 20]

25
Starting material
21
22
23
24
Naphtha feedstock
Light olefin
11
12
13
14
15
17
16

[Figure 21]

Starting material
21
22
23
26
Naphtha feedstock
11
12
13
14
15
17
16
Light olefin

[Figure 22]

Starting material
21
22
Naphtha feedstock
11
12
13
14
15
17
16
Light olefin

[Figure 23]

25
Starting material
21
22
23
24
Naphtha feedstock
Light olefin
11
12
13
14
15
17
16

[Figure 24]

25
Starting material
21
22
23
24
Naphtha feedstock
Light olefin
11
12
13
14
15
17
16

[Figure 25]

Starting material
21
22
23
26
Naphtha feedstock
11
12
13
14
15
17
16
Light olefin

[Figure 26]

Naphtha feedstock
Light olefin
11
12
13
14
15
17
16

## INTERNATIONAL SEARCH REPORT

International application No.
**PCT/JP2024/040521**

**A. CLASSIFICATION OF SUBJECT MATTER**

***C07C 7/09***(2006.01)i; ***B01J 29/40***(2006.01)i; ***C07B 61/00***(2006.01)i; ***C07C 1/24***(2006.01)i; ***C07C 11/04***(2006.01)i; ***C07C 11/06***(2006.01)i
FI: C07C7/09; B01J29/40 M; C07B61/00 300; C07C1/24; C07C11/04; C07C11/06

According to International Patent Classification (IPC) or to both national classification and IPC

**B. FIELDS SEARCHED**

Minimum documentation searched (classification system followed by classification symbols)

C07B31/00-63/04, C07C1/00-409/44

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | US 2014/0114104 A1 (CARANONI, Laurent) 24 April 2014 (2014-04-24) claims, paragraphs [0054], [0055], example 1, fig. 1 | 1-15 |
| X | US 2016/0318825 A1 (BRASKEM S. A.) 03 November 2016 (2016-11-03) claims, paragraphs [0064]-[0067], [0072]-[0074], [0077], example 2, tables 2, 3, fig. 2, 4, 5 | 1-15 |
| A | JP 2014-047175 A (MITSUBISHI CHEMICAL CORPORATION) 17 March 2014 (2014-03-17) | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 January 2025** | **28 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/040521**

| Patent document cited in search report | Publication date (day/month/year) | Patent family member(s) | Publication date (day/month/year) |
|---|---|---|---|
| US 2014/0114104 A1 | 24 April 2014 | WO 2013/004544 A1 | |
| | | EP 2729433 A1 | |
| | | CN 103649021 A | |
| US 2016/0318825 A1 | 03 November 2016 | WO 2015/089593 A1 | |
| | | EP 3085754 A1 | |
| | | BR 112016013092 B | |
| JP 2014-047175 A | 17 March 2014 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016117800 A
- WO 2009037992 A
- JP 5426983 B


### Non-patent literature cited in the description


- Adiabatic Fixed-Bed Reactors. Elsevier, 2014, 4
- *Stud. Surf. Sci. Catal.*, 1987, vol. 33, 167-215
- The Hydrothermal Synthesis of Zeolites. *Chemcal Reviews*, 2003, vol. 103, 663-702